(19) Europäisches Patentamt
European Patent Office
Office européen des brevets

(11) Publication number: **0 004 466**
**B1**

(12) # EUROPEAN PATENT SPECIFICATION

(45) Date of publication of patent specification: 27.01.82

(21) Application number: 79300469.8

(22) Date of filing: 23.03.79

(51) Int. Cl.[3]: C 07 D 498/16, A 61 K 31/535, C 12 P 17/18 //(C07D498/16, 303/00, 265/00, 225/00)

(54) **Demethyl maytansinoids and methods for producing them.**

(30) Priority: 24.03.78 JP 34645/78
22.12.78 JP 160787/78

(43) Date of publication of application:
03.10.79 Bulletin 79/20

(45) Publication of the grant of the European patent:
27.01.82 Bulletin 82/4

(84) Designated Contracting States:
BE CH DE FR GB IT NL SE

(56) References cited:
US - A - 3 896 111

CHEMICAL ABSTRACTS, vol. 86, no. 15, April 11, 1977, American Chemical Society, no. 106552x
Columbus, Ohio, U.S.A.
W. A. SHITE: "Part I. Synthesis of maytansine analogs. Part II. Synthesis of side-chain oxygen analogs of the cephalosporins"

(73) Proprietor: Takeda Chemical Industries, Ltd.
27, Doshomachi 2-Chome
Higashi-ku Osaka, 541 (JP)

(72) Inventor: Mitsuko, Asai
22-20, Showadaicho 2-chome
Takatsuki Osaka 569 (JP)
Inventor: Kazuo, Nakahama
16-17, Sawanonishi Kaidecho Muko
Kyoto 617 (JP)
Inventor: Motowo, Izawa
14-10, Tsukaguchicho 1-chome
Amagasaki Hyogo 661 (JP)

(74) Representative: Laredo, Jack Joseph et al,
Elkington and Fife High Holborn House 52/54
High Holborn
London, WC1V 6SH (GB)

(56) References cited:
JOURNAL OF THE AMERICAN CHEMICAL SOCIETY, vol. 96, no. 11, May 29, 1974
S. MORRIS KUPCHAN et al.: "Novel Maytansinoids, Structural Interrelations and Requirements for Antileukemic Activity 1—3", pages 3706—3708

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 004 466 B1

(56) References cited:
MICROBIAL TRANSFORMATIONS OF NONSTEROID CYCLIC COMPOUNDS BY KLAUS KIESLICH: G. Thieme Publishers Stuttgart 1976
*pages 740—746*

Demethyl maytansinoids and methods for producing them

This invention relates to demethylmaytansinoids which are maytansinoid compounds the methoxy group at the 20-position of which has been demethylated. The invention also relates to a method of producing such demethylmaytansinoid compounds.

The research we undertook to develop a method by which maytansinoid compounds could be microbiologically transformed into various other compounds led us to the finding that, when a culture broth of a certain type of microorganism, inclusive of a processed matter of such a culture broth, was allowed to act upon a maytansinoid compound, the 20-methoxy group of the maytansinoid compound was transformed into a hydroxy group, and that, when the resulting compound was further deacylated, the corresponding compound having a hydroxyl group in the 3-position was obtained. This finding was followed by further research which has resulted in the present invention.

The invention is directed to:

(1) a demethylmaytansinoid compound of the formula (I):

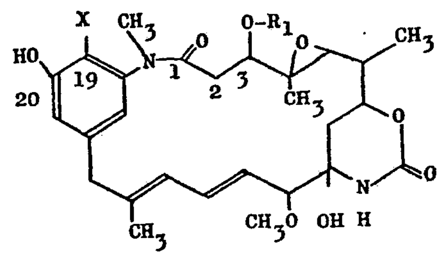

(I)

[wherein X is Cl or H; and $R_1$ is H or an acyl group];

(2) a method of producing a demethylmaytansinoid compound of the formula (I):

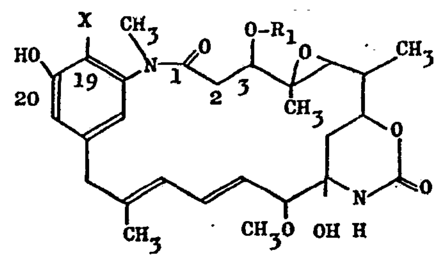

(I)

[wherein X is Cl or H; and $R_1$ is H or an acyl group] characterized in that a maytansinoid compound of the formula (II):

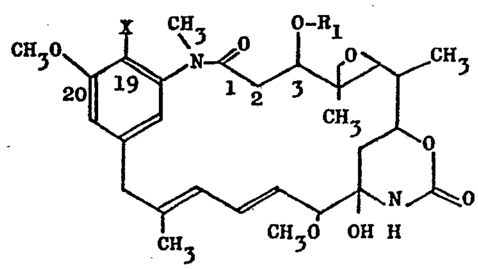

(II)

[wherein X and $R_1$ are as respectively defined above], is contacted with a culture broth, inclusive of a processed matter of the culture broth, of a microorganism belonging to the genus *Bacillus,* the genus *Streptomyces* or the genus *Actinomyces* which is able to transform the 20-methoxy group of the compound (II) into a hydroxy group; and

(3) a method of producing a demethylmaytansinoid compound of the formula (VI):

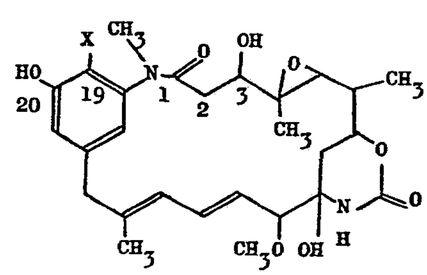

(VI)

[wherein X is Cl or H]

characterised in that a maytansinoid compound (IV), which corresponds to the compound (II) wherein $R_1$ is an acyl group $R_1'$, is contacted with a culture broth, inclusive of a processed matter of the culture broth, of a microorganism belonging to the genus *Bacillus*, the genus *Streptomyces* or the genus *Actinomyces* which is able to transform the 20-methoxy group of compound (IV) into a hydroxy group to yield a compound (V) which corresponds to compound (I), wherein $R_1$ is an acyl group $R'_1$, and this latter compound (V) is then deacylated.

In the general formulae given above, the acyl group represented by $R_1$ and $R'_1$ is an acyl group derived from a carboxylic acid, a carbamic acid or a carbonic acid derivative having a molecular weight of not more than 300, or an acyl group containing from 1 to 20 carbon atoms. The term "acyl group" encompasses, among others, saturated and unsaturated aliphatic acyl groups, saturated and unsaturated alicyclic acyl groups; aromatic acyl groups; saturated and unsaturated heterocyclic acyl groups; N-acyl-$\alpha$-amino acid-type acyl groups; carbamoyl-type acyl groups and acyl groups derived from half esters of carbonic acid.

Thus, such acyl groups may be, for example, groups represented by the formula:

$$—CO—R_2$$

(wherein $R_2$ is hydrogen or an optionally substituted or unsubstituted alkyl, alkenyl, cycloalkyl, cycloalkenyl, aryl or heterocyclic group, cycloalkyl, cycloalkenyl, aryl or heterocyclic group being optionally attached to the carbonyl carbon through an alkylene chain;

groups having the formula:

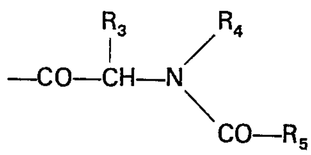

(wherein $R_3$ is hydrogen or an optionally substituted or unsubstituted alkyl, cycloalkyl, aryl, indolyl or imidazolyl group, the cycloalkyl, aryl, indolyl or imidazolyl group being optionally attached to the *alpha*-carbon atom through an alkylene chain; $R_4$ is hydrogen or an optionally substituted or unsubstituted alkyl, cycloalkyl, cycloalkylalkyl, aryl or benzyl group; $R_5$ is hydrogen, alkoxy, bornyloxy, isobornyloxy, benzyloxy or an optionally substituted or unsubstituted alkyl, alkenyl, cycloalkyl, cycloalkenyl, aryl or heterocyclic group, the cycloalkyl, cycloalkenyl, aryl or heterocyclic group being optionally attached to the carbonyl carbon atom adjacent to the nitrogen atom through an alkylene chain);

groups represented by the formula:

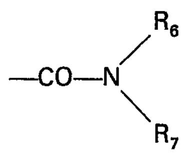

(wherein $R_6$ and $R_7$ may be the same or different and each is hydrogen or an optionally substituted or unsubstituted hydrocarbon residue or heterocyclic groups, $R_6$ and $R_7$ optionally forming a heterocyclic group as taken together with the adjacent nitrogen atom),

or the groups represented by the formula:

$$—CO—O—R_8$$

(wherein $R_8$ may be an optionally substituted or unsubstituted hydrocarbon residue).

When the acyl group represented by $R_1$ or $R'_1$ is a group having the formula:

$$—CO—R_2$$

(wherein $R_2$ is as defined above), the alkyl group represented by $R_2$ may be an alkyl group having 1 to 18 carbon atoms (e.g. methyl, ethyl, propyl, isopropyl, butyl, isobutyl, sec-butyl, tert-butyl, pentyl, isopentyl, 1-methylpropyl, hexyl, isohexyl, heptyl, 3-heptyl, octyl, nonyl, decyl, undecyl, dodecyl, tridecyl, tetradecyl, pentadecyl, hexadecyl, heptadecyl, octadecyl, 1,2-dimethylpropyl, 1-ethylpropyl, 1,2,2-trimethylpropyl, 1-propylbutyl or 2-ethylhexyl), although alkyl groups of 1 to 6 carbon atoms are preferable. Preferred examples of the alkenyl group $R_2$ are alkenyls of 2 to 18 carbon atoms (e.g. vinyl, allyl, 1-methylvinyl, 2-methylvinyl, 1-octenyl, 1-decenyl, 1,3-pentadienyl or oleyl), with alkenyls of 2 to 4 carbon atoms being particularly desirable.

As examples of the cycloalkyl group $R_2$, there may be mentioned cycloalkyls of 3 to 10 carbon atoms (e.g. cyclopropyl, cyclobutyl, cyclopentyl, cyclohexyl, cycloheptyl, cyclooctyl, norbornyl or

adamantyl), to which a benzene ring may be optionally fused, as in, e.g., 1- or 2-indanyl or benzocyclobutyl.

As examples of the cycloalkenyl group $R_2$, there may be mentioned cycloalkenyls of 3 to 10 carbon atoms (e.g. 1-cyclobutenyl, 1-, 2- or 3-cyclopentenyl, 1-, 2- or 3-cyclohexenyl, 4-cycloheptenyl, 4-cyclooctenyl, 1,4-cyclohexadienyl, 4-norbornenyl or 2,4,6-cycloheptatrienyl).

The aryl group $R_2$ may be, for example, phenyl or $\alpha$ or $\beta$-naphthyl, although phenyl is especially desirable.

The heterocyclic group $R_2$ may be, for example, a 4-, 5- or 6-membered heterocyclic group including N, O or/and S, whether saturated or unsaturated, and may have a benzene ring fused thereto. As examples of such N-containing 4-, 5- or 6-membered heterocyclic group, there may be mentioned, for instance, azetidinyl, pyridyl (e.g. 2-, 3- or 4-pyridyl), 1,2,3,4-tetrahydropyridyl, piperidyl, quinolyl, 1,2-dihydroquinolyl, 3- or 4-isoquinolyl, 1,2-dihydroisoquinolyl, pyrrolyl, pyrrolinyl, pyrrolidinyl or indolyl. The oxygen-containing 5- or 6-membered heterocyclic group may be for example, furyl, pyranyl, dihydropyranyl, benzofuryl or benzopyranyl, while the sulfur-containing 5- or 6-membered heterocyclic group may be, for example, thienyl or benzothienyl. The above heterocyclic groups may each include 2 to 4 hetero-atoms, which may be the same or different, such as N, O or/and S. As example of such heterocyclic groups, there may be mentioned imidazolyl, pyrazolyl, pyrazinyl, pyrimidinyl, pyridazinyl, 2-imidazolyl, imidazolidinyl, benzoimidazolyl, indazolyl, quinoxalyl, quinazolinyl, cinnolinyl, 1,4-dioxanyl, 1,4-benzodioxanyl, 1,2- or 1,3-dithiolanyl, 1,3-dithianyl, isooxazolyl, oxozolyl, morpholinyl, benzoisoxazolyl, benzoxazolyl, isothiazolyl, thiazolyl, benzoisothiazolyl, benzothiazolyl, benzothiazinyl, 1,2,4-, 1,2,5- or 1,3,4-oxadiazolyl, 1,2,3-, 1,2,4-, 1,2,5- or 1,3,4-thiadiazolyl, 1,2,3-, 1,2,5- or 1,3,4-triazolyl, 1,3,5-triazinyl, benzotriazolyl or 1,2,3,4-tetrazolyl. Generally speaking, when the heterocyclic groups are strongly basic groups having an NH group, such as azetidinyl, 1,2,3,4-tetrahydropyridyl, piperidyl, 1,2-dihydroquinolyl, 1,2-dihydroisoquinolyl, pyrrolyl, pyrrolinyl, pyrrolidinyl, pyrazolyl, 2-imidazolinyl, imidazolidinyl, indazolyl, morpholinyl, 1,2,3- 1,2,5- or 1,3,4-triazolyl, benzotriazolyl or 1,2,3,4-tetrazolyl, it is desirable that a suitable substituent, which will be described hereinafter, be present in the N-position, or an alkylene group, which will also be described hereinafter, be attached to the N atom.

The cylic group $R_2$ (i.e. the optionally substituted cycloalkyl, cycloalkenyl or aryl or heterocyclic group) may optionally be attached to the carbonyl carbon of —CO—$R_2$ through an alkylene chain. Therefore, when the cyclic group is attached to an alkylene chain, $R_2$ represents an optionally substituted cycloalkylalkyl, cycloalkenylalkyl, aralkyl or heterocyclic-alkyl group. The alkylene chain may consist in a straight-chain or branched alkylene group of 1 to 4 carbon atoms (e.g. methylene, ethylene, methylmethylene (ethylidene), propylene, butylene, 1-, 2- or 3-methylpropylene, 1- or 2-ethylethylene, propylmethylene, 1,1- or 1,2-dimethylethylene or isopropylmethylene). As examples of the cycloalkylalkyl group, there may be mentioned 1-adamantylmethyl, cyclohexylmethyl, 3-cyclohexylpropyl, 2-cyclopentenylmethyl, 2-cyclopentylethyl, cyclopropylmethyl, cyclobutylethyl, cyclopentylmethyl, 2-cyclohexylethyl and 2-methyl-2-cyclohexylpropyl.

As examples of the cycloalkenylalkyl group, there may be mentioned 1-, 2- or 3-cyclopentenylmethyl, 1-, 2- or 3-cyclohexenylmethyl, 4-cycloheptenyl-3-propyl and 1,4-cyclohexadienylmethyl.

Typical examples of the aralkyl group are benzyl, phenethyl, 1-, 2- or 3-phenylpropyl, $\alpha$-methylbenzyl, 1-methyl-3-phenylpropyl and 4-phenylbutyl.

As examples of the heterocycle-alkyl group, there may be mentioned 3-indolylmethyl, 3-(3-indolyl)propyl, 2-pyridylmethyl, 3-pyridylmethyl, 4-pyridylmethyl, 3-(2-thienyl)propyl, 2-benzothiazolylmethyl, 2-benzoxazolylmethyl, 3-benzoisothiazolylmethyl, 3-benzoisoxazolylmethyl, furfuryl, 2-thenyl and 2-(1-piperidinyl)ethyl.

When $R_2$ is an N-containing heterocyclic group, with its N atom linked to the carbonyl carbon of the acyl group —CO—$R_2$, the particular heterocyclic group is defined as being always attached to the carbonyl group through the alkylene chain mentioned above. As examples of such heterocycle-alkyl group with an alkylene chain attached to its N atom, there may be mentioned 1-pyrrolylmethyl, 2-oxo-1-pyrrolidinylmethyl, 1-imidazolyl-methyl, 3,5-dimethyl-1-pyrazolylmethyl, 1-piperidylethyl (or 1-piperidinoethyl), 4-morpholinylmethyl (or 4-morpholinomethyl), 1-tetrazolylmethyl, 2,5-dioxo-1-pyrrolidinylmethyl, 1,3-dioxo-2-isoindolylmethyl, 2-thioxo-4-oxo-3-thiazolidinylmethyl, 3,5-diiodo-4-oxo-1,4-dihydropyridyl-1-methyl, 4-methyl-1-piperazinylmethyl and 1-indolyethyl.

The alkyl, alkenyl, cycloalkyl, cycloalkenyl, aryl or heterocyclic group $R_2$ may optionally be substituted, the substituents being exemplified by alkoxy groups of 1 to 4 carbon atoms (e.g. methoxy, ethoxy, propoxy, isopropoxy, butoxy, isobutoxy, sec-butoxy or tert-butoxy), alkanoyl groups of 2 to 4 carbon atoms (e.g. acetyl, propionyl, butyryl or isobutyryl), alkanoyloxy groups of 2 to 4 carbon atoms (e.g. acetyloxy, propionyloxy, butyryloxy or isobutyryloxy), alkoxycarbonyl groups of 2 to 4 carbon atoms (e.g. methoxycarbonyl, ethoxycarbonyl, propoxycarbonyl or isopropoxycarbonyl), halogens (e.g. chlorine, fluorine, bromine or iodine), nitro, cyano, trifluoromethyl, di-$C_{1-4}$ alkylamino (e.g. dimethylamino, diethylamino, dipropylamino, diisopropylamino or dibutylamino), $C_{1-4}$ Alkylthio (e.g. methylthio, ethylthio, n-propylthio, isopropylthio, butylthio, isobutylthio, sec-butylthio, or tert-butylthio), methylsulfinyl, methylsulfonyl, oxo, thioxo and $C_{1-4}$ alkanoylamido (e.g. formamido, acetamido, propionylamino, butyrylamino or isobutyrylamino). When $R_2$ is a cyclic group (i.e. cycloalkyl, cycloalkenyl, aryl or

heterocyclic), alkyl groups of 1 to 4 carbon atoms (e.g. methyl, ethyl, propyl, isopropyl, butyl, isobutyl, sec-butyl or tert-butyl) may also be mentioned as the substituents. These substituents may be the same or different and may be present in the number of 1 to 3.

The substituted $C_{1-18}$ alkyl group $R_2$ may be, for example, methoxymethyl, butoxymethyl, methylthiomethyl, 2-methylthioethyl, 2-ethylthioethyl, 2-isopropylthioethyl, 2-butylthioethyl, 2-isobutylthioethyl, acetyloxymethyl, 2-acetyloxyethyl, ethoxycarbonylmethyl, 2-butoxycarbonylethyl, fluoromethyl, chloromethyl, 2-chloroethyl, 3-chloropropyl, 4-chlorobutyl, 3,3,3-trichloropropyl, trifluoromethyl, bromomethyl, 4-bromobutyl, 5-bromopentyl, iodomethyl, 2-iodoethyl, 1,1-dimethyl-2,2-dichloroethyl, 2-chloro-1-chloromethyl-1-methylethyl, cyanomethyl,2-methylsulfinylethyl or methylsulfonylmethyl. 1-Chlorovinyl may be mentioned as an example of the substituted $C_{2-10}$ alkenyl group $R_2$.

As examples of the substituted cyclo-$C_{3-10}$ alkyl group $R_2$, there may be mentioned 2,2-dimethylcyclopropyl, 2-propylcyclopropyl, 2-butylcyclopropyl, 4-isobutylcyclohexyl, 2-bromocyclopropyl, 2-chlorocyclobutyl, 4-chlorocyclohexyl, 2-iodocyclohexyl, 2,2-difluorocyclobutyl, 3-methoxycyclohexyl, 2,2-dimethyl-3-acetylcyclobutyl, 4-acetylcyclohexyl, 2-cyanocyclohexyl, 2-cyanocyclobutyl, 4-cyanocyclohexyl and 4-dimethylaminocyclohexyl.

As examples of the substituted cyclo-$C_{3-10}$ alkenyl group $R_2$, there may be mentioned 2-cyano-2-cyclohexenyl, 3,3-dimethyl-4-cyclobutenyl, 4-ethoxycarbonyl-1-cyclohexenyl and 4-butoxycarbonyl-1-cyclohexenyl.

Typical examples of the substituted aryl group $R_2$ are 2-, 3- or 4-methylphenyl, 4-tert-butylphenyl, 2-, 3- or 4-chlorophenyl, 2-, 3- or 4-bromophenyl, 2-, 3- or 4-iodophenyl, 2-, 3- or 4-fluorophenyl, 2- or 4-methoxyphenyl, 4-butoxyphenyl, 4-methoxycarbonylphenyl, 3-acetylphenyl, 2-, 3- or 4-nitrophenyl, 3- or 4-cyanophenyl, 4-dimethylaminophenyl, 4-diethylaminophenyl, 4-acetoxyphenyl, 4-butyryloxyphenyl, 2,4-, 2,5-, 2,6-, 3,4- or 3,5-dichlorophenyl, 3,4-dimethoxyphenyl, 3,4,5-trimethoxyphenyl, 3,4-methylenedioxyphenyl, 3-trifluoromethylphenyl, 4-methylthiophenyl, 4-methylsulfonylphenyl and 4-acetamidophenyl.

The optionally substituted or unsubstituted 4-, 5- or 6-membered heterocyclic group $R_2$ may be, for example, 1-acetyl-2-azetidinyl, 1-methyl-2-pyrrolyl, 3-methoxy-2-furyl, 3-methyl-2-furyl, 5-methyl-2-furyl, 5-nitro-2-furyl, 3-methyl-2-thienyl, 3-bromo-4,5-dimethyl-2-thienyl, 2-methyl-4-thiazolyl, 1,2-dimethyl-4-chloro-5-imidazolyl, 1-butyl-4-pyrazolyl, 2,4-dichloro-4-isothiazolyl, 5-methyl-1,2,3,4-thiadiazolyl, 3,5-dimethyl-4-isooxazolyl, 2-methyl-5-diisopropylamino-4-oxazolyl, 5-methyl-1, 2,5-oxadiazolyl-3, 4-methoxy-1, 2,5-oxadiazolyl-3, 5-methyl-1,3,4-oxadiazolyl-2, 3-methyl-1,2,3-thiadiazolyl-5-, 5-methyl-1,3,4-thiadiazolyl-2, 5-methyl-1,2,3-thiadiazolyl-4, 1-methyl-1,2,3-triazolyl-4, 2-ethyl-1,2,3,4-tetrazolyl-5, 5-nitro-2-pyridinyl, 6-ethyl-4-pyridinyl, 5-ethoxycarbonyl-3-pyridinyl, 5-chloro-3-pyridinyl, 1-butyryl-2-piperidyl, 2-oxo-5-pyranyl, 7-methoxy-3,4-dihydro-2H-pyranyl-2, 1-acetyl-2-pyrrolidinyl, 1-propyl-5-oxo-3-pyrrolidinyl, 3-methyl-2,4-dioxo-5-thiazolinyl, 4-, 5-, 6- or 7-nitro-3-indolyl, 5-fluoro-2-indolyl, 2-methyl-5-methoxy-3-indolyl, 1-methyl-2-indolyl, 5-chloro-2-benzothienyl, 3-methyl-2-benzofuryl, 1-methyl-2-benzoimidazolyl, 6-nitro-2-benzothiazolyl, 4-chloro-3-quinolyl, 6-methoxy-2-quinolyl, 2,4-dimethoxy-3-quinolyl, 2-methyl-1-oxo-3-isocarbostyryl, 7-methyl-3-coumaryl, 4-methyl-2-quinazolyl, 3-propyl-2,4-dioxo-5-imidazolinyl, 7-methoxycarbonyl-2-oxo-1,2-dihydro-3-quinazolyl, 2-furyl, 2-thienyl, 3-isoxazolyl, 4-imidazolyl, 1,2,5-thiadiazolyl-3, 2-, 3- or 4-pyridyl, 2-pyrazinyl, 2-pyrimidinyl, 2-*s*-triazinyl, 1,2-dithiolanyl, 3-indolyl, 2-benzothienyl, 2-benzofuryl, 3-benzopyrazolyl, 2-benzoimidazolyl, 2-benzoxazolyl, 3-benzoisoxazolyl, 3-benzoisothiazolyl, 2-benzothiazolyl, 2-benzo-1,4-oxazinyl, 3-quinolyl or 1-isoquinolyl.

As examples of the substituted cycloalkylalkyl group $R_2$, there may be mentioned 3-acetyl-2,2-dimethyl-1-cyclobutylmethyl, 3-acetoxy-2,2-dimethyl-1-cyclobutylmethyl, 2-(3-chloro-1-cyclobutyl)-ethyl, 2,3-dimethyl-1-cyclopentylmethyl, 2-isopropyl-1-cyclopentylmethyl, *cis*- or *trans*-4-acetamido-1-cyclohexylmethyl, *cis*- or *trans*-4-tert-butyl-1-cyclohexylmethyl and *cis*- or *trans*-2-(4-acetamido-1-cyclohexyl)ethyl.

As examples of the substituted cycloalkenylalkyl group $R_2$, there may be mentioned 2-(4-isopropyl-1-cyclohexenyl)ethyl, 1-ethyl-2-(4-isopropyl-1-cyclohexenyl)ethyl and 3-methoxy-4-methyl-3-cyclohexenylmethyl.

The substituted aralkyl group $R_2$ may be, for example, 4-bromobenzyl, 2-, 3- or 4-chlorobenzyl, 2,5- or 3,4-dimethoxybenzyl, 4-ethoxybenzyl, 4-fluorobenzyl, 3- or 4-methoxybenzyl, 4-methoxyphenethyl, 1- or 2-naphthylmethyl, 2-, 3- or 4-nitrobenzyl, 3-nitrophenethyl, 2-, 3- or 4-methylbenzyl, 3,4,5-trimethoxy-benzyl or $\alpha$-methylphenethyl.

As examples of the substituted heterocyclylalkyl group $R_2$, there may be mentioned 5-ethyl-3-indolylmethyl, 5-fluoro-3-indolylmethyl, 5-methoxy-3-indolylmethyl, 5-methyl-3-indolylmethyl and 1-methyl-5-tetrazolylmethyl.

When the acyl group $R_1$ or $R'_1$ is a group of the formula:

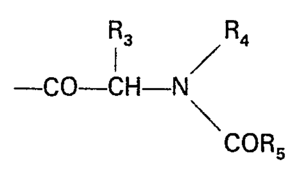

(wherein $R_3$, $R_4$ and $R_5$ are as respectively defined hereinbefore), the alkyl, cycloalkyl and aryl groups represented by $R_3$, $R_4$ and $R_5$ may be, for example, the corresponding groups mentioned for $R_2$. Thus, as examples of the cycloalkylalkyl group, there may be mentioned the corresponding groups mentioned for $R_2$.

As examples of the alkenyl, cycloalkenyl or heterocyclic group $R_5$, there may be mentioned the corresponding groups mentioned for $R_2$.

As examples of the alkoxy group $R_5$, there may be mentioned alkoxy groups of 1 to 7 carbon atoms (e.g. methoxy, ethoxy, propoxy, isopropoxy, butoxy, isobutoxy, sec-butoxy, tert-butoxy, pentoxy, hexyloxy or heptyloxy), although alkoxy groups of 1 to 4 carbon atoms are preferred.

The cycloalkyl, aryl, indolyl or imidazolyl group $R_3$ may optionally be attached to the $\alpha$-carbon atoms through an alkylene chain. Moreover, the cycloalkyl, cycloalkenyl, aryl or heterocyclic group $R_5$ may optionally be attached to the carbonyl carbon adjacent to the nitrogen atom through an alkylene chain. As examples of such alkylene chains, there may be mentioned the alkylene chains given hereinbefore for $R_2$.

Therefore, when such an alkylene chain is interposed, $R_3$ represents a cycloalkylalkyl, aralkyl, indolylalkyl, imidazolylalkyl, cycloalkenylalkyl or heterocycle-alkyl group.

As examples of such cycloalkylalkyl, cyclo alkenylalkyl, aralkyl- and heterocycle-alkyl groups, there may be mentioned the corresponding groups given for $R_2$.

The indolylalkyl group may be, for example, 3-indolylmethyl, 2-(3-indolyl)ethyl, 3-(3-indolyl)-propyl or 4-(3-indolyl)butyl. The imidazolylalkyl group may be, for example, 4-imidazolylmethyl, 2-(4-imidazolyl)ethyl, 3-(4-imidazolyl)propyl or 4-(4-imidazolyl)butyl.

The groups represented by $R_3$ and $R_4$, as well as the alkyl, alkenyl, cycloalkyl, cycloalkenyl, aryl, heterocyclic, cycloalkylalkyl, cycloalkenylalkyl, aralkyl and heterocycle-alkyl group represented by $R_5$, may optionally be substituted, and such substituents may be similar to those mentioned as substituents on the groups represented by $R_2$.

As examples of such substituted groups $R_3$, $R_4$ and $R_5$, there may be mentioned the corresponding substituted groups $R_2$.

As examples of the substituted indolyl $R_3$, there may be mentioned 5-bromo-2-indolyl, 5-chloro-2-indolyl, 5-fluoro-2-indolyl, 5-methoxy-2-indolyl, 1-methyl-2-indolyl, 5-methyl-2-indolyl and so on. The substituted imidazolyl group $R_3$ may be, for example, 1-methyl-5-imidazolyl, 3-methyl-5-imidazolyl or 2-methyl-4-imidazolyl.

As examples of the substituted indolylalkyl group $R_3$, there may be mentioned 5-bromo-2-indolylmethyl, 5-bromo-2-indolylethyl, 5-chloro-2-indolylmethyl, 5-chloro-2-indolylethyl, 5-fluoro-2-indolylmethyl, 5-fluoro-2-indolylethyl, 5-methoxy-2-indolylmethyl, 5-methoxy-2-indolylethyl, 1-methyl-2-indolylmethyl, 1-methyl-2-indolylethyl, 5-methyl-2-indolylmethyl and 5-methyl-2-indolylethyl.

As examples of the substituted imidazolylalkyl $R_3$, there may be mentioned 1-methyl-5-imidazolylmethyl, 1-methyl-5-imidazolylethyl, 3-methyl-5-imidazolylmethyl, 3-methyl-5-imidazolylethyl, 2-methyl-4-imidazolylmethyl, 2-methyl-4-imidazolylethyl, 1-methyl-4-imidazolylmethyl and 1-methyl-4-imidazolylethyl.

As examples of the substituted benzyl $R_4$, there may be mentioned 4-bromobenzyl, 2-, 3- or 4-chlorobenzyl, 4-fluorobenzyl, 4-methoxybenzyl, 4-ethoxybenzyl, 2-, 3- or 4-methylbenzyl and 3,4-dimethoxybenzyl.

As typical examples of the above-described N-acyl-$\alpha$-aminoacyl group:

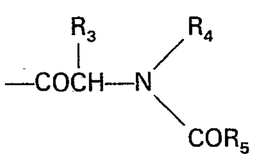

there may be mentioned N-acetyl-n-methylglycyl, N-benzoyl-N-methylglycyl, N-(4-chlorobenzoyl)-N-methylglycyl, N-acetyl-N-benzylalanyl, N-acetyl-N-methylleucyl, N-acetyl-N-methylphenylalanyl, 2-(N-acetyl-N-methyl)amino-3-methoxycarbonylpropionyl, 2-(N-acetyl-N-methyl)amino-3-methylmercaptopropionyl, 2-(N-acetyl-N-methyl)amino-3-ethylmercaptopropionyl, $N^{\alpha}$-acetyl-$N^{\alpha}$, N'-dimethylhistadinyl, N-acetyl-N-methylisoleucyl, N-acetyl-N-methylmethionyl, N-acetyl-N-methyltriptophanyl, N-acetyl-N-methyl-4'-acetoxytyrosinyl, N-benzyl-N-methyl-valyl, N-acetyl-N-methylphenylglycyl, N-isonicotinoyl-N-methyl-$\alpha$-aminobutyryl, (N-acetyl-N-methyl)amino-3-cyanopropionyl, N-acetyl-N-methyl-$\alpha$-(2-thiazolyl)glycyl and N-acetyl-N-methyl-(4'-dimethylamino)-phenylalanyl.

When the acyl group represented by $R_1$ or $R'_1$ is a group of the formula:

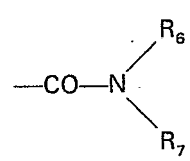

(wherein $R_6$ and $R_7$ are as respectively defined hereinbefore), the hydrocarbon residues $R_6$ and $R_7$ may be, for example, alkyl, alkenyl, cycloalkyl, cycloalkenyl, cycloalkylalkyl, cycloalkenylalkyl, aryl, aralkyl, phenylcycloalkyl, cycloalkylphenyl or biphenyl.

As examples of the alkyl, alkenyl, cycloalkyl, cycloalkenyl, cycloalkylalkyl, cycloalkenylalkyl, aryl and aralkyl groups, there may be mentioned the corresponding groups already given for $R_2$.

As examples of the phenylcycloalkyl group, there may be mentioned, among others, cycloalkyl groups of 3 to 10 carbon atoms (in particular, 3 to 7 carbon atoms) substituted by a phenyl group, e.g. 2-phenylcyclopropyl or 4-phenylcyclohexyl. As examples of the cycloalkylphenyl group, there may be mentioned a phenyl group substituted by one of the cycloalkyl groups, e.g. 4-cyclopentylphenyl or 4-cyclohexylphenyl. The biphenyl group may be, for example, 4-biphenyl.

As examples of the heterocyclic groups $R_6$ and $R_7$, there may be mentioned 4-, 5- or 6-membered heterocyclic groups including N, O or/and S atoms, which may optionally be saturated or unsaturated, and optionally be fused to a benzene ring. As examples of such heterocyclic groups, there may be mentioned azetidinyl, pyrrolyl, furyl, thienyl, thiazolyl, isothiazolyl, oxazolyl, isoxazolyl, pyrazolyl, imidazolyl, pyridyl, pyrimidinyl, pyrazinyl, triazinyl, quinolyl, quinazolyl, quinoxalyl, indolyl, benzofuranyl and benzothienyl.

Optionally, $R_6$ and $R_7$ may, taken together, form a heterocyclic group in combination with the adjacent N atom and, as examples of such a heterocyclic group, there may be mentioned azetidinyl, pyrrolidinyl, piperidinyl and morpholinyl.

The hydrocarbon residues and heterocyclic groups, as represented by $R_6$ and $R_7$, as well as the heterocyclic group

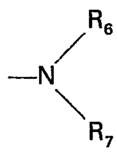

may optionally be substituted. As examples of the substituents thereon, there may be mentioned alkoxy groups of 1 to 4 carbon atoms (e.g. methoxy, ethoxy, propoxy, isopropoxy, butoxy, isobutoxy or sec-butoxy), $C_{1-4}$ alkylthio (e.g. methylthio, ethylthio, propylthio, isopropylthio or butylthio), phenoxy, phenylthio, cyclohexyloxy, halogen (e.g. fluorine, chlorine, bromine or iodine), cyano, $C_{2-5}$ alkoxycarbonyl (e.g. methoxycarbonyl, ethoxycarbonyl, propoxycarbonyl, isopropoxycarbonyl, butoxycarbonyl, isobutoxycarbonyl or sec-butoxycarbonyl), benzyloxycarbonyl, nitro, amino-sulfonyl and dialkylamino (e.g. dimethylamino, diethylamino, diisopropylamino or dibutylamino). These substituents may be the same or different and may be present in the number of 1 to 3.

As to substituents on the cyclic hydrocarbon moieties, of the hydrocarbon residues, and on the heterocyclic groups mentioned above, there may be mentioned, in addition to the above-mentioned substituent groups, alkyl groups of 1 to 4 carbon atoms (which may be further substituted by the hydrocarbon residues $R_6$ and $R_7$), such as methyl, ethyl, propyl, isopropyl, butyl, trifluoromethyl, chloromethyl, 2-cyanoethyl, methoxymethyl, ethoxycarbonylmethyl and dimethylaminomethyl.

As examples of substituents on alkyl groups $R_6$ and $R_7$, there may be mentioned the groups given as substituents on the hydrocarbon residues as well as heterocyclic groups (which may be substituted) similar to those mentioned as the heterocyclic groups $R_6$ and $R_7$.

As examples of substituted hydrocarbon residues $R_6$ and $R_7$, there may be mentioned 2-methoxyethyl, 3-methoxypropyl, 2-methoxy-1-methylethyl, 3-isopropoxypropyl, 3-sec-butoxy-propyl, 3-cyclohexyloxypropyl, 3-phenoxypropyl, 2-chloroethyl, 3-chloropropyl, 4-chlorobutyl, 2-propylthioethyl, 2-phenylthioethyl, 2-cyanoethyl, 5-cyanopentyl, 4-cyanocyclohexylmethyl, methoxycarbonylmethyl, ethoxycarbonylmethyl, 1- or 2-methoxycarbonylethyl, 1-methoxycarbonylisobutyl, 5-methoxycarbonylpentyl, 5-dimethylaminopentyl, trifluoromethyl, 2-, 3- or 4-tolyl, xylyl, 2,4,5- or 2,4,6-trimethylphenyl, 2-, 3- or 4-bromophenyl, 4-iodophenyl, 2- or 3-trifluorophenyl, 2-, 3- or 4-nitrophenyl, 4-chloro-3-trifluoromethylphenyl, 2-methyl-4-nitrophenyl, 5-nitro-1-naphthyl, 8-chloro-1-naphthyl, 4-methoxycarbonylphenyl, 4-ethoxycarbonylphenyl, 4-aminosulfonylphenyl, 2-, 3- or 4-methoxyphenyl, 4-ethoxyphenyl, 2,5-dimethoxyphenyl, 1-methoxycarbonyl-2-phenethyl, 1-methoxycarbonyl-1-phenylmethyl, 2-, 3- or 4-methylbenzyl, 2-, 3- or 4-chlorobenzyl, 2- or 3-fluorobenzyl, 3-iodobenzyl, 2,4- or 3,4-dichlorobenzyl, 4-methoxybenzyl, $\alpha$-methylbenzyl, 1,1-dimethylphenethyl, 4-methoxyphenethyl, 2- 3- or 4-picolyl, 5-methyl-2-thenyl, 5-methylfurfuryl, 3-piperazinopropyl, 2-morpholinoethyl, 4-methyl-1-piperazinopropyl, 2-morpholinoethyl, 4-methyl-1-piperazinylpropyl, 2-(1-methyl-2-pyrrolidinyl)ethyl, 2-thiazolylmethyl, 2-methyl-4-oxazolylmethyl and 5-chloro-1-methyl-3-indolylethyl.

As examples of the optionally substituted or unsubstituted heterocyclic groups $R_6$ and $R_7$, there may be mentioned 1-methyl-2-azetidinyl, 1-methyl-2-pyrrolyl, 5-methyl-2-furyl, 5-nitro-2-furyl, 3-methyl-2-thienyl, 4,5-dichloro-2-thienyl, 2-methyl-4-thiazolyl, 1-methyl-4-imidazolyl, 1,2-dimethyl-4-chloro-5-imidazolyl, 3,5-bismethylthio-4-isothiazolyl, 3-methyl-5-isoxazolyl, 2-methyl-4-oxazolyl, 1-methyl-3-pyrazolyl, 2-, 3- or 4-pyridyl, 4,5,6-trichloro-2-pyrimidinyl, 3,5,6-trichloro-2-pyrazinyl, 4,6-dichloro-2-*s*-triazinyl, 3- or 4-quinolyl, 2-quinazolyl, 2-quinoxazolyl, 5-fluoro-1-methyl-3-indolyl, 2-benzofuryl and 2-benzothienyl. When the heterocyclic group of

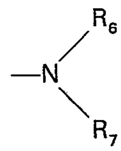

is substituted, the resulting group may be, for example, 2-, 3- or 4-methyl-1-piperidinyl, 4-methyl-1-piperazinyl, 2,6-dimethylmorpholino or 2-propyl-1-piperidinyl.

As typical examples of

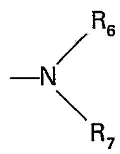

wherein both $R_6$ and $R_7$ are other than hydrogen, there may be mentioned dimethylamino, diethylamino, dipropylamino, diisopropylamino, diisobutylamino, dibenzylamino, diphenethylamino, diphenylpropylamino, (N-methyl-N-benzyl)amino, (N-ethyl-N-butyl)-amino, (N-methyl-N-butyl)-amino, (N-methyl-N-cyclopentyl)amino, (N-methyl-N-cyclohexyl)amino and (N-methyl-N-furfuryl)amino.

When the acyl group represented by $R_1$ or $R'_1$ is a group of the formula:

$$—CO—O—R_8$$

(wherein $R_8$ is as defined hereinbefore), the hydrocarbon residue $R_8$ may be, for example, alkyl, cycloalkyl, aryl or aralkyl.

Alkyl groups of 1 to 8 carbon atoms are preferred. As examples of the alkyl group $R_8$, there may be mentioned alkyl groups of 1 to 18 carbon atoms (e.g. methyl, ethyl, propyl, isopropyl, butyl, isobutyl, sec-butyl, tert-butyl, pentyl, hexyl, octyl, nonyl, decyl, undecyl, dodecyl, tetradecyl, hexadecyl, octadecyl, 1-ethylpropyl, neopentyl, 1-ethylpentyl or 1- or 2-ethylhexyl).

As examples of the cycloalkyl group $R_8$, there may be mentioned cycloalkyl groups of 3 to 10 carbon atoms (e.g. cyclopropyl, cyclobutyl, cyclopentyl, cyclohexyl, cycloheptyl, cyclooctyl or adamantyl).

As examples of the aryl group $R_8$, there may be mentioned phenyl or $\alpha$ or $\beta$-naphthyl.

As examples of the aralkyl group $R_8$, there may be mentioned alkyl groups of 1 to 4 carbon atoms substituted by an aryl group, especially a phenyl group (e.g. benzyl, phenethyl, 1- or 3-phenylpropyl, 1-phenylethyl, 1-methyl-3-phenylpropyl or 4-phenylbutyl).

The hydrocarbon residue $R_8$ mentioned above may optionally be substituted, the substituents being exemplified by alkoxy groups of 1 to 4 carbon atoms (e.g. methoxy, ethoxy, propoxy, isopropoxy, butoxy, isobutoxy or sec-butoxy), phenoxy, benzyloxy, halogens (e.g. fluorine, chlorine, bromine and iodine) and cyano. These substituents may be the same or different and may be present in the number of from 1 to 3.

As to substituents on the cyclic hydrocarbon moieties of the hydrocarbon groups (e.g. the cycloalkyl or aryl moieties), we may mention, in addition to the above-mentioned substituent groups, alkyl groups of 1 to 4 carbon atoms (e.g. methyl, ethyl, propyl, isopropyl or butyl), and halogenated alkyl of 1 to 4 carbon atoms (e.g. chloromethyl, bromomethyl, dichloromethyl, chlorodifluoromethyl or trifluoromethyl).

As examples of the substituted alkyl groups $R_8$, we may mention, among others, 2-methoxyethyl, 2-ethoxyethyl, 2-butoxyethyl, 4-ethoxybutyl, chloromethyl, 1- or 2-chlorethyl, 2-bromoethyl, 2-fluoroethyl, 3-chloropropyl, 2,3-dichloropropyl, 2-chloroisopropyl, 1-chloromethyl-2-chloroethyl, 2,2,2-trichloroethyl, 2,2,2-trifluoroethyl and 1- or 2-cyanopropyl.

As examples of the substituted cycloalkyl group $R_8$, we may mention, among others, 1-methylcyclobutyl, 1-methylcyclopentyl and 1-methylcyclohexyl.

Typical examples of the substituted aralkyl groups $R_8$ are, among others, 2-, 3- or 4-chlorobenzyl, 4-bromobenzyl, 4-methoxybenzyl, 2,5- or 3,4-dimethoxybenzyl and 3-chloro-4-methylbenzyl.

Typical examples of the substituted aryl groups $R_8$ are, among others, 2-, 3- or 4-methylphenyl, 2-, 3- or 4-methoxyphenyl, 4-ethoxyphenyl, 2-, 3- or 4-chlorophenyl, 4-fluorophenyl, 2,4-dichlorophenyl, 2-, 3- or 4-chloromethylphenyl, 4-trifluoromethylphenyl, 4-bromophenyl and 3-dimethylaminophenyl.

The microorganism employable in the method of this invention may be any microorganism of the genus *Bacillus*, the genus *Streptomyces* or the genus *Actinomyces*, inclusive of any mutants thereof, which is able to transform the 20-methoxy group of the maytansinoid compound (II) into a hydroxy group. As examples of such microorganisms, there may be mentioned *Bacillus megaterium* IFO 12108, *Streptomyces flavotricini* IFO 12770, *Streptomyces platensis* IFO 12901, *Streptomyces libani* IFO

13452 and *Actinomyces nigrescens* IFO 12894.

Morphologies of the microorganisms are as follows:

(A) *Bacillus megaterium* IFO 12108
colonies on nutrient agar (DIFCO):
circular, crenate, low-convex, fine granular surface, pale-brown.
Cells:
Young cells:
Gram-positive rods, 0.8 to 1.2 microns by 2.8 to 3.6 microns, lightly, stained cells filled with unstained globules: motile by peritrichous flagella.
Spores: ellipsoidal: central or paracentral:
not appreciably swelling the sporangia.

(B) *Streptomyces flavotricini* IFO 12770 = ISP 5152
Aerial mycelium: rectiflexibles
Spore surface: smooth
see International Journal of Systematic Bacteriology
Vol. 18(2), p. 114 (1968).

(C) *Streptomyces platensis* IFO 12901 = ISP 5041
Aerial mycelium: spirals
Spore surface: smooth
See int. J. Syst. Bacteriol. Vol. 18(4), p. 360 (1968).

(D) *Streptomyces libani* IFO 13452 = ISP 5555
Aerial mycelium: spirals
Spore surface: smooth
See Int. J. Syst. Bacteriol. Vol. 22(4), p. 314 (1972).

(F) *Actinomyces nigrescens* IFO 12894 = ISP 5276
Aerial mycelium: spirals
Spore surface: Smooth to warty, see Int. J. Syst. Bacteriol.
Vol. 18(4), p. 353 (1968).

The microorganisms assigned the above-mentioned IFO numbers are found on the Institute for Fermentation Osaka List of Cultures 1978 sixth edition, published by the Institute for Fermentation, Osaka (IFO) Japan. The above-mentioned microorganisms assigned the IFO numbers of IFO 12108, IFO 12770, IFO 12901, IFO 12894 are also found on the Institute for Fermentation Osaka List of Cultures 1972 fifth edition, and the microorganisms assigned the IFO number of IFO 13452 is also found on the Institute for Fermentation Osaka List of Cultures 1975 Supplement to the fifth edition. The microorganisms appearing on the above-mentioned Lists can be obtained from the latter Institute.

Generally speaking, microorganisms of the genera *Bacillus, Streptomyces* and *Actinomyces* are liable to vary in their characteristics, and mutants can be easily derived therefrom, for example by such artificial mutagenic treatments as irradiation with X-rays, ultraviolet rays or other radiation, or by means of an artificial mutagen (e.g. nitrosoguanidine or ethyleneimine). All such mutants can be employed in the practice of this invention, insofar as they are able to transform the 20-methoxy group of the maytansinoid compound (II) into an hydroxy group.

The media used in the cultivation of the above microorganisms may be any fluid or solid media containing the nutrients which the particular organisms are able to utilize, although a fluid medium is preferred for commercial scale operations. The culture medium is prepared using the carbon sources which will be assimilated by the microorganism and the nitrogen sources, inorganic materials and trace nutrients, *inter alia*, which will be digested by the microorganisms. Thus, as such carbon sources, there may be employed glucose, lactose, sucrose, maltose, dextrin, starch, glycerin, mannitol, sorbitol, fats or oils (e.g. soybean oil, lard oil or chicken oil), while the nitrogen sources may be meat extract, yeast extract, dried yeast, soybean meal, corn steep liquor, peptone, cottonseed meal, spent molasses, urea or ammonium salts (e.g. ammonium sulfate, ammonium chloride, ammonium nitrate or ammonium acetate). The medium may further contain appropriate amounts of various salts such as salts of sodium, potassium, calcium or magnesium, metal salts such as salts of iron, manganese, zinc, cobalt or nickel, salts of phosphoric acid and boric acid, and salts of organic acids such as acetic acid or propionic acid. The medium may also contain other components such as aminoacids (e.g. glutamic acid, aspartic acid, alanine, glycine, lysine, methionine or proline), peptides (e.g. dipeptides or tripeptides), vitamins (e.g. vitamin $B_1$ or $B_2$, nicotinic acid or vitamin $B_{12}$, C or E) and nucleic acids (e.g. purine or pyrimidine or their derivatives. It is, of course possible to add inorganic or organic acids, alkalies, buffers, and so on for the purpose of adjusting the pH of the medium, or to add suitable amounts of oils and surfactants, for example, for defoaming purposes.

The cultivation may be carried out by various procedures such as stationary, shake or aerobic stirred culture. Submerged aerobic culture is preferred for commercial operations. While the cultural conditions depend on the condition and composition of the medium, the microorganisms, the culture procedure and so on, it is normally desirable that the cultivation be carried out at a temperature of from 20 to 45°C and at an initial pH of substantial neutrality. The temperature in the intermediate stage of cultivation is desirably between 24°C and 37°C, and the initial pH of the medium is desirably between 6.5 and 8.5. While the cultivation can generally be completed in 6 to 100 hours, an incubation period of 16 to 60 hours is particularly satisfactory.

By the term "culture broth", as used herein, is meant the product obtained by the cultivation process described above. By the term "processed matter of the culture broth", as used herein, is meant the cells or disrupted cells containing a demethylation enzyme system which are obtainable by subjecting the abovementioned culture broth to any of such physical or chemical treatments as filtration, centrifuging, ultrasonic disruption, treatment with a French press, alumina grinding or treatment with a bacteriolytic enzyme, a surfactant or an organic solvent.

The demethylation enzyme system purified by the conventional procedure and the bacterial cells or demethylation enzyme system immobilized by the conventional procedure may also be successfully utilized.

The method of this invention comprises bringing the starting compound (II) into contact with the culture broth or the processed matter of the culture broth. The concentration of the starting compound in the reaction system is within the range of from 1 to 400 $\mu$g/ml, advantageously from 1 to 200 $\mu$g/ml, and preferably from 50 to 200 $\mu$g/ml. The preferred reaction temperature is from 20 to 50°C and the preferred pH is from 5 to 10, although temperatures from 24 to 40°C and pH levels from 6 to 9 are especially desirable. The reaction time may range from 10 minutes to 100 hours, preferably from 1 to 48 hours, and more preferably from 8 to 48 hours. While the reaction may be carried out under stationary, shake, aerobic submerged or stirred conditions, it is more advantageous to conduct the reaction under shake, aerated or stirred culture conditions. If desired, reaction stimulators, enzyme stabilizers and other agents may be added to the reaction system.

As example of a reaction stimulator, there may be mentioned coenzymes such as nicotinamideadenine dinucleotide (NAD), nicotinamideadenine dinucleotide phosphate (NADP), flavine mononucleotide (FMN) or flavine-adenine dinucleotide (FAD), their precursors (e.g. adenine, adenosine, adenylic acid, nicotinamide, flavine or riboflavine), metal salts (e.g. magnesium chloride, manganese chloride, ferrous chloride, ferric chloride or zinc chloride), surfactants [e.g. Triton X-100 (trade mark) (Rohm and Haas Co., U.S.A.) or Briji-58 (trade mark) (Kao-Atlas Co., Japan)] and 3',5'-cyclic adenylic acid. The enzyme stabilizer may be, for instance, cysteine, 2-mercaptoethanol, dithiothreitol, sucrose or glycerol.

The novel substance (I) thus produced can be detected by thin-layer chromatography (hereinafter referred to briefly as TLC). First, the reaction mixture is extracted with ethyl acetate, the extract is concentrated to one-hundredth of its initial volume and TLC is carried out on the concentrate using a precoated silica gel plate (Kieselgel 60 $F_{254}$ (trade mark), Merck, Germany, 0.25 mm, 20 × 20 cm) and a 9:1 mixture of chloroform and methanol. The chromatogram is irradiated with ultraviolet light at 2537 Å to detect the desired compound.

The product compound (I) can be isolated from the reaction mixture by utilizing the purification procedures normally used for the recovery of microbial metabolites, by taking advantage of the weakly acidic and lipophilic property of compounds of this class. By way of example, the procedure utilizing the difference in solubility between the compound (I) and impurities, the procedure which takes advantage of the differential adsorptive affinity of various adsorbents, e.g. activated charcoal, macroporous non-ionic resins, silica gel or alumina, for the compound (I) versus any impurities, and the procedure of removing impurities by means of ion-exchange resins may be employed alone, in combination or in repetition. In the case of a procedure relying on solubility difference, use is made of a suitable extraction solvent such as a water-immiscible organic solvent, e.g. a fatty acid ester (e.g. ethyl acetate or amyl acetate), an alcohol (e.g. butanol), a halogenated hydrocarbon (e.g. chloroform) or a ketone (e.g. methyl isobutyl ketone). The extraction procedure is carried out in the weakly acidic pH region; preferably, the compound (I) is extracted with ethyl acetate from the culture broth filtrate preadjusted to pH 6. The extract thus obtained is washed with water and concentrated under reduced pressure. A non-polar solvent such as petroleum ether is added to the residue and the crude product (i) containing the active compound is recovered. Since the TLC of this crude product gives many spots other than that of the novel demethyl-maytansinoid compound (I), the following stepwise purification procedure is carried out. Various adsorption chromatographic techniques are useful for this purpose, and common supports such as silica gel, alumina or macroporous non-ionic adsorbent resins may be used as the absorbents. However, for the purification of the crude product (i), silica gel is the most advantageous of all the adsorbents. Development is started with petroleum ether and hexane, followed by the elution of the novel demethylmaytansinoid compound (I) with a solvent admixed with one or more polar solvents such as ethyl acetate, acetone, ethanol or methanol. For example, column chromatography may be carried out using silica gel (Merck, Germany, 0.05 to 0.2 mm) as the carrier and with incremental ratios of ethyl acetate to hexane, for example hexane-ethyl acetate (50:1), Hexane-ethyl acetate (20:1),

hexane-ethyl acetate (7:1), ethyl acetate and finally ethyl acetate-methanol (19:1). The eluate is assayed by TLC and the fractions containing the desired demethylmaytansinoid compound (I) are pooled, concentrated under reduced pressure and treated with petroleum ether or hexane to recover a crude product (ii). Because this product still includes impurities, it is further purified as follows. This second purification process is carried out for example, on a second silica gel column using a different solvent system.

The development is started with a halogenated hydrocarbon such as dichlormethane or chloroform, followed by elution with solvent admixed with a polar solvent such as an alcohol (e.g. ethanol or methanol or a ketone (e.g. acetone or methyl ethyl ketone) to recover the novel demethylmaytansinoid compound (I). The order of the solvent systems used for the first and the second silica gel column chromatography treatments may be reversed, and it is also possible to employ other suitable combinations of common organic solvents which are usually available.

When a macroporous adsorbent resin is employed for the purification of the crude product (ii), the novel demethylmaytansinoid compound (I) may be separated by elution with a mixture of a lower alcohol, a lower ketone or an ester with water. As examples of the lower alcohols, there may be mentioned, among others, methanol, ethanol, propanol, butanol, and so on. The lower ketone may be for example, acetone or methyl ethyl ketone. The ester may be, for example, ethyl acetate. Thus, by way of illustration, the crude product (ii) may first be dissolved in 50 V/V % aqueous methanol and adsorbed on a column of Diaion HP-10 (trade mark) (Mitsubishi Chemical Industries Ltd.). The column is then washed with 50 V/V % methanol, followed by elution with 90 V/V % aqueous methanol to yield the desired novel demethylmaytansinoid compound (I).

The demethylmaytansinoid compound (I) thus obtained is concentrated under reduced pressure and crystallized from ethyl acetate. Alternatively, after the concentration, petroleum ether is added to the concentrate and the resulting powder is recovered.

Moreover, by deacylating the compound (V), which is a demethylmaytansinoid compound (I) wherein $R_1$ is an acyl group $R'_1$, a demethylmaytansinoid compound (VI) can be obtained.

Thus, deacylation of the demethylmaytansinoid compound (V) gives a demethylmaytansinoid compound (VI) which is a compound (I) wherein $R_1$ is hydrogen. In this connection, since the acyl group is present in the $\beta$-position of the carbonyl group, the conventional reductive cleavage reaction procedure can be utilized with advantage. Thus, the compound (VI) can be obtained by the reductive cleavage of the O-ester bond in the 3-position with the use of a complex metal hydride compound [e.g. lithium aluminium hydride ($LiAlH_4$)] at a low temperature (e.g. —20 to 0°C), the reaction being thus accomplished without affecting the other functional groups such as, e.g., the carbonyl or epoxy group, or the carbon-carbon double bond. The compound (VI) can be isolated and purified by the procedure mentioned hereinbefore.

When the above demethylmaytansinoid compound (I) is liable to stereoisomerism at the acyl group $R_1$ (e.g. D- and L-forms), the compound (I) encompasses such isomers and mixtures thereof. Generally, such an isomeric configuration may already be present in the starting compound (II) and, as will be described in detail, there are cases in which the compound (II) has already been resolved into the component isomers during the process of its production by a *per se* conventional separation procedure such as silica gel chromatography or high pressure liquid chromatography.

In the method of this invention, the isomeric relationship in the compound (I) is in many cases identical with that in the compound (II).

Moreover, when a mixture of such isomers is employed as the starting compound (II), the product compound (I) is obtained as a mixture of isomers. These isomers can in general be separated from each other by a procedure known *per se*, e.g. by silica gel chromatography.

The compound (I) according to this invention can be used as an antifungal, antiprotozoal or antitumour agent. The toxicity of the compound (I) is low.

The compound (I) can also be used as an intermediate for the synthesis of useful medicines.

Tests of biological activity have been performed on the compounds obtained in the Examples given hereinafter.

Biological activity

A) *Antimicrobial activity*

With a trypticase-soy-agar medium (Baltimore Biologicals Limited, U.S.A.) as the assay medium, the minimal inhibitory concentrations of the compound (I) were assayed against the microorganisms mentioned below employing the paper disc method. Thus, on the plates of those microorganisms, the antibacterial activity of the compound (I) was assayed with paper discs which had been impregnated with 0.02 ml of a 300 $\mu$g/ml solution of the demethylmaytansinoid compound (I) (the paper discs were supplied by Toyo Seisakusho, Japan, thin-type, 8 mm diam). The results showed that the demethylmaytansinoid compound (I) was inactive against the following species of microorganisms: *Escherichia coli, Proteus vulgaris, Proteus mirabilis, Pseudomonas aeruginosa, Staphylococcus aureus, Bacillus subtilis, Bacillus cereus, Klebsiella pneumoniae, Serratia marcescens* and *Mycobacterium avium*.

On the other hand, on agar plates [3.5 g disodium hydrogen phosphate, 0.5 g monopotassium phosphate, 5 g of yeast extract (Difco (trade mark)), 10 g glucose, 15 g agar, 1000 ml distilled water,

pH 7.0], the antifungal activity of the test compound was assayed against *Hamigera avellanea* IFO 7721 by the paper disc method. Thus, on the plates inoculated with the above microorganism, the zones of growth inhibition were determined using paper discs impregnated with 0.02 ml of a 100 μg/ml solution of the demethylmaytansinoid compound (I).

The results showed that the demethylmaytansinoid compound (I) inhibited the growth of the said microorganism *Hamigera avellanea*. The zones of growth inhibition by the compound (I) are shown in Table 1 below.

Then, with *Tetrahymena pyriformis* W as the test organism and a medium composed of 20 g tryptose-peptone (Difco CO.), 1 g yeast extract, 2 g glucose, 1000 ml distilled water and 10 ml IM phosphate buffer (pH 7.0) as the assay medium, the microorganism was incubated at 28°C for 44 to 48 hours, and the growth inhibitory activity of the demethylmaytansinoid compound (I) was assayed by the serial dilution method. The results showed that the demethylmaytansinoid compound (I) was active against the test organism. The zones of growth inhibition and the minimal inhibitory concentrations of compound (I) are also shown in Table 1 below.

TABLE 1

| Test Compound | Antifungal activity, (φ mm) *Hamigera avellanea* IFO 7721 | Antiprotozoan activity, MIC (μg/ml) *Tetrahymena pyriformis* W |
|---|---|---|
| 20-Demethoxy-20-hydroxymaytansinol | <8 | 40 |
| 20-Demethoxy-20-hydroxymaytanacine | 10 | 10 |
| 20-Demethoxy-20-hydroxymaytansinol propionate | 15 | 2 to 4 |
| 20-Demethoxy-20-hydroxymaytansinol 3-isobutyrate (PDM–3) | 21 | 2 |
| 20-Demethoxy-20-hydroxymaytansinol 3-butyrate (PDM–3′) | 21 | 2 |
| 20-Demethoxy-20-hydroxymaytansinol 3-isovalerate (PDM–4) | 24 | 1 |
| 20-Demethoxy-20-hydroxymaytansinol 3-hexanoate | 21 | 1 to 2 |
| 20-Demethoxy-20-hydroxymaytansinol 3-cyclohexanecarboxylate | 20 | 1 to 2 |
| 20-Demethoxy-20-hydroxymaytansinol 3-phenylacetate | 23 | 1 to 2 |
| 20-Demethoxy-20-hydroxymaytansinol 3-p-chlorobenzoate | 10 | 4 |
| 20-Demethoxy-20-hydroxymaytansinol 3-picolinate | 10 | ≧4 |
| 20-Demethoxy-20-hydroxymaytansinol 3-N-phenylcarbamate | <8 | ≧8 |
| 20-Demethoxy-20-hydroxymaytansine (L) | 19 | 2 to 4 |
| 20-Demethoxy-20-hydroxymaytansine (L) | <8 | >16 |
| 20-Demethoxy-20-hydroxymaytanprine (L) | 23 | 1 to 2 |
| 20-Demethoxy-20-hydroxymaytanbutine (L) | 24 | 1 to 2 |

TABLE 1

| Test Compound | Antifungal activity, ($\phi$ mm) *Hamigera avellanea* IFO 7721 | Antiprotozoan activity, MIC ($\mu$g/ml) *Tetrahymena pyriformis* W |
|---|---|---|
| 20-Demethoxy-20-hydroxymaytanvaline (L) | 24 | 2 |
| 20-Demethoxy-20-hydroxydechloro-maytansinol 3-isobutyrate | 16 | 4 to 8 |
| 20-Demethoxy-20-hydroxymaytansinol 3-phenylacetate | 15 | 4 to 8 |

B) *Antitumour activity*

In a therapeutic test on mice into which P-388 tumour cells had been intraperitoneally transplanted showed that the demethylmaytansinoid compound (I) administered intraperitoneally once daily for 9 consecutive days produced a definite prolongation of the life spans of the mice.

C) *Acute toxicity*

The acute toxicity test on mice by the intravenous route showed that the demethylmaytansinoid compound (I) produced no deaths at all at the dose level of 1000 $\mu$g/kg.

Because the demethylmaytansinoid compound (I) has thus a strong growth-inhibitory activity against fungi and protozoa, it is useful as an antifungal or antiprotozoal agent. Moreover, since the demethylmaytansinoid compound (I) has a life-span extending activity in tumour-bearing mammalian animals (e.g. mouse), it is expected to be of value as an anti-tumour agent.

As an antifungal or antiprotozoal agent, the demethylmaytansinoid compound (I) can be used with advantage to test bacterial flora in samples of soil, active sludges or animal body fluids. Thus, when useful bacteria are to be isolated from soil samples or when the activity of bacteria to the exclusion of that of fungi is to be tested in the operation and analysis of an active sludge system for the disposal of waste water, the compound (I) can be utilized to ensure the selective growth of bacterial flora without permitting the growth of the fungi concomitantly present in the samples. Thus, for example, a test sample may be added to a fluid or solid medium and 0.1 ml of a 10 to 100 $\mu$g/ml solution of the maytansinoid compound (I) in 1% aqueous methanol is then added per ml of the medium, followed by incubation.

Since the present compound (I) has a life-span extending activity in for example, mice, it can also be used as an antitumour agent for treating tumour-bearing, warm-blooded mammalian animals (e.g. mouse, rat, dog or cat).

As an antitumour agent, the compound (I) according to this invention can be administered orally or by other routes. For administration by routes other than the oral route, injections are preferred. Thus, subcutaneous, intraperitoneal, intravenous and intramuscular injections, for instance, may be selectively employed. The dosage may range from 12.5 to 1000 $\mu$g, preferably from 50 to 800 $\mu$g per kg body weight per dose, although it may be increased or decreased according to the condition, animal species and other factors. Such an injection may be prepared, for example, by dissolving from 500 $\mu$g to about 10 mg of the compound (I) in 0.5 ml of alcohol (e.g. methanol or ethanol) and making up the resulting solution with a sufficient amount of physiological saline to a total volume of 10 ml. When the dosage is small, this solution may be further diluted with physiological saline.

We have found that the solubility of the demethylmaytansinoid compound (I) in water is markedly higher than the starting compound (II).

In this specification, the compound of the general formula (I) wherein $R_1 = -COCH(CH_3)_2$ and $X = Cl$ (i.e. 20-demethoxy-20-hydroxymaytansinol 3-isobutyrate) will hereinafter be referred to as PDM-3, the compound (I) in which $R_1 = -CO(CH_2)_2-CH_3$ and $X = Cl$ (i.e. 20-demethoxy-20-hydroxymaytansinol 3-butyrate) will be referred to as PDM-3', and the compound (I) in which $R_1 = -COCH_2-CH(CH_3)_2$ and $X = Cl$ (i.e. 20-demethoxy-20-hydroxymaytansinol 3-isovalerate) will be referred to as PDM-4.

The starting compound (II) wherein $R_1 = -CO-CH(CH_3)_2$ and $X = Cl$ will be called ansamitocin P-3, the compound (II) wherein $R_1 = -CO(CH_2)_2-CH_3$ and $X = Cl$ will be referred to as ansamitocin P-3' and the compound (II) wherein $R_1 = -COCH_2-CH(CH_3)_2$ and $X = Cl$ will be referred to as ansamitocin P-4.

The starting compound (II) may be one of the known maytansines, ansamitocins and other

compounds. Maytansine itself has been investigated by S. M. Kupchan et al as reported in JACS, 94, 1354 (1972). Related maytanside esters have been investigated by Kupchan and co-workers as reported in U.S. Patent No. 3,896,111 and by Kupchan et al in J. Chem. Soc. Chem. Commune, 1065 (1972). A similar disclosure of the investigation of antileukaemic activity in maytanside esters has been made by M. C. Wani and co-workers in J. Chem. Soc. Chem. Commune, 390 (1973). The maytanside esters described, namely, maytansine and other related maytanside esters are examples of starting compounds (II) which may be used to prepare the compounds of the invention. Maytancine and maytansinol propionate can also be produced in the manner described in the Journal of the American Chemical Society *97*, 5294 (1975) or by growing *Nocardia* sp. No. C-15003 (FERM-P No. 3992, IFO 13726, ATCC-31281) in a culture medium and isolating the metabolite from the culture broth [See, e.g., Japanese Patent Application As-Laid-Open No. 121998/1978; and Federal Republic of Germany (DT) Offenlegungsschrift 2746 253]. Ansamitocin P-3, ansamitocin P-3′ and ansamitocin P-4 can be produced by cultivating the above-mentioned *Nocardia* sp. No. C-15003 [See, e.g., Japanese Patent Application As-Laid-Open No. 130693/1978; and Federal Republic of Germany (DT) Offenlegungsschrift 2746 209).

The compound (II) can also be produced by acylating maytansinol or dechloromaytansinol with a carboxylic acid of the formula $R_1OH$ [wherein $R_1$ is as previously defined], or a reactive derivative with respect to the carboxyl function of the carboxylic acid.

Maytansinol, which is used for the production of the compound (II), is a compound known as a plant principle [Kupchan *et al.,* J. Amer. Chem. Soc. *97*, 5294 (1975)], which can also be obtained by the reductive cleavage of a maytansine compound.

Moreover, maytansinol can also be produced by the steps of growing *Nocardia* sp. No. C-15003 (FERM-P No. 3992, IFO 13726, ATCC-31281) in a culture medium to obtain maytanacine, maytansinol propionate or ansamitocins of the formula:

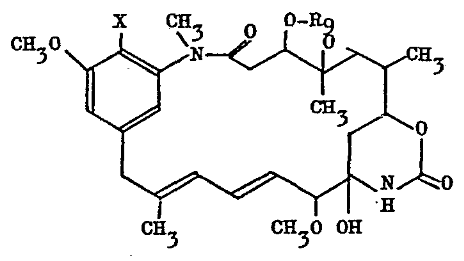

(VII)

[wherein $R_9$ is acetyl, propionyl, iso-butyryl, *n*-butyryl or isovaleryl]; and subjecting the metabolite produced to a reductive ester cleavage reaction with a metal hydride compound, e.g. $LiAlH_4$ [See e.g., Nature, Vol. 270, 721 (1977), or Japanese Patent Application As-Laid-Open No. 130693/1978; and Federal Republic of Germany (DT) Offenlegungsschrift 2746 209].

Dechloromaytansinol can be produced by the reduction of the compound (II) in which X is Cl with a metal hydride compound. The metal hydride compound is preferably a metal complex such as lithium aluminium hydride ($LiAlH_4$), which is used normally in the amount of 1 to 25 moles, preferably about 4 to 10 moles per mole of the starting compound (II) (X = Cl). Normally, this reduction is conducted in a solvent which may, for example, by an ether (e.g. diethylether or tetrahydrofuran), and is preferably, tetrahydrofuran. The reaction can be carried out normally at from −70°C to +80°C, and preferably at from −40°C to +20°C. In many cases, this reaction gives rise to a compound corresponding to the compound (II) (X = Cl) but the 3-acyl group of which has been removed, that is to say maytansinol, as a by-product. After the reduction, the excess reducing agent is destroyed, e.g. by the addition of water, acetic acid or ethyl acetate, and, after the reaction mixture has been made acidic, it is extracted with a suitable solvent (e.g. ethyl acetate). The resulting crude product is then purified, for example by silica gel chromatography or high-pressure liquid chromatography, whereby the desired dechloromaytansinol is obtained.

The maytansinoid compound (II) starting material wherein $R_1$ is acyl, i.e. the compound of the formula (II):

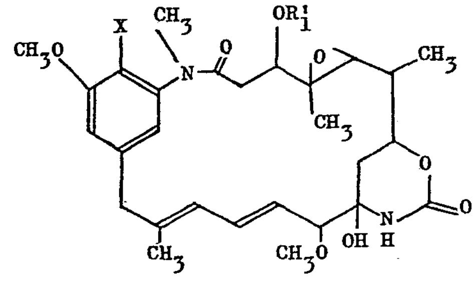

(II)

[wherein X and $R'_1$ have the meanings respectively defined hereinbefore], can be produced by reacting maytansinol or dechloromaytansinol with a carboxylic acid of the formula (VIII):

$$R'_1—OH \qquad (VIII)$$

[wherein $R'_1$ is as defined hereinbefore], or a reactive derivative with respect to the carboxyl function of the carboxylic acid (VIII).

The above acylation may be effected, for example, by reacting maytansinol or dechloromaytansinol with the carboxylic acid (VIII) in the presence of a carbodiimide.

The carboxylic acid (VIII) may be used in a proportion of from 1 to 500 mole equivalents based on maytansinol or dechloromaytansinol and, in many cases, is preferably used in a proportion of from 1 to 30 mole equivalents on the same basis. The carbodiimide may be used in an amount of from 1 to 700 mole equivalents based on maytansinol or dechloromaytansinol, and, in many cases, is preferably used in an amount of from 1 to 50 mole equivalents on the same basis. The carbodiimide is a compound containing a carbodiimide linkage (—N=C=N—) which will be transformed into a urea bond (—NH—CO—NH—) during the acylation reaction. Thus, it may be a compound of the formula (IX):

$$R_{10}—N=C=N—R_{11} \qquad (IX)$$

[wherein $R_{10}$ and $R_{11}$ are organic residues which are capable of permitting the conversion of the carbodiimide linkage into a urea linkage].

As the organic residues $R_{10}$ and $R_{11}$, there may be mentioned cycloalkyl groups having, or not having, di-lower ($C_{1-6}$; the same applied hereinafter) alkylamino groups, lower alkyl groups having, or not having, di-lower alkylamino or morpholino groups and phenyl having, or not having, lower alkyl groups. As the carbodiimide, there may be mentioned dicyclohexylcarbodiimide (DCC), which is in practice preferred. However, diphenylcarbodiimide, di-*o*-tolylcarbodiimide, di-*p*-tolylcarbodiimide, di-tert-butylcarbodiimide, 1-cyclohexyl-3-(2-morpholinoethyl)carbodiimide, 1-cyclohexyl-3-(4-diethylaminocyclohexyl)carbodiimide, 1-ethyl-3-(2-diethylaminopropyl)carbodiimide and 1-ethyl-3-(3-dimethylaminopropyl)carbodiimide are also mentioned by way of example.

The acylation reaction may be carried out in the presence of a suitable solvent. As examples of such solvents there may be mentioned esters (e.g. ethyl acetate), ethers (e.g. diethyl ether, dioxane or tetrahydrofuran), halogenated hydrocarbons (e.g. methylene chloride or chloroform), nitriles (e.g. acetonitrile), aromatic hydrocarbons (e.g. benzene), nitromethane, pyridine, dimethylformamide, dimethylsulfoxide, sulforane and various suitable mixtures thereof.

Normally, this acylation can be conducted at a suitable temperature from a temperature under ice cooling to the reflux temperature of the reaction system.

The acylation reaction can be conducted more advantageously in the presence of a catalyst which will promote the acylation of maytansinol or dechloromaytansinol. The catalyst may, for example, be a base catalyst or an acid catalyst. As examples of the base catalyst, there may be mentioned tertiary amines (such as aliphatic tertiary amines (e.g. triethylamine) and aromatic amines (e.g. pyridine, $\alpha$-, $\beta$- or $\gamma$-picoline, 2,6-lutidine, 4-dimethylaminopyridine, 4-pyrrolidinopyridine, dimethylaniline or diethylaniline)), alkali metal halides (e.g. potassium fluoride or anhydrous potassium iodide) and salts of organic acids (e.g. sodium acetate). As examples of the acid catalyst, there may be mentioned Lewis acids (e.g. anhydrous zinc chloride, anhydrous aluminium chloride ($AlCl_3$), anhydrous ferric chloride, titanium tetrachloride ($TiCl_4$), stannous tetrachloride ($SnCl_4$), antimony pentachloride, cobalt chloride, cupric chloride or boron trifluoride ethoxide), strong inorganic acids (e.g. sulfuric acid, perchloric acid, hydrogen chloride or hydrogen bromide), strong organic acids (e.g. benzenesulfonic acid, *p*-toluenesulfonic acid, trifluoroacetic acid or trichloroacetic acid) and acidic ion-exchange resins (e.g. polystyrene-sulfonic acid). When a carboxylic acid (VIII) having an acyl group $R'_1$ of the formula —CO—$R_2$ (where $R_2$ is as previously defined) is employed in the reaction, it is generally preferred that, as the catalyst, 4-dimethylaminopyridine or 4-pyrrolidinopyridine be employed. When a carboxylic acid (VIII) having an N-acyl-$\alpha$-aminoacyl group ($R'_1$) of the formula:

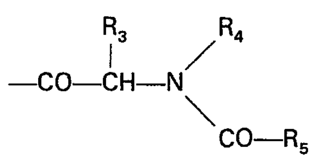

[where $R_3$, $R_4$ and $R_5$ are as previously defined], which is among the said acyl groups, is employed, anhydrous zinc chloride is a preferred catalyst.

The catalyst may be employed in a catalytic amount which will be sufficient to promote the acylation of maytansinol or dechloromaytansinol with the carboxylic acid (VIII). Thus, it is normally used in the range of from 0.001 to 10 mole equivalents, and preferably in the range of from 0.01 to 1 mole equivalents based on the carboxylic acid (VIII). The use of such a catalyst often leads to marked

increases in the yield of the maytansinoid compound (II). It also helps save on the amount of the carboxylic acid (VIII). Thus, the requirement of the carboxylic acid (VIII) may often be reduced to from 1 to 10 mole equivalents based on maytansinol or dechloromaytansinol.

When isomerism exists in the carboxylic acid (VIII), e.g. one having an N-acyl-$\alpha$-aminoacyl group:

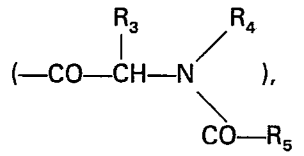

the isomers (e.g. the D- or L-form) of the carboxylic acid (VIII) may be employed either severally or as a mixture of optional proportions.

When an optically active acyl group is to be introduced into the 3-hydroxy function of maytansinol or dechloromaytansinol, it is sometimes useful to employ the corresponding optical isomer of the carboxylic acid (VIII). At other times, the use of an optically active carboxylic acid (VIII) may yield the maytansinoid compound (II) as a mixture of the D- and L-isomers.

The acylation reaction with the reactive derivative of the carboxylic acid (VIII) may, for example, be conducted using a derivative containing a functional group which is able to acylate the 3-position of maytansinol or dechloromaytansinol, e.g. an acid anhydride of the carboxylic acid (VIII). The solvent and catalyst may be the same as those mentioned in connection with the acylation reaction in the presence of a carbodiimide compound. The reaction temperature is normally from –20°C to 100°C, and preferably from 20 to 40°C, although the reaction may be accelerated by being conducted at a higher temperature.

The starting compound (II) in which $R'_1$ is

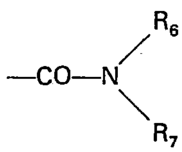

[where $R_6$ and $R_7$ have the meanings respectively defined hereinbefore] can be produced, for example, by carbamoylating maytansinol or dechloromaytansinol.

This carbamoylation may be effected, for example, by reacting maytansinol or dechloromaytansinol with a carbamic acid halide of the formula (X):

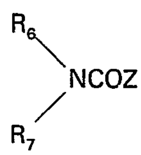

(X),

[wherein $R_6$ and $R_7$ have the meanings respectively defined hereinbefore; and Z is a halogen], in the presence of a base.

In formula (X), the halogen Z may, for example, be chlorine or bromine. As examples of the base just mentioned, there may be cited alkali metals (e.g. lithium or sodium), and alkali metal alkyls (e.g. *n*-butyllithium, sec-butyllithium, phenyllithium, naphthalenelithium or sodium methylsulfinylmethide). *n*-Butyllithium is particularly desirable.

The reaction is generally conducted in a solvent. As examples of the solvent, there may be mentioned ethers (e.g. tetrahydrofuran, dimethylether or diethyl ether), aromatic hydrocarbons (e.g. toluene or xylene) and suitable mixtures of such solvents, although tetrahydrofuran is the most versatile. To conduct the reaction, maytansinol or dechloromaytansinol is dissolved in such a solvent and, after the addition of the alkali metal compound, a carbamic acid halide of the formula (X) is added. The alkali metal compound is desirably employed in a proportion of 3 to 10 mole equivalents and, for still better results, 4 to 6 equivalents based on maytansinol or dechloromaytansinol. The carbamic halide is generally used in a proportion of from 2 to 10 mole equivalents and, preferably, 3 to 5.5 equivalents on the same basis.

The reaction may be carried out at a suitable temperature within the range of from –78°C to +50°C, although it is normally preferable to conduct the reaction at from –30° to +40°C.

After the reaction has been completed, the reaction product, as it is or after neutralization with dilute aqueous acid or after removal of the solvent by distillation, if necessary, can be isolated by conventional procedures as will be described hereinafter. This reaction may in certain cases be conducted with a reactive derivative, with respect to the carboxyl function, of carbamic acid other than the said carbamic acid halide.

An alternative carbamoylation procedure comprises reacting maytansinol or dechloromaytansinol with a compound of the formula (XI):

$$R_6—N=C=O \qquad (XI)$$

[wherein $R_6$ has the same meaning as defined hereinbefore). This reaction procedure yields a maytansinoid compound (II) in which $R_7$ is hydrogen. This reaction is preferably conducted in solution. The solvent may in practice be any solvent free from active hydrogen atom of —OH or —NH—) which is reactive to the compound (XI). Examples of suitable solvents include ethers (e.g. diethyl ether, dimethoxyethane, dioxane or tetrahydrofuran), halogenated hydrocarbons (e.g. dichloromethane, chloroform, carbon tetrachloride or 1,2-dichloroethane), aromatic hydrocarbons (e.g. benzene, toluene, xylene or chlorobenzene), nitriles (e.g. acetonitrile or propionitrile) and esters (e.g. ethyl acetate), as well as suitable mixtures of such solvents. Such a solvent is desirably as dry as possible.

The amount of the compound (XI) necessary for the reaction is one equivalent relative to maytansinol or dechloromaytansinol. However, it is desirable to employ an excess of the compound (XI), for it may be partially consumed by the water contained in the solvent and, in certain cases, by the dimerization or other reaction of the compound (XI) which *per se* is reactive. Thus, it is normally advisable to employ 2 to 20 mole equivalents and, preferably 2 to 5 equivalents. The reaction may be conducted at from −20 to +80°C and, preferably at from 5° to 40°C. In the case of the reactive compound (XI) (normally compounds having nitro or polyhalogen substituents), it may be simply admixed with the mating compound. The use of a catalyst is normally desirable, however. As examples of the catalyst there may be mentioned the catalysts commonly employed in the carbamoylation of alcohols or phenols with isocyanic acid esters, e.g. bases [e.g. tertiary amines (e.g. triethylamine or pyridine)], alkali metal alkoxides (e.g. potassium tert-butoxide or sodium methoxide), alkali metal acetates (e.g. the acetates of lithium, sodium, potassium, rubidium and cesium), metal salts (e.g. chlorides and organic carboxylates of lead, bismuth, tin cobalt, zinc, cadmium, manganese, titanium, iron and copper) and metal complexes or organometallic compounds (e.g. 2,4-pentadiene-metal complexes, forrocenes, dibutyltin oxide, dioctyltin oxide and dibutyltin dilaurate). Among these, anhydrous zinc chloride is used as an especially suitable catalyst for the reaction from the viewpoints of selectivity and reaction rate, for example. The catalyst is used in an amount sufficient to accelerate the reaction; in general, an amount of from 0.01 to 10 moles, and preferably from 0.1 to 3 moles, per mole of compound (XI), is sufficient.

When cuprous chloride is used as the catalyst mentioned above, for example, there may be formed compounds of the formula (XII):

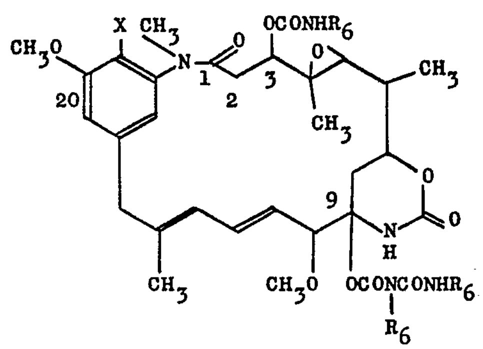

(XII),

[wherein X and $R_6$ have the same meaning as above]. These compounds (XII) can however, be easily converted into compounds (II) which have a hydrogen atom instead of

$$\begin{array}{l}—CON—CONHR_6\\ \quad\ \ |\\ \quad\ R_6\end{array}$$

at the 9-position by treatment with an acid. Acids usable for this reaction include mineral acids (e.g. hydrogen chloride, hydrochloric acid, sulfuric acid or phosphoric acid) or strong organic acids (e.g. benzene-sulfonic, toluenesulfonic, methanesulfonic, trifluoroacetic and trichloroacetic acid). Trifluoroacetic acid is preferred among others, however. Preferably, the reaction is carried out in solution, and the solvent may be the same as that used in the carbamoylation of the above compound (XI). Generally, this reaction proceeds rapidly at a temperature of from −20°C to 40°C. This reaction can also sometimes be effected simply by passing the reaction mixture of the carbamoylation agent containing the compound (XII) through a silica gel column.

The starting compound (II) in which $R_1$ or $R'_1$ is —CO—O—$R_8$ [wherein $R_8$ has the same meaning as defined hereinbefore] can be produced by allowing maytansinol or dechloromaytansinol to react with

an halogenocarbonic ester of the formula:

$$Z'—CO—O—R_8$$

[wherein $R_8$ has the same meaning as defined hereinbefore, and Z' is halogen], in the presence of a base. As examples of halogen, there may be mentioned chlorine and bromine.

The reaction may be conducted in any of the solvents exemplified for the said carbamoylation.

As the base, any of the bases exemplified for carbamoylation can be employed. The base is desirably employed in a proportion of from 3 to 20 mole equivalents and, for still better results, 4 to 10 mole equivalents, based on maytansinol or dechloromaytansinol. The halogenocarbonic ester is used in a proportion of from 3 to 20 mole equivalents and, preferably, from 5 to 10 mole equivalents on the same basis. The reaction may be carried out at a suitable temperature within the range of from −78° to +50°C, although it is normal to conduct the reaction at from −30° to +40°C, preferably from −20° to +30°C.

The maytansinoid compounds (II) of the invention produced by the above methods can be isolated and collected from the reaction mixtures by conventional methods, e.g. by appropriately applying such methods as concentrations, solvent extraction, chromatography or recrystallization. When the compound (II) are produced in the form of a mixture of isomers (e.g. D- and L-isomers), these isomers can generally be separated from each other by separating means known *per se*, e.g. by silica gel column chromatography. The maytansinoid compound (II) of the present invention include these individual isomers as well as mixtures thereof.

The following Reference Examples and Examples are further illustrative of this invention but should not be considered to be limitative of the scope of the invention. "Percent (%)" is based on "weight/volume" unless otherwise noted. Furthermore, "demethyl" stands for the abbreviation of "20-demethoxy-20-hydroxy".

Reference Example 1

99.6 mg. (0.176 mmole) of maytansinol were dissolved in 5 ml of dry dichloromethane, followed by the addition of 377.2 mg. (1.76 mmoles) of hexanoic anhydride (caproic anhydride) and 43.5 mg (0.366 mmole) of *p*-dimethylamino pyridine (DMAP). The mixture was stirred at room temperature (ca. 23°C) for 6 hours, at the end of which time a further 30.5 mg. (0.25 mmole) of DMAP were added. The mixture was stirred at room temperature for an additional 18 hours. 5 ml of 1N-HCl and 5 ml of water were added to the reaction mixture, and the organic layer was then collected, washed with 10% aqueous sodium bi-carbonate (10 ml) and dried over anhydrous sodium sulfate. The solvent was distilled off and the residue was chromatographed on a column of silica gel (75 g) with ethyl acetate (ca. 250 ml) and, then, with ethyl acetate/ethyl acetate saturated with water = 2:1 (V/V) (ca. 900 ml). The eluate was collected in 16 g. fractions and fraction No. 13 to No. 30 inclusive, were pooled and concentrated to remove the solvent, whereby 3.0 mg. of crude product were obtained as a residue. This residue was dissolved in ethyl acetate and, after the addition of diethyl ether, a white powdery solid was recovered by filtration. The above procedure yielded 34.3 mg. of maytansinol 3-hexanoate, m.p. 159—162°C (decomp.).

Reference Example 2

23.5 mg. of maytansinol were dissolved in 1.0 ml of dichloromethane, and, at 22°C, 70.5 mg (ca. 10 mmoles) of acetic-formic anhydride (prepared by cooling 2 ml. of acetic anhydride, adding 1 ml of 99% formic acid under stirring over 10 minutes at −5 to 0°C, heating the mixture at 50°C for 15 minutes and quenching it at 0°C) and 11.7 mg of DMAP were added. The mixture was stirred at room temperature (ca. 22°C) overnight. 10 Drops of methanol were added to this reaction mixture, and, after stirring for 3 hours, the mixture was concentrated to dryness under reduced pressure. The residue was spotted on a silica gel preparative TLC and developed twice with ethyl acetate saturated with water. The silica gel in the zone 6.0 to 8.0 cm above the base line was scraped up and extracted with 10% methanol-dichloromethane. The solvent was then distilled off under reduced pressure to recover 8.35 mg. of maytansinol 3-formate as a colourless glassy residue.

Reference Example 3

The following compounds can be prepared by a similar procedure to that described in Reference Example 1 or 2:

(A) Maytansinol 3-octanoate is obtained from maytansinol and octanoic anhydride (caprylic anhydride) as a white sandy solid melting at 151—160°C (decomp.).

(B) Maytansinol 3-decanoate is obtained from maytansinol and decanoic acid (capric acid) as a white sandy solid melting at 130—134°C (decomp.).

(C) Maytansinol 3-heptanoate is obtained from maytansinol and heptanoic acid m.p. 158—160°C (decomp.).

(D) Maytansinol 3-tridecanoate is obtained from maytansinol and tridecanoic acid, m.p. 110—116°C (decomp.).

(E) Maytansinol 3-hexadecanoate is obtained from maytansinol and hexadecanoic acid (palmitic acid) as a white powder melting at 105—116°C (decomp.).

(F) Maytansinol 3-valerate is obtained from maytansinol and valeric anhydride, m.p. 165—168°C.

Reference Example 4

DCC (267 mg, 1.296 mmoles) was added to a mixed solution of maytansinol (103.2 mg. 0.183 mmole) and cyclohexanecarboxylic acid (140 mg, 1.094 mmoles) in 5 ml of dry dichloromethane, and, after stirring at room temperature for a short while until insolubles began to separate out, DMAP (50.8 mg, 0.416 mmole) was added. The mixture was stirred at room temperature overnight. The insolubles were filtered off, and the filtrate was successively washed with 0.5N-HCl (ca. 10 ml) and saturated aqueous sodium bicarbonate (ca. 10 ml), and dried over anhydrous sodium sulfate. The solvent was distilled off and the residue was chromatographed on a column of silica gel (75 g.). Elution was carried out with ethyl acetate, the eluate being collected in 16 g. fractions, and fractions No. 14 to No. 30 inclusive, were pooled and concentrated to remove the solvent, whereupon 59 mg of crude product were obtained. This crude product was dissolved in ethyl acetate, followed by the addition of diethyl ether. This procedure yielded 24.3 mg of maytansinol 3-cyclohexanecarboxylate as crystals melting at 202—206°C (decomp.).

Reference Example 5

The following compounds can be produced by a similar procedure to that described in Reference Example 4:

(A) From maytansinol and cyclopropanecarboxylic acid, there is obtained maytansinol 3-cyclopropanecarboxylate, m.p. 182—187°C (decomp.).

(B) From maytansinol and phenylacetic acid, there is obtained maytansinol 3-phenylacetate, m.p. 180—182°C (decomp.).

(C) From maytansinol and benzoic acid, there is obtained maytansinol 3-benzoate, m.p. 174—177°C (decomp.).

(D) From maytansinol and p-chlorobenzoic acid, there is obtained maytansinol and 3-p-chlorobenzoate, m.p. 178—183°C (decomp.).

(E) From maytansinol and 2-furancarboxylic acid, there is obtained maytansinol 3-(2-furan)carboxylate, m.p. 180—189°C (decomp.).

(F) From maytansinol and phenylpropionic acid, there is obtained maytansinol 3-phenylpropionate, m.p. 160—163°C (decomp.).

(G) From maytansinol and nicotinic acid, there is obtained maytansinol 3-nicotinate as a white powder melting at 184—187°C (decomp.).

(H) From maytansinol and picolinic acid, there is obtained maytansinol 3-picolinate, m.p. 190—193°C (decomp.).

(I) From maytansinol and isonicotinic acid, there is obtained maytansinol 3-isonicotinate as white crystals melting at 185—187°C (decomp.).

(J) From maytansinol and N-acetyl-L-proline, there is obtained maytansinol 3-(N-acetyl)-L-prolinate, both as white crystals melting at 195—198°C (decomp.) and as a glassy product with an UV spectrum ($\lambda_{max}^{MeOH}$) nm: 233, 244, 253, 282 and 292.

(K) From maytansinol and 2-thiophenecarboxylic acid, there is obtained maytansinol 3-(2-thiophene)carboxylate as a glassy solid melting at 161—163°C (decomp.).

Reference Example 6

15.0 g of the antibiotic ansamitocin mixture (ansamitocin P-2 = 12%); ansamitocin P-3 = 71%; ansamitocin P-4 = 17%) were dissolved in 800 ml of dry tetrahydrofuran (THF), and the resulting solution was chilled to −50°C in dry gaseous nitrogen by means of a Dry-Ice-ethanol bath. 13.0 g of lithium aluminium hydride (LAH) were added at one time and the resulting mixture was stirred at from −50°C to −22°C for 2 hours. The mixture was cooled to −28°C and an additional 3 g. of LAH were added. The mixture was stirred at from −28°C to −22°C for 80 minutes, followed by cooling again to −50°C. Thereafter, 750 ml of 2N-HCl were carefully added dropwise over 30 minutes. The reaction mixture was extracted three times with 2.6 l, 1.6 l and 0.8 l portions of ethyl acetate, and the extracts were combined, washed with saturated aqueous sodium chloride (100 ml x 2) and dried (250 g $MgSO_4$). The solvent was distilled off under reduced pressure and the residue (13.6 g) was chromatographed on a column of silica gel (1.2 kg), elution being carried out with ethyl acetate/water = 98.5:1.5 (V/V). The eluate was collected in 400 g fractions and fractions No. 35 to No. 52, inclusive, were pooled. The solvent was distilled off and the residue was dried *in vacuo* to recover 7.25 g of maytansinol. The same procedure yielded 1.55 g of an approximately equimolar mixture of maytansinol and dechloromaytansinol from fractions No. 53 to No. 68, inclusive. Similarly, 0.78 g of dechloromaytansinol was recovered from fractions No. 69 to No. 86, inclusive. Recrystallization from chloroform-hexane yielded 0.71 g of dechloromaytansinol as a white powder. m.p. 174—179°C (decomp.).

Reference Example 7

100.0 mg (0.189 mmole) of dechloromaytansinol were dissolved in 15 ml of dry dichloromethane, followed by the successive addition of 69 mg (0.476 mmole) of N-acetyl-N-methyl-L-alanine, 117 mg (0.568 mmole) of DCC and 39 mg (0.287 mmole) of anhydrous zinc chloride. The resulting mixture was stirred at room temperature (ca. 23°C) for 30 minutes, after which time a further 55 mg (0.379 mmole) of N-acetyl-N-methyl-L-alanine, a further 98 mg (0.476 mmole) of DCC and a further 31 mg (0.228 mmole) of anhydrous zinc chloride were added. The mixture was stirred again at room temperature for 2 hours. The insolubles were filtered off, the filtrate was concentrated to dryness and the resulting residue was dissolved in about 5 ml of ethyl acetate and chromatographed on a column of silica gel (25 mm out. dia x 500 mm). Elution was successively carried out with ethyl acetate/ethyl acetate saturated with water = 2:1 (V/V) and ethyl acetate saturated with water, and the eluate was collected in 15 g fractions. Fractions No. 55 to No. 103, inclusive, were pooled and the solvent was distilled off, whereupon 53 mg of crude dechloromaytansine were obtained. This residue was dissolved in ethyl acetate and diethyl ether was added, followed by cooling. The above procedure yielded 24 mg of L-dechloromaytansine as colourless crystals. m.p. 184—186°C (decomp.).

The chromatographic fractions No. 168 to No. 221, inclusive, were pooled and the solvent was distilled off to recover 65 mg of D-dechloromaytansine. This residue was dissolved in chloroform, diethyl, ether was added, and the resulting crystals were recovered by filtration. The above procedure yielded 21 mg of D-dechloromaytansine as colourless microcrystals. m.p. 175—178°C (decomp.).

Reference Example 8

The following compounds can be produced in a similar manner to Reference Example 7.

(A) From dechloromaytansinol and isobutyric anhydride, there is obtained dechloromaytansinol 3-isobutyrate as white prisms melting at 250—252°C (decomp.).

(B) From dechloromaytansinol and nicotinic acid, there is obtained dechloromaytansinol 3-nicotinate, m.p. 170—173°C (decomp.).

(C) From dechloromaytansinol and cyclohexanecarboxylic acid, there is obtained dechloromaytansinol 3-cyclohexane-carboxylate, m.p. 217—220°C (decomp.).

(D) From dechloromaytansinol and phenylacetic acid, there is obtained dechloromaytansinol 3-phenylacetate, m.p. 165—170°C (decomp.).

Reference Example 9

150.0 mg (0.265 mmole) of maytansinol were dissolved in 30 ml of dry dichloromethane, followed by the addition of 24.0 mg (0.663 mmole) of N-acetyl-N-methyl-L-leucine and 174.2 mg (0.846 mmole) of DCC. The resulting mixture was stirred at room temperature for a while, after which 46 mg (0.338 mmole) of anhydrous zinc chloride were added. After mixing at room temperature for 30 minutes, an additional 46 mg of anhydrous zinc chloride were added and the mixture was further stirred at the same temperature for about 45 minutes. Then, a further 104.3 mg (0.558 mmole) of N-acetyl-N-methyl-L-leucine, 141 mg (0.686 mmole) of DCC and 46 mg of anhydrous zinc chloride were added, followed by stirring at that temperature for another 2.5 hours. The reaction mixture was washed with water, the organic layer was dried over $Na_2SO_4$ and the solvent was then distilled off. The residue was chromatographed on a column of silica gel (75 g.) and elution was successively carried out with ethyl acetate (ca. 600 ml) and ethyl acetate saturated with water, the eluate being collected in 17 g fractions. Fractions Nos. 14 to 34, inclusive, were pooled, the solvent was distilled off and the residue (100 mg) was rechromatographed on a column of silica gel (35 g) (solvent system: chloroform/methanol = 60:1 (V/V), the eluate being collected in 25 g fractions. Fractions Nos. 16 to 30, inclusive, were pooled, the solvent was distilled off and the residue was dissolved in ethyl acetate. After cooling, the resulting crystals were recovered by filtration. This procedure yielded 89 mg of compound *A*. The initial chromatographic fractions Nos. 35 to 56 were pooled, the solvent was distilled off and the residue was rechromatographed on silica gel (40 g) [solvent system: chloroform/methanol = 60:1 (V/V) (ca. 200 ml), *do.* 40:1 (1 l)], the eluate being collected in 25 g fractions. Fractions Nos. 17 to 35 were pooled, the solvent was distilled off, the residue was dissolved in ethyl acetate and, after the addition of diethyl ether, the precipitate was recovered by filtration. This procedure yielded 52 mg of compound *B*.

Both compounds A and B are the desired compounds, and, based on the following physical data, are considered to be maytansinol 3-(N-acetyl-N-methyl)-L-leucine ester and maytansinol 3-(N-acetyl-N-methyl)-D-leucine ester, respectively.

Compound A: m.p. 172—175°C (decomp.)
Compound B: m.p. 157—159°C (decomp.)

Reference Example 10

The following compounds can be produced in the same manner as Reference Example 9.

(A) From maytansinol and N-acetyl-N-benzyl-D-alanine, there are obtained two maytansinol 3-(N-acetyl-N-benzyl) alanine esters only dissimilar with respect to the stereo-chemical arrangement of the 2′-substituent: m.p. 174—177°C (decomp.); and 163—166°C (decomp.), respectively.

(B) From maytansinol and N-acetyl-N-methyl-L-phenylalanine, there are obtained two maytansinol 3-(N-acetyl-N-methyl)-phenylalanine esters only dissimilar with respect to the stereochemical arrangement of the 2'-substituent: m.p. 189—193°C (decomp.); and 212—214°C (decomp.), respectively.

(C) From maytansinol and N-tert-butoxycarbonyl-N-methyl-L-alanine, there are obtained maytansinol 3-(N-tert-butyloxy-carbonyl-N-methyl-L-alanine) ester [UV spectrum ($\lambda_{max}^{MeOH}$) nm: 234, 244, 254, 282, 290)] and maytansinol 3-(N-tert-butyl-oxycarbonyl-N-methyl-D-alanine) ester [UV spectrum ($\lambda_{max}^{MeOH}$) nm: 234, 241 (sh.), 253.5, 282, 290.]

(D) From maytansinol and N-acetylsarcosine, there is obtained maytansinol 3-(N-acetyl)sarcosine ester as a glassy product.

NMR spectrum ($CDCl_3$) $\delta$ ppm:

0.87 (3H, s), 1.28 (3H, d, J = 5Hz), 1.68 (3H, s), 2.14 (3H, s), 2.19 (1H, dd, J = 3Hz & 14Hz), 2.55 (1H, dd, J = 11Hz & 14Hz), 2.76 (1H, d, J = 9Hz), 3.07 (2H, s), 3.13 (3H, s), 3.18 (3H, s), 3.35 (3H, s), 3.47 (1H d, J = 9Hz), 3.52 (1H, d, J = 13Hz), 3.98 (3H, s), 4.18 (1H, m), 4.92 (1H, dd, J = 3Hz & 11Hz), 5.74 (1H, dd, J = 9Hz & 15Hz), 6.18 (1H, d, J = 11Hz), 6.44 (1H, dd, J = 11Hz & 15Hz), 6.53 (1H, s), 6.82 (2H, s), etc.

(E) From maytansinol and N-acetylglycine, there is obtained maytansinol 3-(N-acetyl)-glycine ester, m.p. 189—192°C (decomp.).

Reference Example 11

In 80 ml of dichloromethane were dissolved maytansinol (300 mg, 0.5315 mmole) and N-acetyl-N-methyl-L-alanine (1.585 g, 10.62 mmoles), followed by the addition of 3.285 g of dicyclohexylcarbodiimide and 72.5 mg (0.532 mmole) of anhydrous zinc chloride. The mixture was stirred at about 20°C for 6 hours and then allowed to stand at that temperature for 11 hours. Further N-acetyl-N-methyl-L-alanine (530 mg), dicyclohexylcarbodiimide (1095 mg) and anhydrous zinc chloride (150 mg) was then added. After 2 hours, the reaction mixture was filtered and the filtrate was washed with about 150 ml of water and dried over anhydrous sodium sulfate. The insolubles were filtered off and the filtrate was chromatographed on a column of silica gel (60 g), elution being carried out with chloroform/methanol = 40:1 (V/V). After the forerun had been discarded, the eluate was collected in 25 g fractions.

Fractions Nos. 14 to 25 were pooled, concentrated and rechromatographed on silica gel (65 g), elution being carried out with ethyl acetate/ethyl acetate saturated with water = 2:1 (V/V). The forerun was discarded and the subsequent eluate was collected in 16 g fractions. Fractions Nos. 25 to 60 yielded 149.3 mg of compound *A*. The rechromatographic fractions Nos. 23 and 24 and Nos. 61 to 100 were pooled and concentrated to dryness to recover 20.5 mg of product. This product was subjected to preparative silica gel TLC (Kieselgel $60F_{254}$, Art. 5717, Merck), and the chromatogram was developed with 10% isopropyl alcohol-chloroform to recover an additional 6.3 mg of Compound *A*. The above rechromatographic fractions No. 101 to 105 were also pooled and concentrated to yield 320 mg of product. This product was rechromatographed on a column of silica gel (75 g) (solvent system: chloroform/methanol = 40:1 (V/V) to recover 95.7 mg of compound *B*, which was an isomer of compound *A*.

The total yield of compound *A* was 155.6 mg and that of compound *B* was 95.7 mg.

Compound A was identified with maytansine (L-form) obtained by plant extraction through a comparison of the following data with the data on the maytansine obtained by plant extraction, as given in the Journal of Organic Chemistry *42*, No. 14, 2349—2357 (1977). UV spectrum ($\lambda$ max, EtOH) nm: 289, 281, 254, 242 (sh.), 233 Mass spectrum (m/e): 691, 630, 485, 470, 450, 128, 100, 58 $[\alpha]_D^{23}$ −136° ± 30° (c = 0.055, $CHCl_3$)

Compound *A* was dissolved in a mixture of ethyl acetate and diethyl ether and allowed to stand, whereupon crystals separated out. These crystals were recrystallized once from ethyl acetate-diethyl ether and, then, twice from dichloromethane-diethyl ether. The above procedure yielded compound *A* as colourless plates melting at 191—195°C (decomp.).

Compound *B* was identified with an isomer of maytansine and assumed to be D-maytansine by comparison of the following data with the data on maytansine.

UV spectrum ($\lambda$ max, EtOH) nm: 289, 281, 253, 240 (sh.), 233 Mass spectrum (m/e): 691, 630, 485, 470, 450, 128, 100, 58 $[\alpha]_D^{23}$ −129°±30° (c = 0.055, $CHCl_3$)

A solution of compound *B* in chloroform was treated with diethyl ether to yield crystals which were then recrystallized twice from the same solvent system. This procedure yielded compound *B* as crystals melting at 155—178°C (gradually decomposed.)

Reference Example 12

The following compounds can be produced in the same manner as Reference Example 11.

(A) From maytansinol and N-methyl-N-propionyl-L-alanine, there are obtained natural type (L-form) maytanprine [colourless needles, m.p. 185—189°C (slightly decomp.)] and D-maytanprine [colourless needles, m.p. 192—197°C (decomp.)].

(B) From maytansinol and N-isobutyryl-N-methyl-L-alanine, there are obtained natural type (L-form) maytanbutine [colourless needles, m.p. 185—187°C (decomp.)] and D-maytanbutine [colourless

needles, m.p. 195—198°C (decomp.)].

(C) From maytansinol and N-isovaleryl-N-methyl-L-alanine, there are obtained the natural type (L-form) maytanvaline and D-maytanvaline:

*Natural-type maytanvaline*

NMR spectrum ($CDCl_3$) $\delta$:

0.79 (3H, s), 0.91 (3H, d, J = 6Hz), 0.95 (3H, d, J = 6Hz), 1.27 (3H, d, J = 6Hz), 1.30 (3H, d, J = 7Hz), 1.64 (3H, s), 2.13 (2H, d, J = 7Hz), 2.15 (1H, dd, J = 14Hz & 3Hz), 2.60 (1H, dd, J = 14Hz & 11Hz), 2.83 (3H, s), 3.00 (1H, d, J = 9Hz), 3.07 (1H, d, J = 13Hz), 3.17 (3H, s), 3.34 (3H, s), 3.47 (1H, d, J = 9Hz), 3.59 (1H, br.), 3.65 (1H, d, J = 13Hz), 3.95 (3H, s), 4.27 (1H, m), 4.74 (1H, dd, J = 12Hz & 3Hz), 5.35 (1H, q, J = 7Hz), 5.64 (1H, dd, J = 15Hz & 9 Hz), 6.28 (1H, br.s), 6.39 (1H, dd, J = 15Hz & 11Hz), 6.67 (1H, d, J = 2Hz), 6.69 (1H, d, J = 11Hz), 6.79 (1H, d, J = 2Hz), 0.7—2.0 (3H).

Mass spectrum (m/e): 733, 672, 485, 470, 450, 170

*D-maytanvaline*

NMR spectrum ($CDCl_3$) $\delta$:

0.89 (3H, s), 0.93 (3H, d, J = 6Hz), 0.96 (3H, d, J = 6Hz), 1.26 (3H, d, J = 4Hz), 1.49 (3H, d, J = 7Hz), 1.69 (3H, s), 2.66 (1H, dd, J = 15Hz & 12Hz), 3.02 (3H, s), 3.12 (3H, s), 3.18 (1H, d, J = 13Hz), 3.32 (3H, s), 3.42 (1H, d, J = 9Hz), 3.50 (1H, d, J = 13Hz), 3.96 (3H, s), 4.29 (1H, m), 4.92 (1H, dd, J = 11Hz & 3Hz), 5.00 (1H, q, J = 7Hz), 5.05 (1H, br.), 5.78 (1H, dd, J = 15Hz & 9Hz), 6.17 (1H, d, J = 11Hz), 6.24 (1H, s), 6.43 (1H, dd, J = 15Hz & 11Hz), 6.77 (1H, d, J = 1.5Hz), 6.83 (1H, d, J = 1.5Hz), 0.8—2.5 (7H).

Mass spectrum (m/e): 733, 672, 485, 470, 450, 170

Reference Example 13

(i) In 600 ml of methanol were suspended 53.5 g (0.52 mole) of N-methyl-L-alanine, and, under ice-cooling and stirring, 76 g of dry hydrogen chloride gas were dissolved. The suspension of the starting material was liquidised during the course of the reaction, and, after stirring at room temperature overnight, a homogeneous solution was obtained. After 85 g (0.8 mole) of methyl orthoformate had been added, the reaction mixture was further allowed to stand at room temperature for 24 hours. The minor amounts of insolubles were filtered off and the filtrate was concentrated under reduced pressure. By the above procedure there was obtained the hydrochloride of N-methyl-L-alanine methyl ester as a solid product.

NMR spectrum (in DMSO-$d_6$) $\delta$:

1.50 (3H, d, J = 7Hz), 2.60 (3H, m; after addition of $D_2O$, s), 3.75 (3H, s), 4.12 (1H, m; after addition of $D_2O$, q, J = 7Hz), 9.83 (2H, br.)

(ii) In 300 ml of chloroform there were dissolved 33.7 g (0.22 mole) of N-methyl-L-alanine methyl ester hydrochloride, followed by the addition of 65 ml of acetic anhydride and 110 ml of triethylamine. The mixture was allowed to stand at room temperature for 24 hours and, after the excess acetic anhydride had been decomposed with water, was neutralized with sodium bicarbonate. The chloroform layer was separated, the water layer was extracted with ethyl acetate (120 ml x 5), and the chloroform and ethyl acetate layers were combined and concentrated under reduced pressure. The brown oil thus obtained was dissolved in chloroform, washed with aqueous sodium bicarbonate and concentrated under reduced pressure. The above procedure yielded 31.8 g of N-acetyl-N-methyl-L-alanine methyl ester as a brown oil.

NMR spectrum ($CDCl_3$) $\delta$:

1.38 (3H, d, J = 7Hz), 2.12 (3H, s), 2.97 (3H, s), 3.70 (3H, s), 5.23 (1H, q, J = 7Hz)

The ester obtained above was dissolved in a mixture of 100 ml methanol and 170 ml 1N-aqueous sodium hydroxide. The solution was allowed to stand at room temperature for 2 hours, after which time the methanol was removed under reduced pressure. The aqueous alkaline solution was extracted with chloroform. The water layer was brought to pH 1 with concentrated hydrochloric acid under ice-cooling and extracted with ethyl acetate (140 ml x 5). The extract was dried over anhydrous sodium sulfate and concentrated under reduced pressure to recover a white solid. Recrystallization from ethyl acetate-hexane yielded 8.1 g of colourless needles of N-acetyl-N-methyl-L-alanine.

$[\alpha]_D^{25}$ −58.5° (c = 1, DMF)

−74.3° (c = 1, $H_2O$)

m.p. 121—122°C

(iii) The following compounds can be produced in the same manner as above.

(A) From N-methyl-L-alanine methyl ester . HCl and propionic anhydride, there is obtained N-methyl-N-propionyl-L-alanine (colourless prisms), m.p. 108—110°C.

(B) From N-methyl-L-alanine methyl ester . HCl and isobutyryl chloride, there is obtained N-isobutyryl-N-methyl-L-alanine (colourless prisms), m.p. 117—118°C.

(C) From N-methyl-L-alanine methyl ester . HC and isovaleryl chloride, there is obtained N-methyl-N-isovaleryl-L-alanine (colourless scales), m.p. 88—89°C.

Reference Example 14

(i) Production of β-methoxycarbonylethyl isocyanate

In 600 ml of dry toluene there were dissolved 26.4 g of monomethyl succinate, followed by the addition of 55 g of diphenylphosphorylazide and 22 g of triethylamine. The mixture was allowed to stand at room temperature with stirring for 3 hours, after which time it was washed with water and dried. The solvent was concentrated to about one-third of its original volume and, finally, the mixture was refluxed for 2 hours. After the solvent had been completely evaporated off, the residue was distilled under reduced pressure. By the above procedure there were obtained 13.6 g of the above-indicated compound. b.p.$_8$: 64—66°C (b.p.$_8$ = b.p. under 8mmHg)

(ii) Production of 5-dimethylaminopentyl isocyanate.

In 136 ml of ethanol there were dissolved 23.5 g of methyl 6-N,N-dimethylaminocaproate and 10.2 g of hydrazine hydrate, and the solution was allowed to stand under reflux overnight. To the reaction mixture was added an excess of ethanolic oxalic acid and the resulting white precipitate was recovered by filtration and extracted with 300 ml of 50% aqueous ethanol while hot. After cooling, some insoluble matter was removed and the filtrate was concentrated to dryness, whereupon 23.3 g of white powder were obtained. The entire amount of this powdery residue was suspended in 136 ml of water and treated under cooling with 12.3 g of sodium nitrite. The reaction mixture was adjusted to pH 10.5 with 5N-sodium hydroxide and extracted three times with 150 ml of benzene each. The benzene layers were combined, washed with water, dried and kept under reflux for an hour. The solvent was carefully evaporated off and the residue was distilled under reduced pressure. By the above procedure there were obtained 6.1 g of the above-indicated compound, b.p.$_{14}$: 110—115°C (b.p.$_{14}$ = boiling point under 14mm Hg).

Reference Example 15

In 10 ml of dry dichloromethane there were dissolved 56 mg (0.099 mmole) of maytansinol, followed by the addition of 24 mg (0.202 mmole) of phenyl isocyanate. At room temperature (18—23°C), 30 mg (0.221 mmole) of anhydrous zinc chloride were added and the resulting mixture was stirred at that temperature for 3 hours. The reaction mixture was washed with water, dried ($Na_2SO_4$) and concentrated. The residue was chromatographed on silica gel [solvent system: ethyl acetate/ethyl acetate saturated with water = 4:1 (V/V) through 3:1 (V/V)], the eluate being collected in 17 g fractions. Fractions Nos. 9 to 17 were pooled and the solvent was distilled off. By the above procedure there were obtained 58 mg of maytansinol 3-N-phenylcarbamate.
m.p. 187—189°C (recrystallized from ethyl acetate-hexane)

Reference Example 16

In 10 ml of dichloromethane there were dissolved 54 mg (0.0956 mmole) of maytansinol, followed by the addition of 50 mg (0.877 mmole) of methyl isocyanate and 30 mg of cuprous chloride. The mixture was stirred at room temperature for 4 hours, after which time it was filtered and concentrated. The residue was chromatographed on a column (25 mm dia. x 45 cm long) of silica gel and elution was carried out with chloroform/methanol = 40:1 (V/V), the eluate being collected in 25 g fractions. Fractions Nos. 34 to 44 were pooled and concentrated to dryness to yield 44 mg of a white glassy product. This product was reprecipitated from chloroform-hexane. By the above procedure there were obtained 28 mg of maytansinol 3-(N-methyl)carbamate 9-(2,4-dimethyl)-allophanate as a white powder. m.p. 149—151°C (decomp.)

In 0.2 ml of dichloromethane there were dissolved 10 mg of maytansinol 3-(N-methyl)carbamate 9-(2,4-dimethyl)allophanate, followed by the addition of 2 drops of trifluoroacetic acid. The mixture was stirred at room temperature for 5 minutes and, after a further amount of dichloromethane had been added, it was washed with aqueous sodium bicarbonate. The solvent was distilled off and the residue was chromatographed on 12 g of silica gel. By the above procedure there were obtained 5.2 mg of maytansinol 3-(N-methyl)carbamate.

Reference Example 17

The following compounds can be produced in the same manner as Reference Examples 14 to 16.

(A) From maytansinol and methyl isocyanate, there is obtained maytansinol 3-(N-methyl)carbamate, m.p. 196—200°C (decomp.).

(B) From maytansinol and butyl isocyanate, there is obtained maytansinol 3-(N-butyl)carbamate, m.p. 162—165°C.

(C) From maytansinol and octadecyl isocyanate, there is obtained maytansinol 3-(N-octadecyl carbamate, m.p. 105—109°C).

(D) From maytansinol and cyclohexyl isocyanate, there is obtained maytansinol 3-(N-cyclohexyl) carbamate, m.p. 175—178°C.

(E) From maytansinol and $\alpha$-naphthyl isocyanate, there is obtained maytansinol 3-(N-$\alpha$-naphthyl) carbamate, m.p. 172—175°C.

(F) From maytansinol and p-ethoxyphenyl isocyanate, there is obtained maytansinol 3-(N-p-ethoxyphenyl) carbamate, m.p. 221—223°C.

(G) From dechloromaytansinol and phenyl isocyanate, there is obtained dechloromaytansinol 3-(N-phenyl) carbamate as a colourless glassy product.
NMR spectrum ($CDCl_3$) $\delta$ ppm:
0.87 (3H, s), 1.26 (3H, d, J = 6Hz), 1.70 (3H, s), 2.03 (3H, s), 2.23 (1H, dd, J = 2.5Hz & 14Hz), 2.69 (1H, dd, J = 11Hz & 14Hz), 2.87 (1H, d, J = 9Hz), 3.23 (3H, s), 3.30 (3H, s), 3.42 (1H, d, J = 9Hz), 3.49 (1H, d, J = 14Hz), 3.85 (3H, s), 4.30 (1H, m), 4.78 (1H, dd, J = 2.5Hz & 11Hz), 5.37 (1H, dd, J = 9Hz & 15Hz), 6.10 (1H, d, J = 10.5Hz), 6.39 (1H, s), 6.43 (1H, dd, J = 10.5Hz & 15Hz), 6.57—7.56 (ca. 9H, m), etc.

(H) From maytansinol and isopropyl isocyanate, there is obtained maytansinol 3-N-isopropyl-carbamate. Mass spectrum, (m/e): 588 ($M^+$ −61).

(I) From maytansinol and 3-pyridylisocyanate, there is obtained maytansinol 3-(N-m-pyridyl) carbamate. Mass spectrum (m/e), 623 ($M^+$ −61).

(J) From maytansinol and 5-dimethylaminopentyl isocyanate, there is obtained maytansinol 3-(N-5-dimethylaminopentyl)-carbamate. Mass spectrum (m/e), 659 ($M^+$ −61).

(K) From maytansinol and $\beta$-methoxycarbonylethyl isocyanate, there is obtained maytansinol 3-(N-$\beta$-methoxycarbonylethyl)-carbamate. Mass spectrum (m/e), 632 ($M^+$ −61).

(L) From maytansinol and N,N-dimethylcarbamoyl chloride, there is obtained maytansinol 3-N,N-dimethylcarbamate.
Rf = 0.39 (solvent: chloroform/methanol = 95:5); mass spectrum (m/e), 574 ($M^+$ −61).

## Reference Example 18

A solution of 57 mg of maytansinol in 2.0 ml of dry tetrahydrofuran was treated with 5 molar equivalents of *n*-butyllithium (15% solution in hexane) at −20°C in a stream of nitrogen. 61 mg of isopropyl chloroformate were added and the resulting mixture was stirred for 15 minutes at the same temperature. The reaction mixture was then warmed up to 0°C, treated with 0.5 ml of a saturated aqueous solution of sodium chloride and 2.0 ml of tetrahydrofuran. The organic layer was separated off and dried, and the solvent was evaporated. The residue was chromatographed on a silica gel column to give 5 mg of maytansinol 3-isopropyl-carbonate.

Rf value in a silica gel thin-layer chromatography with chloroform/methanol = 95/5 (V/V) on a precoated plate (Art. 5642, Merck, Germany).
Mass spectrum (m/e): 650 ($M^+$), 589 ($M^+$ −61)

## Reference Example 19

A solution of dechloromaytansinol (53 mg) in 2.0 ml of dry tetrahydrofuran was treated as in Reference Example 18 with 10 mole equivalent of *n*-butyllithium. 10 molar equivalents of benzyl chloroformate were then added as a 30% solution in toluene. After 15 minutes' stirring at the same temperature, the reaction mixture was warmed up to 0°C and treated with 0.5 ml of a saturated aqueous solution of sodium bicarbonate. The organic layer was separated and dried and the solvent was evaporated. The residue was chromatographed as in Reference Example 18 to give 23 mg of dichloromaytansinol 3-benzylcarbonate.

Rf value (conditions are the same as in Reference Example 18) = 0.54,
Mass spectrum (m/e): 603 ($M^+$ −61)

## Reference Example 20

The following compound can be produced in the same manner as Reference Example 18 or 19.

(A) From maytansinol and *n*-octyl chloroformate, there is obtained maytansinol 3-n-octyl-carbonate. Thin layer chromatography, silica gel (E Merck, HPTLC), Rf = 0.61 (developing solvent: chloroform:methanol = 95:5, Mass spectrum (m/e): 659 ($M^+$ −61).

(B) From maytansinol and phenyl chloroformate, there is obtained maytansinol 3-phenylcarbonate. Thin layer chromatography, silica gel (E. Merck, HPTLC), Rf = 0.45 (developing solvent: chloroform:methanol = 95:5);
Mass spectrum (m/e): 623 ($M^+$ −61).

## Reference Example 21

Maytansinol, maytanacine and maytansinol propionate producible *Nocardia* sp. No. C-15003 (IFO 13726; FERM-P No. 3992, ATCC-31281) as grown on a slant medium (yeast extract malt extract agar) was used to inoculate a 200 ml conical flask containing 40 ml of a seed culture medium (2% glucose, 3% soluble starch, 1% raw soybean meal, 1% corn steep liquor, 0.5% Polypepton (trade mark), 0.3% NaCl, 0.5% $CaCO_3$, pH 7.0). The inoculated medium was incubated at 28°C on a rotary shaker for 48 hours to obtain an inoculum. A 0.5 ml portion of the inoculum thus obtained was transferred to a 200 ml conical flask containing 40 ml of a fermentation medium composed of 5% dextrin, 3% corn steep

liquor, 0.1% polypepton and 0.5% $CaCO_3$ (pH 7.0), and cultivated on a rotary shaker at 28°C for 90 hours to give seed culture.

As determined by the serial dilution method using *Tetrahymena pyriformis* W as an assay organism and maytansinol propionate as the standard product, the production potency of the above culture was found to be 25 μg/ml. Polypepton is a product of Daigo Nutritive Chemicals Ltd., Japan.

Reference Example 22

A 10 ml portion of the seed culture obtained in Reference Example 21 was transferred to a 2 l Sakaguchi flask containing 500 ml of a seed culture medium and incubated on a rotary shaker at 28°C for 48 hours. A 500 ml portion of the resulting culture was transferred to a 50 l tank of stainless steel containing 30 l of seed culture medium and cultivated for 48 hours at 28°C, 30 l/min. aeration, 280 r.p.m. (1/2DT) and 1 kg/cm² internal pressure to obtain a seed culture. This culture was used to seed a 200 l tank of stainless steel containing 100 l of a fermentation medium similar to that used in Reference Example 21 at an inoculation rate of 10%. The inoculated medium was incubated for 90 hours at 28°C, 100 l/m. aeration, 200 r.p.m. (1/2DT) and 1 kg/cm² internal pressure. As determined by the same procedure as that described in Reference Example 21, the production potency of the culture obtained above was found to be 25 μg/ml.

To 90 l of the culture obtained above were added 2kg of Hyflo Super Cel (trade mark) (Johns Manville Products, U.S.A.), and, after thorough mixing, the resulting mixture was filtered on a pressure filter to yield 85 l of filtrate and 32 kg of moist cells. The filtrate (85 l) was stirred and extracted with 30 l of ethyl acetate. This procedure was repeated once again. The ethyl acetate layers were pooled, washed twice with 30 l portions of water, dried by the addition of 500 g of anhydrous sodium sulfate and concentrated under reduced pressure to 200 ml. Petroleum ether was added to the concentrate and the resulting precipitate was recovered by filtration (53 g). This crude product (I) was stirred with 100 ml of ethyl acetate and the insolubles were filtered off. The filtrate was stirred with 10 g of silica gel (E. Merck AG, Germany, 0.05—0.2 mm) and the ethyl acetate was removed under reduced pressure. The residue was applied to the top of a silica gel column (400 ml). Elution was carried out with 500 ml of *n*-hexane, 500 ml of hexane/ethyl acetate (3:1), 500 ml of hexane/ethyl acetate (1:1), 500 ml of hexane/ethyl acetate (1:3), 500 ml of ethyl acetate, 1 l of ethyl acetate/methanol (50:1), and 1 l of ethyl acetate-methanol (25:1), with the eluate being collected in 100 ml fractions. One ml portion of each fraction was concentrated to dryness, and 0.1 ml of ethyl acetate was added to the concentrate. The mixture was spotted at 2.5 cm from the bottom edge of a silica gel-glass plate (E. Merck, AG, Germany, Kieselgel (trade mark) 60 $F_{254}$, 0.25 mm, 20 x 20 cm) and developed for about 17 cm with a solvent system of ethyl acetate/methanol (19:1). After development, detection was carried out with ultraviolet rays (2537 Å). The active fractions No. 25—No. 30 of Rf 0.58—0.63 and the fractions Nos. 38—40 of Rf 0.25—0.30 were collected and concentrated under reduced pressure to about 20 ml, respectively. To each of these concentrates were added 150 ml of petroleum ether to yield 5 g of a crude product (II) and 2 g of crude maytansinol.

In 10 ml of ethyl acetate was dissolved 0.5 g of the crude product (II) obtained above and the resulting solution was thoroughly stirred with 4 g of silica gel (E. Merck AG, Germany, 0.05—0.2 mm). The ethyl acetate was removed under reduced pressure. The residue was applied to the top of a column of 300 ml silica gel and the column was first washed with 500 ml of chloroform and then eluted with 500 ml of chloroform/methanol (50:1), 500 ml of chloroform/methanol (20:1) and 500 ml of chloroform/methanol (10:1). The eluate was collected in 25 ml fractions.

A 0.5 ml portion of each fraction was concentrated under reduced pressure. 0.05 ml of ethyl acetate was added to the concentrate, and the resulting mixture as a sample was subjected to thin layer chromatography (developing system: chloroform/methanol = 9:1). Fractions Nos. 40 and 41 absorbing at 2537 Å in the zone of Rf 0.48—0.50 were collected and concentrated to dryness under reduced pressure. 0.5 ml of ethyl acetate was added to the residue and the resulting mixture was allowed to stand, whereupon 50 ml of mixed crystals of maytanacine and maytansinol propionate were obtained.

50 Milligrams of the above mixed crystals of maytanacine and maytansinol propionate were dissolved in 5 ml of methanol, followed by the addition of 100 mg of sodium chloride and 5 ml of water. A column measuring 1.8 cm in diameter was packed with 200 ml of Diaion HP-10 (trade mark) (Mitsubishi Chemical Industries, Ltd., Japan), and washed with 600 ml of 50% methanol/water containing 5% of NaCl. The sample solution prepared above was passed through the column, and gradient elution was carried out using 1.5 l of 60% methanol/water containing 5% NaCl and 1.5 l of 95% methanol/water. The eluate was collected in 15 ml fractions and each fraction was investigated by thin layer chromatography. The fractions 130 to 135 contained maytanacine, and the fractions 138—142 contained maytansinol propionate.

Each group of fractions was concentrated and dissolved by the addition of 30 ml of water and 50 ml of ethyl acetate. The solution was shaken in a separation funnel, the water layer was separated off, and, after washing twice with 30 ml portions of water, the ethyl acetate layer was dried over anhydrous sodium sulfate, concentrated and allowed to stand. In the above manner, crystals were obtained from each group of fractions. The crystals were collected by filtration and dried.

| | |
|---|---|
| Maytanacine | 13 mg. |
| Maytansinol propionate | 25 mg. |

0.2 g of the crude maytansinol obtained above was dissolved in 3 ml of ethyl acetate and the resulting solution was thoroughly stirred with 0.5 g of silica gel (E. Merck AG, Germany, 0.05—0.2 mm). The ethyl acetate was removed under reduced pressure. The residue was applied to a column of 80 ml silica gel and the column was first washed with 150 ml of chloroform and then eluted with 150 ml of chloroform/methanol (50:1), 150 ml of chloroform/methanol (20:1) and 300 ml of chloroform/methanol (10:1). The eluate was collected in 10 ml fractions and a 0.5 ml portion of each fraction was concentrated under reduced pressure. 0.05 ml of ethyl acetate was added to the concentrate, and the mixture as a sample was subjected to thin layer chromatography (developing system: chloroform/methanol = 9:1). The fractions which were detected as corresponding to an absorption band of 2537 Å, having the Rf 0.33—0.38, were collected and concentrated to dryness under reduced pressure. 0.5 ml of ethyl acetate was added to the residue and the mixture was allowed to stand, whereupon 20 mg crystals of maytansinol were obtained.

The physico-chemical properties of the resulting maytansinol, maytanacine and maytansinol propionate are shown in Table 2 as follows:

TABLE 2

| | Maytanacine $C_{30}H_{39}ClN_2O_9$ | Maytansinol propionate $C_{31}H_{41}ClN_2O_9$ | Maytansinol $C_{28}H_{37}ClN_2O_8$ |
|---|---|---|---|
| Melting point (°C) | 235–236°C | 188–190°C | 172.5–174°C |
| Specific rotation | $(\alpha)_D^{22°}$ −121° ± 10° (C = 0.25 $CHCl_3$) | $(\alpha)_D^{22°}$ −127° ± 10° (C = 0.35 $CHCl_3$) | $(\alpha)_D^{22°}$ −313° ± 10° (C = 0.22 $CHCl_3$) |
| Analysis Found (%) | C 59.62<br>H 6.93<br>N 4.28<br>Cl 5.74 | C 59.93<br>H 6.82<br>N 4.32<br>Cl 5.57 | C 59.28<br>H 6.38<br>N 5.02<br>Cl 6.15 |
| Analysis Calcd. (%) | C 59.85<br>H 6.48<br>N 4.61<br>Cl 5.84 | C 59.94<br>H 6.65<br>N 4.51<br>Cl 5.71 | C 59.52<br>H 6.60<br>N 4.96<br>Cl 6.27 |
| Ultraviolet absorption spectrum | 233(30330)<br>240(sh 28240)<br>252(27850)<br>280(5680)<br>288(5660) | 233(30240)<br>240(sh 28400)<br>252(27650)<br>280(5740)<br>288(5710) | 232(32750)<br>244(sh 30850)<br>252(31650)<br>281(5750)<br>288(5700) |
| Infrared absorption spectrum | 1740, 1730, 1670, 1580 | 1740, 1730, 1670, 1580 | 1715, 1670, 1580 |

TABLE 2 (Continued)

| | Maytanacine $C_{30}H_{39}ClN_2O_9$ | Maytansinol propionate $C_{31}H_{41}ClN_2O_9$ | Maytansinol $C_{28}H_{37}ClN_2O_8$ |
|---|---|---|---|
| Mass spectrum m/e | 545, 485, 470, 450 | 559, 485, 470, 450 | 503, 485, 470, 450 |
| Acid, neutral or basic | lipophyl and neutral substance | lipophyl and neutral substance | lipophyl and neutral substance |
| Colour reactions | Dragendorff: Positive Beilstein: Positive | Dragendorff: Positive Beilstein: Positive | Dragendorff: Positive Beilstein: Positive |

Reference Example 23

*Nocardia* sp. No. C-15003 (IFO 13726; FERM-P No. 3992; ATCC-31281) as grown on a slant medium (yeast extract-malt extract-agar) was inoculated into a 200 ml conical flask containing 40 ml of a seed culture medium (2% glucose, 3% soluble starch, 1% raw soybean meal, 1% corn steep liquor, 0.5% Polypeptone), 0.3% NaCl, 0.5% $CaCO_3$, pH 7.0). The inoculated medium was incubated at 28°C on a rotary shaker for 48 hours to obtain an inoculum. A 0.5 ml portion of the inoculum thus obtained was transferred to a 200 ml conical flask containing 40 ml of a fermentation medium composed of 5% dextrin, 3% corn steep liquor, 0.1% Polypepton and 0.5% $CaCO_3$ (pH 7.0), and cultivated on a rotary shaker at 28°C for 90 hours.

As determined by the serial dilution method using *Tetrahymena pyriformis* W as an assay organism and ansamitocin P-3 as the standard sample, the production potency of the above culture was found to be 25 μg/ml.

Reference Example 24

A 10 ml portion of the seed culture obtained in Reference Example 23 was transferred to a 2 l Sakaguchi flask containing 500 ml of a seed culture medium and incubated on a rotary shaker at 28°C for 48 hours. A 500 ml portion of the resulting culture was transferred to a 50 l tank of stainless steel containing 30 l of seed culture medium and cultivated at 28°C, 30 l/min aeration, 280 r.p.m. (1/2DT) and 1 kg/cm² internal pressure to obtain a seed culture. This culture was used to seed a 200 l tank of stainless steel containing 100 l of a fermentation medium similar to that used in Reference Example 23 at an inoculation rate of 10%. The inoculated medium was incubated at 28°C, 100 l/m. aeration, 200 r.p.m. (1/2DT) and 1 kg/cm² internal pressure for 90 hours. As determined by the same procedure as that described in Reference Example 23, the production potency of the culture obtained above was found to be 25 μg/ml.

Reference Example 25

To 95 l of the culture obtained in Reference Example 24 were added 2 kg of Hyflo Super Cel (trade mark) (Johns Manville Sales Corp., Products, U.S.A.) and, after thorough mixing, the mixture was filtered on a pressure filter to obtain 85 l of filtrate and 32 kg of moist cells. The filtrate (85 l) was stirred and extracted with 30 l of ethyl acetate. This procedure was repeated once again. The ethyl acetate layers were pooled, washed twice with 30 l portions of water, dried by the addition of 500 g of anhydrous sodium sulfate and concentrated under reduced pressure to 200 ml. Petroleum ether was added to the concentrate and the resulting precipitate was recovered by filtration (53 g). This crude product (I) was stirred with 100 ml of ethyl acetate and the insolubles were filtered off. The filtrate was stirred with 10 g of silica gel (E. Merck AG, Germany, 0.05—0.2 mm) and the ethyl acetate was removed under reduced pressure. The residue was applied to the top of a silica gel column (400 ml). Elution was carried out with 500 ml of hexane, 500 ml of hexane/ethyl acetate (3:1), 500 ml of hexane/ethyl acetate (1:1), 500 ml of hexane/ethyl acetate (1:3), 500 ml of ethyl acetate and 1 l of ethyl acetate/methanol (50:1), with the eluate being collected in 100 ml fractions.

A 1 ml portion of each fraction was concentrated to dryness, and 0.1 ml of ethyl acetate was added to the concentrate. The resulting mixture was spotted at 2.5 cm from the bottom edge of a silica gel-glass plate (E. Merck AG, Germany, 60 $F_{254}$, 0.25 mm, 20 x 20 cm) and developed for about 17 cm

with a solvent system of ethyl acetate/methanol (19:1). After development, detection was carried out with ultraviolet light (2537 Å). The active fractions No. 23—28 of Rf 0.6—0.65, were collected and concentrated under reduced pressure to about 20 ml. To this concentrate were added 150 ml of petroleum ether to obtain 15 g of a crude product (II).

Reference Example 26

32 kg of the cells obtained in Reference Example 25 were extracted with 50 l of 70% acetone-water for 3 hours under stirring and were then filtered on a pressure filter. The extraction with 50 l of 70% acetone-water and the subsequent filtration was repeated once again. The filtrates were pooled and the acetone was removed by concentration under reduced pressure. The resulting aqueous system was passed through a column of 5 l of Diaion HP-10 (Mitsubishi Chemical Industries, Ltd., Japan). The column was washed with 20 l of water and 50% aqueous methanol, followed by elution with 15 l of 90% methanol-water. The eluate was concentrated under reduced pressure to 3 l and shaken with 3 l of water and 3 l of ethyl acetate. The above procedure was repeated once again. The ethyl acetate layers were combined, washed with water, dried by the addition of anhydrous sodium sulfate and concentrated under reduced pressure to 200 ml. Following the addition of petroleum ether, the precipitate was recovered by filtration (28 g). The above product was purified by means of a column of silica gel to recover 8.0 g of crude product (II).

Reference Example 27

1.5 g of the crude product (II) obtained in Reference Example 25 were dissolved in 10 ml of ethyl acetate and the resulting solution was thoroughly stirred with 4 g of silica gel (E. Merck AG, Germany, 0.05—0.2 mm). The ethyl acetate was removed under reduced pressure. The residue was applied to the top of a column of 300 ml silica gel and the column was first washed with 500 ml of chloroform and then eluted with 500 ml of chloroform/methanol (50:1), 500 ml of chloroform/methanol (20:1) and 500 ml of chloroform/methanol (10:1). The eluate was collected in 25 ml fractions.

A 0.5 ml portion of each fraction was concentrated under reduced pressure. 0.05 ml of ethyl acetate was added to the concentrate, and the mixture as a sample was subjected to silica gel thin layer chromatography (developing system: chloroform-methanol = 9:1).

The fractions which were detected as corresponding to an absorption band of 2537 Å, having the Rf values of 0.50—0.60, were collected and concentrated to dryness under reduced pressure. 2 ml of ethyl acetate were added to the residue, and the mixture was allowed to stand, whereupon 150 mg crystals of a mixture of ansamitocin P3, P-3′ and P-4 were obtained.

The above crystals of the mixture of ansamitocin P-3, P-3′ and P-4 (150 mg) were dissolved in 15 ml of methanol, followed by the addition of 300 mg of sodium chloride and 15 ml of water. A column measuring 1.8 cm in diameter was packed with 200 ml of Diaion HP-10 (trade mark) (Mitsubishi Chemical Industries, Ltd.) and calibrated with 600 ml of 50% methanol-water containing 5% of NaCl. The sample solution prepared above was passed through the column, and gradient elution was carried out using 1.5 l of 60% methanol-water containing 5% NaCl and 1.5 l of 95% methanol-water. The eluate was collected in 15 ml fractions and each fraction was investigated by silica gel thin layer chromatography. The 145 to 153 fractions contained ansamitocin P-3, the 167—180 fractions contained ansamitocin P-3′ and P-4 and the 185—190 fractions contained ansamitocin P-4.

Each group of fractions was concentrated and dissolved by the addition of 50 ml of water and 100 ml of ethyl acetate. The solution was shaken in a separation funnel, the water layer was separated off and, after washing with two 50 ml-portions of water, the ethyl acetate layer was dried over anhydrous sodium sulfate, concentrated and allowed to stand. In the above manner, crystals were obtained from each group of fractions. The crystals were collected by filtration and dried:

| | |
|---|---|
| ansamitocin P-3 | 70 mg |
| ansamitocin P-3′, P-4 | 18 mg |
| ansamitocin P-4 | 15 mg |

The mixed crystals of ansamitocin P-3′ and P-4 (18 mg) were dissolved in 0.3 ml of ethyl acetate and spotted in a line at a distance of 2.5 cm from the bottom edge of a silica gel glass plate (E. Merck AG, Germany, Kieselgel 60 $F_{254}$, (trade mark) 0.25 mm, 20 x 20 cm), followed by development with ethyl acetate/methanol (19:1). After development to about 18 cm, the absorption bands at Rf 0.68 (P-4) and Rf 0.65 (P-3′) were scraped up and were each independently extracted twice with ethyl acetate containing a small amount of water. The resulting ethyl acetate extract was washed with water, dried over anhydrous sodium sulfate, concentrated under reduced pressure and allowed to stand.

10 mg crystals of ansamitocin P-4 and 3 mg crystals of ansamitocin P-3′ were obtained from the fractions of Rf 0.68 and Rf 0.65, respectively.

The physical and chemical properties of ansamitocin P-3, P-3′ and P-4 obtained as above are shown in Table 3 below.

TABLE 3

| | Ansamitocin | | |
|---|---|---|---|
| | P–3<br>$C_{32}H_{43}ClN_2O_9$ = 635.169 | P–3′<br>$C_{32}H_{43}ClN_2O_9$ = 635.169 | P–4<br>$C_{33}H_{45}ClN_2O_9$ = 649.196 |
| m.p. (°C) | 190–192 | 182–185 | 177–180 |
| Specific rotation $(\alpha)_D^{22°}$ | −136° ± 10°<br>(c = 0.375 $CHCl_3$) | −134° ± 10°<br>(c = 0.11 $CHCl_3$) | −142° ± 10°<br>(c = 0.522 $CHCl_3$) |
| Elemental analysis Found (%) | C 60.06<br>H 7.04<br>N 4.33<br>Cl 5.37 | C 60.09<br>H 7.02<br>N 4.34<br>Cl 5.99 | C 60.65<br>H 7.05<br>N 4.25<br>Cl 5.23 |
| Elemental analysis Calcd. (%) | C 60.51<br>H 6.82<br>N 4.41<br>Cl 5.58 | C 60.51<br>H 6.82<br>N 4.41<br>Cl 5.58 | C 61.05<br>H 6.99<br>N 4.32<br>Cl 5.46 |
| Ultraviolet absorption spectra nm ($\epsilon$) in methanol | 233(30250)<br>240(sh 28450)<br>252(27640)<br>280(5750)<br>288(5700) | 233(30155)<br>240(sh 28250)<br>252(27600)<br>280(5750)<br>288(5700) | 233(29900)<br>240(sh 28240)<br>252(27590)<br>280(5712)<br>288(5680) |
| Infrared absorption spectra ($cm^{-1}$) KBr | 1740, 1730,<br>1670, 1580,<br>1445, 1385,<br>1340, 1255,<br>1180, 1150,<br>1100, 1080<br>1038 | 1740, 1730,<br>1670, 1580,<br>1445, 1385,<br>1340, 1255,<br>1180, 1150,<br>1100, 1080,<br>1038 | 1740, 1730,<br>1670, 1580,<br>1445, 1385,<br>1340, 1255,<br>1180, 1150,<br>1100, 1080,<br>1038 |

TABLE 3 (Continued)

| | Ansamitocin | | |
|---|---|---|---|
| | P-3<br>$C_{32}H_{43}ClN_2O_9$ =<br>635.169 | P-3'<br>$C_{32}H_{43}ClN_2O_9$ =<br>635.169 | P-4<br>$C_{33}H_{45}ClN_2O_9$ =<br>649.196 |
| Nuclear magnetic resonance spectra in 100 MHz $CPCl_3$ (ppm) | 1.27(d) (3H)<br>1.28(d) (3H) | 1.06(t) (3H) | 1.03(d) (6H) |
| Mass spectra m/e | 573, 485, 470,<br>450 | 573, 485, 470,<br>450 | 587, 485, 470,<br>450 |
| Solubility | Insoluble in petr. ether, *n*-hexane and water.<br>Sparingly soluble in benzene and ether.<br>Soluble in chloroform, ethyl acetate, acetone, ethanol, methanol, pyridine, tetrahydrofuran and dimethylsulfoxide. | Insoluble in petr. ether, *n*-hexane and water.<br>Sparingly soluble in benzene and ether.<br>Soluble in chloroform, ethyl acetate, acetone, ethanol, methanol, pyridine, tetrahydrofuran and dimethylsulfoxide. | Insoluble in petr. ether, *n*-hexane and water.<br>Sparingly soluble in benzene and ether.<br>Soluble in chloroform, ethyl acetate, acetone, ethanol, methanol, pyridine, tetrahydrofuran and dimethylsulfoxide. |
| Colour reactions | Dragendorff: Positive<br>Beilstein: Positive | Dragendorff: Positive<br>Beilstein: Positive | Dragendorff: Positive<br>Beilstein: Positive |

Reference Example 28

15 mg. of the mixture of ansamitocin P-3, P-3' and P-4 obtained in Reference Example 27 were dissolved in 1 ml of tetrahydrofuran and, after the solution was cooled to −5°C, 12 mg of lithium aluminium hydride were added. The mixture was allowed to stand for 2 hours. Following the addition of 0.5 ml of a 1% aqueous solution of $H_2SO_4$, the reaction mixture was extracted with 2 ml of ethyl acetate. The ethyl acetate layer was washed with water, dried by the addition of anhydrous sodium sulfate and concentrated under reduced pressure. Preparative TLC with silica gel (solvent: ethyl acetate/methanol 19:1) was carried out on the concentrate and the zone in the neighborhood of Rf 0.25—0.3 was scraped up and extracted with ethyl acetate containing a small volume of water. The extract was washed with water, dried over anhydrous sodium sulfate and concentrated under reduced pressure, whereupon crystals separated out. The crystals were recovered by filtration and dried. By the above procedure there were obtained 10 mg. of maytansinol, melting point 174°C.

Elemental analysis:

Found: C, 59.65; H, 6.58; N, 5.02; Cl, 6.51

Calcd. for $C_{28}H_{37}ClN_2O_8$ C, 59.52; H, 6.60; N, 4.96; Cl, 6.27

IR: 1715, 1670 1580 ($cm^{-1}$)

UV (nm): 232 (32750, 244 (sh 30850), 252 (31650), 281 (5750), 288 (5700)

In properties, this product is in good agreement with the maytansinol obtained in Reference Example 22.

Reference Example 29

100 mg. of maytansinol were dissolved in 0.7 ml of pyridine. To the solution, cooled with ice, there was added 0.35 ml of acetic anhydride. The mixture was left standing for 18 hours at room temperature (23—25°C). The reaction solution was poured into 5 ml of cold water and stirred. The precipitate which separated out was collected by filtration and dried to yield 85 mg of powdery substance. This powdery substance was dissolved in 1 ml of chloroform/methanol 10:1 (V/V).

0.2 g of silica gel was added to the solution and mixed into it sufficiently. The mixture was then applied to the top of a silica gel column (80 ml). Elution was carried out with ethyl acetate saturated with water. The eluate was collected in 10 ml fractions. Fractions Nos. 37—44 were collected and concentrated under reduced pressure. A small volume of ethyl acetate (about 0.5 ml) was added to the residue to redissolve the precipitating crystals and the solution was left standing at room temperature. Precipitating crystals were collected by filtration, whereupon 36.0 mg. of maytanacine were obtained. The melting point of this was 231°C and was found to be identical to the maytanacine obtained in Reference Example 22 in mass spectrum, IR spectrum, UV-spectrum and thin layer chromatogram.

Example 1

*Bacillus megaterium* IFO 12108 was inoculated into a culture medium (pH 7.5) containing 2% dextrin, 0.5% peptone, 0.5% yeast extract and 0.5% meat extract and the inoculated medium was shake-cultured at 30°C for 16 hours. 110 mg of ansamitocin P-4 were added to 275 l of this culture and the reaction was carried out under shaking at 30°C for 51 hours. As assayed by thin-layer chromatography (TLC), the ansamitocin P-4 had disappeared completely and, instead, PDM-4 had been produced in the reaction mixture.

Example 2

To 2.75 l of the culture broth obtained in Example 1 were stirred 1.3 l of ethyl acetate and the mixture was stirred to achieve extraction. The mixture was suction-filtered through a filter coated with 30 g of Hyflo Super Cel (trade mark) (Johns Manville Products Co., U.S.A.). This procedure was repeated for a second time. The ethyl acetate layers were combined, washed twice with 800 ml of 1/200 N-HCl and 400ml of 0.5% aqueous sodium bicarbonate in that order, and washed twice with 400 ml portions of water. The washed extract was dried with 10 g of anhydrous sodium sulfate and concentrated under reduced pressure to 2 ml. To this residue were added 50 ml of petroleum ether and the resulting precipitate was recovered by filtration (126 mg).

This crude product (i) of PDM-4 was dissolved in a small amount of chloroform and run onto a column (1 cm dia.) of 5 g of silica gel (E. Merck, AG, Germany, 0.05—0.2 mm). Elution was carried out with 100 ml of chloroform, 100 ml of chloroform/methanol (40:1) and 200 ml of chloroform/methanol (20:1), the eluate being collected in 10 ml fractions. Each fraction was spotted to a silica gel-glass plate (E. Merck AG, Germany, Kieselgel $^{60}F_{254}$ (trade mark), 0.25 mm, 20 x 20 cm) at a distance of 2.5 cm from its bottom edge and the chromatogram was developed over 17 cm with ethyl acetate/methanol (19:1). The fractions (Nos. 13 to 17) which were detected as absorption spots of 2537 Å, having the Rf value of 0.64, were combined and concentrated under reduced pressure to about 1 ml. 30 ml of petroleum ether were added to the concentrate, whereby 79 mg of crude product (ii) of PDM-4 were obtained.

Example 3

79 mg of the crude product (ii) of PDM-4 obtained in Example 2 were dissolved in a small amount of chloroform, and the solution was linearly applied to each of 4 silica gel-glass plates (E. Merck AG, Germany, Kieselgel $F_{254}$, 2 mm, 20 x 20 cm) at a distance of 2.5 cm from its bottom edge. Each chromatogram was developed with ethyl acetate/methanol (19:1) and the absorbing zone at Rf 0.64 was scraped up and extracted twice with ethyl acetate containing a small amount of water. The ethyl acetate extract thus obtained was washed with water, dried over anhydrous sodium sulfate, concentrated under reduced pressure and treated with petroleum ether. By the above procedure there were obtained 68 mg of PDM-4 as a white powder.

Example 4

To 5.5 l of the culture broth of *Bacillus megaterium* IFO 12108 as obtained in Example 1 were added 220 mg of maytansinol and the reaction was carried out under shaking at 30°C for 30 hours. As assayed by TLC, the maytansinol had decreased, and, instead, demethylmaytansinol had been produced in the reaction system.

Example 5.

The culture broth obtained in Example 4 was purified as in Example 2 and subjected to TLC as in Example 2. The fractions at Rf = 0.25 were collected to yield 61 mg of a crude product of demethylmaytansinol. This was further purified as described in Example 3 to give 33 mg of demethylmaytansinol as a white powder.

Example 6

To 12.5 l of the culture broth of *Bacillus megaterium* IFO 12108 obtained as in Example 1 were added 500 mg of maytansinol propionate and the reaction was carried out under shaking at 30°C for 28 hours. As determined by TLC, maytansinol propionate had completely disappeared and, instead, demethylmaytansinol propionate had been produced in the culture broth.

Example 7

With 12 l of ethyl acetate, 12.5 l of the culture broth obtained in Example 6 were purified as in Example 2 and TLC was performed thereon as in Example 2. The fractions at Rf = 0.58 were collected, concentrated to dryness under reduced pressure, and allowed to stand with 5 ml of ethyl acetate. By this procedure there were obtained 235 mg of crystals of demethylmaytansinol propionate.

Example 8

To 1 l of a culture broth of *Bacillus megaterium* IFO 12108 as obtained in Example 1 was added a mixture (10 mg) of maytansinol, maytanacine, maytansinol propionate, ansamitocin P-3 and ansamitocin P-4, and the reaction was carried out under shaking at 30°C for 24 hours. As assayed by TLC, the above mixture had decreased and, instead, the demethyl analogues of the above mixture had been formed in the culture broth. As the chromatogram was developed with a solvent of a mixture of ethyl acetate/methanol (19:1), there were detected demethylmaytansinol at Rf 0.25 to 0.30, demethylmaytanacine at Rf 0.54, demethylmaytansinol propionate at Rf 0.58 PDM-3 at Rf 0.61 and PDM-4 at Rf 0.64. With chloroform/methanol (9:1) as the developing solvent, there were detected demethylmaytansinol at Rf 0.30, demethylmaytanacine at Rf 0.38, demethylmaytansinol propionate at Rf 0.40, PDM-3 at Rf 0.42 and PDM-4 Rf 0.44.

Example 9

*Streptomyces flavotricini* IFO 12770 was inoculated into a culture medium (pH 7.2) containing 1% dextrin, 1% glucose, 1% glycerol, 0.5% peptone, 0.5% yeast extract, 0.5% meat extract, 0.3% sodium chloride and 0.5% calcium carbonate and shaking culture was carried out at 28°C for 48 hours. To 2 l of the resulting culture were added 20 mg of ansamitocin P-3 and the reaction was carried out under shaking at 28°C for 48 hours. The culture broth was purified as Example 2 and 3. 12 mg of PDM-3 were obtained as a white powder by the above procedure.

Example 10

To 2 l of a culture broth of *Streptomyces flavotricini* IFO 12770 obtained as in Example 9 were added 20 mg of maytanacine, and the reaction was carried out under shaking at 28°C for 48 hours. The culture broth was then purified and allowed to stand with a small amount of ethyl acetate as in Examples 2 and 3. By the above procedure there were obtained 6 mg of crystals of demethylmaytanacine.

Example 11.

*Actinomyces nigrescens* IFO 12894 was cultivated as in Example 9, and 10 mg of maytansinol propionate were added to 1 l of the culture. The reaction was carried out under shaking at 28°C for 48 hours. As assayed by TLC, the maytansinol propionate had completely disappeared and, instead, demethylmaytansinol propionate had been produced in the culture broth.

Example 12

*Streptomyces platensis* IFO 12901 was cultivated as in Example 9, and 10 mg of maytansinol propionate were added to 1 l of the culture. The reaction was carried out under shaking at 28°C for 48 hours. As assayed by TLC, the maytansinol propionate had completely disappeared, and, instead, demethylmaytansinol propionate had been formed in the culture broth.

Example 13.

80 mg of crystals of demethylmaytansinol propionate obtained in Example 7 were dissolved in 30 ml of tetrahydrofuran and the solution was cooled to −5°C. To this solution were added 80 mg of lithium aluminium hydride. The reaction mixture was stirred in an ice-bath for 30 minutes, and, after the addition of 10 ml of 1/200N-hydrochloric acid, the mixture was extracted with 50 ml of ethyl acetate. The ethyl acetate layer was washed with water, dried by the addition of anhydrous sodium sulfate and concentrated under reduced pressure.

Preparative TLC (silica gel) of the product was carried out on the concentrate with ethyl acetate/methanol (19:1) over a distance of 17 cm. The UV absorbing zone at Rf = 0.25 to 0.40 was scraped up, and extracted with ethyl acetate containing a small amount of water. The extract was

washed with water, dried over anhydrous sodium sulfate and concentrated under reduced pressure. By the above procedure there were obtained 41 mg of demethylmaytansinol. The physico-chemical properties of this product were identical with those of the demethylmaytansinol obtained in Example 5.

Example 14

In 20 ml of tetrahydrofuran there were dissolved 50 mg of the powder of PDM-3 obtained in Example 9, and, after the solution had been cooled to −5°C, 50 mg of lithium aluminium hydride were added. The reaction mixture was treated and purified as in Example 13 to yield 27 mg of demethylmaytansinol as a white powder. The physico-chemical properties of this product were identical with those of the demethylmaytansinol obtained in Example 5.

Example 15

The powder of PDM-4 (50 mg) obtained in Example 3 was treated and purified as in Example 13 to yield 23 mg of demethylmaytansinol. Based on its physico-chemical data, this product was identified with the demethylmaytansinol obtained in Example 5.

Example 16

The crystals of demethylmaytanacine (50 mg) obtained in Example 10 were treated and purified as in Example 13 to yield 29 mg of demethylmaytansinol. Based on its physico-chemical data, this product was identified with the demethylmaytansinol obtained in Example 5.

Example 17

In 20 ml of tetrahydrofuran were dissolved 30 mg of a mixture of demethylmaytanacine, demethylmaytansinol propionate, PDM-3 and PDM-4 obtained in Example 8, and, after cooling to −5°C, 30 mg of lithium aluminium hydride were added. The reaction mixture was treated and purified as in Example 13 to yield 12 mg of demethylmaytansinol. Based on its physico-chemical data, this product was identified with the demethylmaytansinol obtained in Example 5.

Example 18

*Streptomyces libani* IFO 13452 was cultivated as in Example 9, and 10 mg of ansamitocin P-3 were added to 1 l of the resulting culture. The reaction was carried out under shaking at 28°C for 48 hours. As assayed by TLC, the ansamitocin P-3 had completely disappeared and, instead, PDM-3 had been formed in the culture broth.

The properties of the compounds obtained in the foregoing Examples are given in the following table.

TABLE 4

| | | Demethyl-maytansinol | Demethyl-maytanacine |
|---|---|---|---|
| Molecular formula, Molecular weight | | $C_{27}H_{35}ClN_2O_8$ = 551.050 | $C_{29}H_{37}ClN_2O_9$ = 593.088 |
| Melting point (°C) | | 194 – 196 | 224 – 226 |
| Specific rotation $(\alpha)_D^{22}$ | $(CHCl_3)$ | -201° ± 10° (c = 0.215) | -115° ± 10° (c = 0.12) |
| | (MeOH) | -209° ± 10° (c = 0.50) | -98° ± 10° (c = 0.505) |
| Elemental analysis Found (%) | C | 58.53 | 58.66 |
| | H | 6.69 | 6.43 |
| | N | 4.81 | 4.67 |
| | Cl | 6.28 | 5.79 |
| Elemental analysis Calculated (%) | C | 58.85 | 58.73 |
| | H | 6.40 | 6.29 |
| | N | 5.08 | 4.72 |
| | Cl | 6.43 | 5.98 |
| Ultraviolet ray absorption spectra nm (ε) (MeOH) | | 232 (31200)<br>242 (33400)<br>251 (34000)<br>280 (6890)<br>288 (6840) | 232 (29700)<br>242 (30700)<br>251 (31000)<br>280 (6110)<br>288 (6050) |

TABLE 4 (Continued)

| | | Demethylmaytansinol-propionate | PDM-3 | PDM-4 |
|---|---|---|---|---|
| Molecular formula, Molecular weight | | $C_{30}H_{39}ClN_2O_9$ = 607.115 | $C_{31}H_{41}ClN_2O_9$ = 621.142 | $C_{32}H_{43}ClN_2O_9$ = 635.169 |
| Melting point (°C) | | 193 – 195 | 165 – 168 | 185 – 188 |
| Specific rotation $(\alpha)^{22}_{D}$ | (CHCl₃) | −104° ± 10° (c = 0.22) | −132° ± 10° (c = 0.20) | −110° ± 10° (c = 0.22) |
| | (MeOH) | −112° ± 10° (c = 0.54) | −120° ± 10° (c = 0.52) | −112° ± 10° (c = 0.51) |
| Elemental analysis Found (%) | C | 59.23 | 59.61 | 60.17 |
| | H | 6.68 | 6.92 | 7.08 |
| | N | 4.55 | 4.36 | 4.27 |
| | Cl | 5.69 | 5.47 | 5.33 |
| Elemental analysis Calculated (%) | C | 59.35 | 59.94 | 60.51 |
| | H | 6.48 | 6.65 | 6.82 |
| | N | 4.61 | 4.51 | 4.41 |
| | Cl | 5.84 | 5.71 | 5.58 |
| Ultraviolet ray absorption spectra nm (ε) (MeOH) | | 232 (30000)<br>242 (30900)<br>251 (31200)<br>280 (6380)<br>288 (6320) | 232 (28900)<br>242 (29200)<br>251 (29200)<br>280 (6150)<br>288 (5970) | 232 (30000)<br>242 (30400)<br>251 (30500)<br>280 (6360)<br>288 (6290) |

TABLE 4 (Continued)

| | Demethyl-maytansinol | Demethyl-maytanacine |
|---|---|---|
| Molecular formula, Molecular weight | $C_{27}H_{35}ClN_2O_8$ = 551.050 | $C_{29}H_{37}ClN_2O_9$ = 593.088 |
| Infrared ray absorption spectra $cm^{-1}$ (KBr) | 1726, 1697, 1640, 1587, 1435, 1390, 1345, 1305, 1276, 1175, 1155, 1107, 1080, 1005, | 1737, 1697, 1660, 1594, 1443, 1397, 1378, 1335, 1280, 1234, 1180, 1158, 1100, 1083, 1030 |
| Nuclear magnetic resonance spectra ppm (100 MHz) (solvent) | 3.06 (3H, s) 3.26 (3H, s) ($d_6$–DMSO) | 2.14 (3H, s) 30.6 (3H, s) 3.27 (3H, s) ($d_6$–DMSO) |
| Mass spectra m/e | 489, 471 | 531, 471, 456, 436 |

TABLE 4 (Continued)

| | Demethylmaytansinol-propionate | PDM–3 | PDM–4 |
|---|---|---|---|
| Molecular formula, Molecular weight | $C_{30}H_{39}ClN_2O_9$ = 607.115 | $C_{31}H_{41}ClN_2O_9$ = 621.142 | $C_{32}H_{43}ClN_2O_9$ = 635.169 |
| Infrared ray absorption spectra $cm^{-1}$ (KBr) | 1737, 1715, 1642, 1594, 1442, 1397, 1344, 1320, 1280, 1238, 1175, 1100, 1085, 1020, | 1740, 1710, 1642, 1593, 1443, 1397, 1355, 1320, 1283, 1238, 1185, 1155, 1110, 1085, 1018 | 1738, 1715, 1662, 1585, 1442, 1395, 1377, 1300, 1283, 1183, 1167, 1110, 1085, 1018, |
| Nuclear magnetic resonance spectra ppm (100 MHz) (solvent) | 1.07 (3H, t) 3.04 (3H, s) 3.27 (3H, s) ($d_6$–DMSO) | 1.27 (3H, d) 1.29 (3H, d) 3.19 (3H, s) 3.38 (3H, s) ($CDCl_3$) | 1.03 (6H, d) 3.17 (3H, s) 3.39 (3H, s) ($CDCl_3$) |
| Mass spectra m/e | 545, 471, 456, 436, | 559, 471, 456, 436, | 573, 471, 456, 436 |

TABLE 4 (Continued)

| | Demethyl-maytansinol | Demethyl-maytanacine |
|---|---|---|
| Molecular formula,<br>Molecular weight | $C_{27}H_{35}ClN_2O_8$ =<br>551.050 | $C_{29}H_{37}ClN_2O_9$ =<br>593.088 |
| Solubility | Freely soluble in pyridine, dimethyl sulfoxide.<br>Soluble in chloroform, ethyl acetate, tetrahydrofuran, acetone, ethanol, methanol.<br>Slightly soluble in benzene, ether.<br>Very slightly soluble in water.<br>Practically insoluble in petroleum ether, n-hexane. | Freely soluble in pyridine, dimethyl sulfoxide.<br>Soluble in chloroform, ethyl acetate, tetrahydrofuran, acetone, ethanol, methanol.<br>Slightly soluble in benzene, ether.<br>Very slightly soluble in water.<br>Practically insoluble in petroleum ether, n-hexane. |
| Colour reactions | Dragendorff: Positive<br>Beilstein: Positive | Dragendorff: Positive<br>Beilstein: Positive |

TABLE 4 (Continued)

| | Demethylmaytansinol propionate | PDM–3 | PDM–4 |
|---|---|---|---|
| Molecular formula, Molecular weight | $C_{30}H_{39}ClN_2O_9$ = 607.115 | $C_{31}H_{41}ClN_2O_9$ = 621.142 | $C_{32}H_{43}ClN_2O_9$ = 635.169 |
| Solubility | Freely soluble in pyridine, dimethyl sulfoxide.<br>Soluble in chloroform, ethyl acetate, tetrahydrofuran, acetone, ethanol, methanol.<br>Slightly soluble in benzene, ether.<br>Very slightly soluble in water.<br>Practically insoluble in petroleum ether, n-hexane. | Freely soluble in pyridine, dimethyl sulfoxide.<br>Soluble in chloroform, ethyl acetate, tetrahydrofuran, acetone, ethanol, methanol.<br>Slightly soluble in benzene, ether.<br>Very slightly soluble in water.<br>Practically insoluble in petroleum ether, n-hexane. | Freely soluble in pyridine, dimethyl sulfoxide.<br>Soluble in chloroform, ethyl acetate, tetrahydrofuran, acetone, ethanol, methanol.<br>Slightly soluble in benzene, ether.<br>Very slightly soluble in water.<br>Practically insoluble in petroleum ether, n-hexane. |
| Colour reactions | Dragendorff: Positive<br>Beilstein: Positive | Dragendorff: Positive<br>Beilstein: Positive | Dragendorff: Positive<br>Beilstein: Positive |

Example 19

*Bacillus megaterium* IFO 12108 was inoculated into a culture medium (pH 7.5) containing 2% of dextrin, 0.5% of peptine, 0.5% of yeast extract and 0.5% of meat extract and shake culture was carried out at 28°C for 16 hours. To 1.5 l of this culture were added 30 mg of maytansinol 3-picolinate and the reaction was carried out under shaking at 28°C for 29 hours. As assayed by TLC, maytansinol 3-picolinate had completely disappeared and, instead, demethylmaytansinol 3-picolinate had been formed in the culture broth.

Example 20

To 1.5 l of the culture broth obtained in Example 19 were added 750 ml of ethyl acetate, and, after extraction under stirring, the mixture was suction-filtered through a filtering set-up with 15 g of Hyflo Super Cel (trade mark) (Johns-Manville Products, Corp., U.S.A.). This procedure was repeated for a second time. The ethyl acetate layers were combined, washed twice with 300 ml portions of water, dried over 10 g of anhydrous sodium sulfate and concentrated under reduced pressure.

By this procedure there was obtained crude product (i). This crude product (i) was dissolved in a small amount of chloroform and run onto a column (1 cm dia.) of 5 g silica gel (Merck, Germany, 0.05—0.2 mm.). Elution was carried out with 150 ml of chloroform and chloroform-methanol (20:1), the eluate being collected in 10 ml fractions. Each fraction was spotted on a silica gel-glass plate (kieselgel (trade mark) 60 $F_{254}$, Merck, Germany, 0.25 mm, 20 × 20 cm) at a distance of 2.5 cm from its bottom edge, and the chromatogram was developed over a distance of 17 cm with chloroform methanol (9:1). The fractions (Nos. 12 to 16) which were detected as an absorption band of 2537 Å, having the Rf value of 0.40, were collected and concentrated under reduced pressure to yield 18 mg of

crude product (ii). This crude product was dissolved in a small amount of chloroform, the resulting solution was linearly applied to each of four silica gel-glass plates at a distance of 2.5 cm from its bottom edge, and the chromatograms were developed with water-saturated ethyl acetate. The UV absorbing zones at Rf 0.30 were scraped up and extracted twice with ethyl acetate containing a small amount of water. The ethyl acetate extract was washed with water, dried over anhydrous sodium sulfate and concentrated under reduced pressure. The concentrate was treated with petroleum ether to yield 6 mg of demethylmaytansinol 3-picolinate as a white powder.
UV spectrum ($\lambda_{max}^{MeOH}$) nm: 232, 243, 252, 280, 289
Mass spectrum (m/e): 655, 594, 471, 456, 436

Example 21

To 2 l of a culture broth of *Bacillus megaterium* IFO 12108 obtained as in Example 19 were added 37 mg of maytansinol 3-(N-phenyl)carbamate, and the reaction was carried out under shaking at 28°C for 48 hours. As assayed by TLC, the maytansinol 3-(N-phenyl)carbamate had completely disappeared and, instead, demethylmaytansinol 3-(N-phenyl)carbamate had been formed in the culture broth.

Example 22

The culture broth obtained in Example 21 was purified as in Example 20 and TLC was performed thereon as in Example 20. The fractions at Rf 0.40 were collected to yield 27 mg of a crude product (ii) of demethylmaytansinol 3-(N-phenyl)carbamate. This product (ii) was further purified as in Example 20. By the latter procedure there were obtained 12 mg of demethylmaytansinol 3-(N-phenyl)carbamate as a white powder.
UV spectrum ($\lambda_{max}^{MeOH}$) nm: 233, 252, 280, 288
Mass spectrum (m/e): 608, 471, 456, 436

Example 23

To 2 l of a culture broth of *Bacillus megaterium* IFO 12108 obtained as in Example 19 were added 37 mg of dechloromaytansinol 3-phenylacetate, and the reaction was carried out under shaking at 28°C for 48 hours. As assayed by TLC, dechloromaytansinol 3-phenylacetate had disappeared and, instead, demethyldechloromaytansinol 3-phenylacetate had been formed in the culture broth.

Example 24

The culture broth obtained in Example 23 was purified as in Example 20 and TLC was performed thereon as in Example 20. The fractions absorbing at Rf = 0.43 were collected to yield 30 mg of a crude product (ii) of demethyldechloromaytansinol 3-phenylacetate. This crude product (ii) was further purified as in Example 20. By the above procedure there were obtained 14 mg of demethyldechloromaytansinol 3-phenylacetate as a white powder.
UV spectrum ($\lambda_{max}^{MeOH}$) nm: 230, 240, 249, 277, 285
Mass spectrum (m/e): 634 ($M^+$), 573, 437, 422

Example 25

To 1 l of a culture broth of *Bacillus megaterium* IFO 12108 as obtained in Example 19 were added 18 mg of maytansinol 3-hexanoate, and the reaction was conducted under shaking at 30°C for 48 hours. As determined by TLC, the maytansinol 3-hexanoate had completely disappeared and, instead, demethylmaytansinol 3-hexanoate had been produced in the culture broth.

Example 26

The culture broth obtained in Example 25 was purified as in Example 20 and TLC was carried out thereon as in Example 20. The fractions at Rf = 0.48 were collected to yield 9 mg of a crude product (ii) of demethylmaytansinol 3-hexanoate. Thereafter, this product was further purified as in Example 20. The latter procedure yielded 4 mg of demethylmaytansinol 3-hexanoate as a white powder.
UV spectrum ($\lambda_{max}^{MeOH}$) nm: 233, 240, 252, 280, 289
Mass spectrum (m/e): 587, 471, 456, 436

Example 27

To 1 l of a culture broth of *Bacillus megaterium* IFO 12108 as obtained in Example 19 were added 19.5 mg of maytansinol 3-*p*-chlorobenzoate and the reaction was carried out under shaking at 30°C for 27 hours. As assayed by TLC, the maytansinol 3-*p*-chlorobenzoate had completely disappeared, and demethylmaytansinol 3-*p*-chlorobenzoate had been produced in the culture broth.

Example 28

The culture broth obtained in Example 27 was purified as in Example 20 and TLC was carried out thereon as in Example 20. The fractions at Rf = 0.43 were collected to yield 12 mg of a crude product (ii) of demethylmaytansinol 3-*p*-chlorobenzoate. This crude product was further purified as in Example 20. The above procedure yielded 6 mg of demethylmaytansinol 3-*p*-chlorobenzoate as a white powder.

UV spectrum ($\lambda_{max}^{MeOH}$) nm: 240, 252, 280, 289
Mass spectrum (m/e): 627, 471, 456, 436

Example 29

*Streptomyces flavotricini* IFO 12770 was cultivated as in Example 9, and 15.9 mg of maytansinol 3-cyclohexanecarboxylate were added to 750 ml of the culture. The reaction was conducted under shaking at 28°C for 48 hours. As assayed by TLC, the maytansinol 3-cyclohexanecarboxylate had completely disappeared and, instead, demethylmaytansinol 3-cyclohexanecarboxylate had been formed in the culture broth.

Example 30

The culture broth obtained in Example 29 was purified as in Example 20 and TLC was carried out thereon as in Example 20. The fractions at Rf = 0.45 were collected to obtain 9 mg of crude product (ii) of demethylmaytansinol 3-cyclohexanecarboxylate. This product was further purified as in example 20. By the above procedure there were obtained 4 mg of demethylmaytansinol 3-cyclohexanecarboxylate as a white powder.
UV spectrum ($\lambda_{max}^{MeOH}$) nm: 233, 240, 252, 280, 289
Mass spectrum (m/e): 599, 471, 456, 436

Example 31

*Streptomyces platensis* IFO 12901 was cultivated as in Example 9, and 40 mg of maytansinol 3-phenylacetate were added to 4 l of the culture. The reaction was carried out under shaking at 28°C for 48 hours. As determined by TLC, the maytansinol 3-phenylacetate had decreased and, instead, demethylmaytansinol 3-phenylacetate had been produced in the broth.

Example 32

The culture broth obtained in Example 31 was purified as in Example 20 and TLC was performed thereon as in Example 20. The fractions at Rf = 0.43 were collected to obtain 22 mg of crude demethylmaytansinol 3-phenylacetate (ii). This crude product was further purified as in Example 20. By the above procedure there were obtained 8 mg of demethylmaytansinol 3-phenylacetate as a white powder.
UV spectrum ($\lambda_{max}^{MeOH}$) nm: 233, 240, 252, 280, 289
Mass spectrum (m/e): 607, 471, 456, 436

Example 33

*Bacillus megaterium* IFO 12108 was cultivated as in Example 19, and 140 mg of dechloromaytansinol 3-isobutyrate were added to 2.8 l of the resulting culture. The reaction was conducted under shaking at 28°C for 50 hours. As assayed by TLC, the dechloromaytansinol 3-isobutyrate had disappeared and, instead, demethyldechloromaytansinol 3-isobutyrate had been formed in the broth.

Example 34

The broth obtained in Example 33 was purified as in Example 20 and TLC was carried out thereon as in Example 20. The fractions at Rf = 0.42 were collected to obtain 113 mg of crude demethyldechloromaytansinol 3-isobutyrate (ii). This crude product (ii) was washed with a small amount of diethyl ether, dissolved in ethyl acetate and allowed to stand. By the above procedure there were obtained 76 mg crystals of demethyldechloromaytansinol 3-isobutyrate.
m.p.213—215°C.
Specific rotation: −116.6° (c = 0.47, $CHCl_3$)
Elemental analysis (%):

| | |
|---|---|
| Found | C, 63.27; H, 7.33; N, 4.61 |
| Calcd. for $C_{31}H_{42}N_2O_9$ | C, 63.46; H, 7.22; N, 4.78 |

UV spectrum ($\lambda_{max}^{MeOH}$) nm ($\varepsilon$): 230 (27200), 240 (29700) 248 (29700), 277 (4100), 285 (3700)
Mass spectrum (m/e): 525, 437, 422

Example 35

*Streptomyces platensis* IFO 12901 was cultivated as in Example 9 and 440 mg of L-maytansine were added to 4.4 l of the culture. The reaction was carried out under shaking at 28°C for 48 hours. As assayed by TLC, the maytansine had completely disappeared and, instead, L-demethylmaytansine had been formed in the broth.

Example 36

To 4.4 l of the broth obtained in Example 35 were added 2.2 l of ethyl acetate and the resulting extract was suction-filtered through a strainer with 40 g of Hyflo Super Cel (trade mark) (Johns Manville Products Co., U.S.A.). This procedure was repeated for a second time. The extracts were combined, washed successively with 1.2 l of 1/200N-hydrochloric acid and 1 l of 0.5% aqueous sodium

bicarbonate, dried with 20 g. of anhydrous sodium sulfate and concentrated under reduced pressure to 2 ml. To the residue were added 50 ml of petroleum ether and the resulting precipitate was recovered by filtration (252 mg). This crude L-demethylmaytansinol (i) was dissolved in a small amount of chloroform and run onto a column (1.2 cm dia.) of 10 g silica gel (Merck, Germany, 0.05—0.2 mm), elution being carried out with 100 ml of chloroform, 200 ml of chloroform/methanol (20:1) and 200 ml of chloroform/methanol (10:1). The eluate was collected in 2 ml fractions and each fraction was applied to a silica gel-glass plate (Merck, Germany, Kieselgel 60 $F_{254}$, 0.25 mm, 20 x 20 cm) at a distance of 2.5 cm from its bottom edge. The chromatogram was developed over a distance of 17 cm with ethyl acetate/methanol (19:1) and the UV absorbing zone (2537 Å) was detected. Fractions Nos. 13—17 which absorbed at Rf = 0.23 were collected and concentrated under reduced pressure to 1 ml. 30 ml of petroleum ether were added to the concentrate. By the above procedure there were obtained 183 mg of crude L-demethylmaytansine (ii).

Example 37

183 mg of the crude L-demethylmaytansine (ii) obtained in Example 36 were dissolved in a small amount of chloroform, and the resulting solution was linearly applied to each of 6 silica gel-glass plates (Merck, Germany, Kieselgel $F_{254}$, 2 mm, 20 x 20 cm) at a distance of 2.5 cm from the bottom edge of each plate. The chromatogram was developed with ethyl acetate/methanol (19:1) and the zone absorbing at Rf 0.23 was scraped up and extracted twice with ethyl acetate containing a small amount of water. The resulting ethyl acetate extract was washed with water, dried over anhydrous sodium sulfate and concentrated under reduced pressure. Hexane was added to the concentrate and the mixture allowed to stand, whereby 147 mg crystals of L-demethylmaytansine were obtained.

Example 38

*Streptomyces platensis* IFO 12901 was cultivated as in Example 9, and 260 mg of D-maytansine were added to 2.6 l of the culture. The mixture was incubated at 28°C and under shaking for 31 hours. As assayed by TLC, the D-maytansine had disappeared and, instead, D-demethylmaytansine had been formed in the broth.

Example 39

The broth obtained in Example 38 was purified as in Example 36 and TLC was carried out thereon as in Example 36. The fractions absorbing at Rf = 0.08 were collected to yield 106 mg of crude D-demethylmaytansine. This crude product was further purified as in Example 37. By the above procedure there were obtained 63 mg of D-demethylmaytansine as a white powder.

Example 40

*Actinomyces nigrescens* IFO 12894 was cultivated as in Example 9, and 217 mg of maytanprine (DL) were added to 2.2 l of the culture. The mixture was incubated at 28°C and under shaking for 48 hours. As assayed by TLC, the maytanprine (D,L) had disappeared and, instead, demethylmaytanprine (D,L) had been formed in the broth.

Example 41

The broth obtained in Example 40 was purified as in Example 36 to yield 153 mg of crude demethylmaytanprine (D,L). Thereafter, as in Example 37, the crude product was linearly applied to each of 5 silica gel-glass plates at a distance of 2.5 cm from the bottom edge of each plate. The chromatogram was similarly developed with ethyl acetate/methanol (19:1) and the zones absorbing at Rf 0.40 and Rf 0.28 were each scraped up and extracted twice with ethyl acetate containing a small amount of water. The extracts were respectively washed with water, dried over anhydrous sodium sulfate, concentrated under reduced pressure and treated with petroleum ether. In the above manner, 47 mg of L-demethylmaytanprine were obtained as a white powder from the fraction absorbing at Rf 0.40 and 38 mg of D-demethylmaytanprine were obtained as a white powder from the fraction absorbing at Rf 0.28.

Example 42

*Actinomyces nigrescens* IFO 12894 was cultivated as in Example 9, and 315 mg of maytanbutine (D,L) were added to 3.5 l of the culture. The mixture was reacted at 28°C and under shaking for 48 hours. As assayed by TLC, the maytanbutine (D,L) had disappeared and, instead, demethylmaytanbutine had been formed in the broth.

Example 43

The broth obtained in Example 42 was purified as in Example 36 to yield 189 mg of crude demethylmaytanbutine (D,L). The preparative TLC was carried out on this crude product and the chromatogram was developed with ethyl acetate/methanol (19:1). The zones absorbing at Rf 0.47 and Rf 0.31 were respectively scraped up and treated as in Example 37. By this procedure, 85 mg of L-demethylmaytanbutine were obtained as white powder from the fraction absorbing at Rf 0.47 and

78mg of D-demethylmaytanbutine were obtained as white powder from the zone absorbing at Rf 0.31.

Example 44

*Actinomyces nigrescens* IFO 12894 was cultivated as in Example 9, and 530 mg of maytanvaline (D,L) were added to 5.3 l of the culture. The mixture was incubated at 28°C and under shaking for 48 hours. As assayed by TLC, the maytanvaline (D,L) had disappeared and, instead, demethylmaytanvaline had been produced in the broth.

Example 45

The broth obtained in Example 44 was purified as in Example 36 to yield 223 mg of crude demethylmaytanvaline (D,L). Preparative TLC was carried out by the same procedure as Example 37 and the chromatogram was developed with ethyl acetate-methanol. The zones absorbing at Rf 0.55 and Rf 0.40 were respectively scraped up and treated as in Example 37. In this manner, 95 mg of L-demethylmaytanvaline were obtained as a white powder from the fraction absorbing at Rf 0.55 and 88 mg of D-demethylmaytanvaline were obtained as a white powder from the fraction absorbing at Rf 0.40.

Example 46

*Streptomyces flavotricini* IFO 12770 was cultivated as in Example 9, and 40 mg of L-maytansine were added to 4 l of the resulting culture. The mixture was incubated at 28°C under shaking for 48 hours and the resulting broth was purified as in Examples 36 and 37. By the above procedure there were obtained 5 mg of L-demethylmaytansine as a white powder.

Example 47

*Streptomyces flavotricini* IFO 12770 was cultivated as in Example 9, and 40 mg of D-maytansine were added to 4 l of the culture broth. The mixture was incubated at 28°C and under shaking for 48 hours, and, then, treated as in Examples 36 and 37. A small amount of ethyl acetate was added to the broth and the mixture was allowed to stand. The above procedure yielded 6 mg of crystals of D-demethylmaytansine.

Example 48

*Actinomyces nigrescens* IFO 12894 was cultivated as in Example 9, and 10 mg of L-maytansine were added to 1 l of the culture. The mixture was incubated at 28°C and under shaking for 48 hours. As assayed by TLC, the L-maytansine had decreased and, instead, L-demethylmaytansine had been produced in the broth.

Example 49

*Streptomyces libani* IFO 13452 was cultivated as in Example 9, and 10 mg of L-maytansine were added to 1 l of the culture. The mixture was reacted at 28°C and under shaking for 48 hours. As assayed by TLC, the titer of L-maytansine had been reduced and, instead, L-demethylmaytansine had been formed in the broth.

The physico-chemical properties of the compounds obtained in the foregoing Examples are shown in the following Table 5:

TABLE 5

| | Demethylmaytansine (L) | Demethylmantansine (D) |
|---|---|---|
| m.p. | 194–196 °C | |
| Specific rotation | −117.9° ($c = 0.56$ ETOH) | |
| Mol. formula | $C_{33}H_{44}ClN_3O_{10}$ | $C_{33}H_{44}ClN_3O_{10}$ |
| Mass spectrum, m/e | 616, 471, 456,<br>436, 128 | 616, 471, 456,<br>436, 128 |
| UV $\lambda_{max}^{MeOH}$ nm | 288, 281, 252,<br>243, 232 | 288, 281, 252,<br>243, 232 |
| NMR<br>Main peaks<br>(methyl signals) | 0.82, 3H, s<br>1.29, 3H, d, J = 6<br>1.33, 3H, d, J = 7<br>1.66, 3H, s<br>2.14, 3H, s<br>2.89, 3H, s<br>3.23, 3H, s<br>3.39, 3H, s | 0.87, 3H, s<br>1.28, 3H, d, J = 6<br>1.52, 3H, d, J = 7<br>1.72, 3H, s<br>2.19, 3H, s<br>3.06, 3H, s<br>3.18, 3H, s<br>3.38, 3H, s |
| TLC<br>.$H_2O$-saturated ethyl-acetate | 0.12 | 0.04 |
| .Ethyl acetate-methanol (19:1) | 0.23 | 0.08 |

TABLE 5 (Continued)

| | Demethylmaytanprine (L) | Demethylmaytanprine (D) |
|---|---|---|
| m.p. | | |
| Specific rotation | | |
| Mol. formula | $C_{34}H_{46}ClN_3O_{10}$ | $C_{34}H_{46}ClN_3O_{10}$ |
| Mass spectrum, m/e | 630, 471, 456, 436, 142 | 630, 471, 456, 436, 142 |
| UV $\lambda_{max}^{MeOH}$ nm | 288, 281, 252, 243, 232 | 288, 281, 252, 243, 232 |
| NMR Main peaks (methyl signals) | 0.83, 3H, s<br>1.13, 3H, t, J = 7<br>1.29, 3H, d, J = 6<br>1.33, 3H, d, J = 7<br>1.65, 3H, s<br>2.87, 3H, s<br>3.23, 3H, s<br>3.38, 3H, s | 0.89, 3H, s<br>1.16, 3H, d, J = 7<br>1.28, 3H, d, J = 6<br>1.52, 3H, d, J = 7<br>1.72, 3H, s<br>3.04, 3H, s<br>3.18, 3H, s<br>3.37, 3H, s |
| TLC .$H_2O$-saturated ethyl-acetate | 0.21 | 0.15 |
| .Ethyl acetate-methanol (19 : 1) | 0.40 | 0.28 |

TABLE 5 (Continued)

| | Demethylmaytanbutine (L) | Demethylmaytanbutine (D) |
|---|---|---|
| Mol. formula | $C_{35}H_{48}ClN_3O_{10}$ | $C_{35}H_{48}ClN_3O_{10}$ |
| Mass spectrum, m/e | 644, 471, 456,<br>436, 156 | 644, 471, 456,<br>436, 156 |
| UV $\lambda_{max}^{MeOH}$ nm | 288, 281, 252,<br>243, 232 | 288, 281, 252,<br>243, 232 |
| NMR<br>Main peaks<br>(methyl signals) | 0.83, 3H, s<br>1.12, 6H, d, J = 7<br>1.29, 3H, d, J = 6<br>1.33, 3H, d, J = 7<br>1.67, 3H, s<br>2.86, 3H, s<br>3.21, 3H, s<br>3.41, 3H, s | 0.89, 3H, s<br>1.18, 6H, d, J = 7<br>1.29, 3H, d, J = 6<br>1.52, 3H, d, J = 7<br>1.72, 3H, s<br>3.08, 3H, s<br>3.21, 3H, s<br>3.37, 3H, s |
| TLC<br>.$H_2O$-saturated ethyl-acetate<br>.Ethyl acetate-methanol (19 : 1) | <br>0.25<br>0.47 | <br>0.16<br>0.31 |

TABLE 5 (Continued)

| | Demethylmaytanvaline (L) | Demethylmaytanvaline (D) |
|---|---|---|
| Mol. formula | $C_{36}H_{50}ClN_3O_{10}$ | $C_{36}H_{50}ClN_3O_{10}$ |
| Mass spectrum, m/e | 658, 471, 456, 436, 170 | 658, 471, 456, 436, 170 |
| UV $\lambda_{max}^{MeOH}$ nm | 288, 281, 252, 243, 232 | 288, 281, 252, 243, 232 |
| NMR Main peaks (methyl signals) | 0.82, 3H, s<br>0.94, 3H, d, J = 6<br>0.98, 3H, d, J = 6<br>1.29, 3H, d, J = 6<br>1.33, 3H, d, J = 7<br>1.67, 3H, s<br>2.89, 3H, s<br>3.22, 3H, s<br>3.39, 3H, s | 0.92, 3H, s<br>0.96, 3H, d, J = 6<br>0.99, 3H, d, J = 6<br>1.29, 3H, d, J = 6<br>1.52, 3H, d, J = 7<br>1.72, 3H, s<br>3.04, 3H, s<br>3.17, 3H, s<br>3.37, 3H, s |
| TLC .$H_2O$-saturated ethyl-acetate | 0.30 | 0.20 |
| .Ethyl acetate-methanol (19:1) | 0.55 | 0.40 |

Example 50

Using *Bacillus megaterium* IFO 12108, procedures similar to those of Examples 1 to 8, 19 to 28 and 33 to 34 were conducted to obtain the following compounds. The table below sets forth the starting compounds, the product compounds and the Rf values of the product compounds (developer solvent: $CHCl_3$/MeOH 9:1; plate: silica gel glass plate (Merck, Germany, 60 $F_{254}$, 0.25 mm thick)):

| Starting compound (II) | Product compound (III) | Rf value |
|---|---|---|
| Maytansinol | Demethylmaytansinol | 0.30 |
| Maytanacine | Demethylmaytanacine | 0.38 |
| Maytansinol propionate | Demethylmaytansinol propionate | 0.40 |
| Ansamitocin P–3 | PDM–3 | 0.42 |
| Ansamitocin P–3′ | PDM–3′ | 0.43 |
| Ansamitocin P–4 | PDM–4 | 0.44 |
| Maytansinol 3-valerate | Demethylmaytansinol 3-valerate | 0.45 |
| Maytansinol 3-hexanoate | Demethylmaytansinol 3-hexanoate | 0.48 |
| Maytansinol 3-heptanoate | Demethylmaytansinol 3-heptanoate | 0.49 |
| Maytansinol 3-octanoate | Demethylmaytansinol 3-octanoate | 0.50 |
| Maytansinol 3-phenylacetate | Demethylmaytansinol 3-phenylacetate | 0.43 |
| Maytansinol 3-phenylpropionate | Demethylmaytansinol 3-phenylpropionate | 0.45 |
| Maytansinol 3-benzoate | Demethylmaytansinol 3-benzoate | 0.42 |
| Maytansinol 3-cyclohexane-carboxylate | Demethylmaytansinol 3-cyclo-hexanecarboxylate | 0.45 |
| Maytansinol 3-*p*-chloro-benzoate | Demethylmaytansinol 3-*p*-chlorobenzoate | 0.43 |
| Maytansinol 3-nicotinate | Demethylmaytansinol 3-nicotinate | 0.31 |
| Maytansinol 3-isonicotinate | Demethylmaytansinol 3-isonicotinate | 0.30 |

| Starting compound (II) | Product compound (III) | Rf value |
|---|---|---|
| Maytansinol 3-picolinate | Demethylmaytansinol 3-picolinate | 0.40 |
| Maytansinol 3-(2-furan)-carboxylate | Demethylmaytansinol 3-(2-furan) carboxylate | 0.38 |
| Maytansinol 3-cyclopropane-carboxylate | Demethylmaytansinol 3-cyclo-propanecarboxylate | 0.39 |
| Dechloromaytansinol | Demethyldechloromaytansinol | 0.28 |
| Dechloromaytansinol 3-isobutyrate | Demethyldechloromaytansinol 3-isobutyrate | 0.42 |
| Dechloromaytansinol 3-phenylacetate | Demethyldechloromaytansinol 3-phenylacetate | 0.43 |
| Dechloromaytansinol 3-nicotinate | Demethyldechloromaytansinol 3-nicotinate | 0.31 |
| Dechloromaytansinol 3-cyclo-hexanecarboxylate | Demethyldechloromaytansinol 3-cyclohexanecarboxylate | 0.45 |
| D-Maytanvaline | Demethyl-D-maytanvaline | 0.47 |
| Maytansinol 3-(N-methyl)-carbamate | Demethylmaytansinol 3-(N-methyl) carbamate | 0.27 |
| Maytansinol 3-(N-butyl)-carbamate | Demethylmaytansinol 3-(N-butyl) carbamate | 0.39 |
| Maytansinol 3-(N-phenyl)-carbamate | Demethylmaytansinol 3-(N-phenyl) carbamate | 0.40 |
| Maytansinol 3-(N-cyclohexyl)-carbamate | Demethylmaytansinol 3-(N-cyclohexyl) carbamate | 0.39 |
| Maytansinol 3-(3-pyridyl)-carbamate | Demethylmaytansinol 3-(3-pyridyl) carbamate | 0.22 |
| Maytansinol 3-isopropyl-carbonate | Demethylmaytansinol 3-iso-propylcarbonate | 0.43 |
| Dechloromaytansinol 3-benzyl-carbonate | Demethyldechloromaytansinol 3-benzylcarbamate | 0.44 |

| Starting compound (II) | Product compound (III) | Rf value |
|---|---|---|
| Maytansinol 3-phenyl-carbonate | Demethylmaytansinol 3-phenyl-carbonate | 0.38 |

Example 51

Using *Streptomyces flavotricini* IFO 12770, procedures similar to those of Examples 9, 29, 30 and 46 were conducted to obtain the following compounds.

The table below sets forth the starting compounds, the product compounds and the Rf values of the product compounds (developer solvent: $CHCl_3$/MeOH 9:1; plate: silica gel-glass plate (Merck, Germany 60 $F_{254}$, 0.25 mm thick)):

| Starting compound (II) | Product compound (III) | Rf value |
|---|---|---|
| Maytansinol | Demethylmaytansinol | 0.30 |
| Maytanacine | Demethylmaytanacine | 0.38 |
| Maytansinol propionate | Demethylmaytansinol propionate | 0.40 |
| Ansamitocin P–3 | PDM–3 | 0.42 |
| Ansamitocin P–3′ | PDM–3′ | 0.43 |
| Ansamitocin P–4 | PDM–4 | 0.44 |
| Maytansinol 3-valerate | Demethylmaytansinol 3-valerate | 0.45 |
| Maytansinol 3-hexanoate | Demethylmaytansinol 3-hexanoate | 0.48 |
| Maytansinol 3-heptanoate | Demethylmaytansinol 3-heptanoate | 0.49 |
| Maytansinol 3-octanoate | Demethylmaytansinol 3-octanoate | 0.50 |
| Maytansinol 3-phenylacetate | Demethylmaytansinol 3-phenylacetate | 0.43 |
| Maytansinol 3-phenyl-propionate | Demethylmayransinol phenylpropionate | 0.45 |

| Starting compound (II) | Product compound (III) | Rf value |
|---|---|---|
| Maytansinol 3-benzoate | Demethylmaytansinol 3-benzoate | 0.42 |
| Maytansinol 3-cyclohexane-carboxylate | Demethylmaytansinol 3-cyclo-hexanecarboxylate | 0.45 |
| Maytansinol 3-*p*-chloro-benzoate | Demethylmaytansinol 3-*p*-chlorobenzoate | 0.43 |
| Maytansinol 3-nicotinate | Demethylmaytansinol 3-nicotinate | 0.31 |
| Maytansinol 3-isonicotinate | Demethylmaytansinol 3-isonicotinate | 0.30 |
| Maytansinol 3-picolinate | Demethylmaytansinol 3-picolinate | 0.40 |
| Maytansinol 3-(2-furan) carboxylate | Demethylmaytansinol 3-(2-furan) carboxylate | 0.38 |
| Maytansinol 3-cyclopropane-carboxylate | Demethylmaytansinol 3-cyclo-propanecarboxylate | 0.39 |
| Dechloromaytansinol 3-isobutyrate | Demethyldechloromaytansinol 3-isobutyrate | 0.42 |
| Dechloromaytansinol 3-phenylacetate | Demethyldechloromaytansinol 3-phenylacetate | 0.43 |
| Dechloromaytansinol 3-nicotinate | Demethyldechloromaytansinol 3-nicotinate | 0.31 |
| Dechloromaytansinol 3-cyclo-hexanecarboxylate | Demethyldechloromaytansinol 3-cyclohexanecarboxylate | 0.45 |
| Maytansinol 3-(N-acetyl-N-benzyl) alanine ester [m.p. 174–177 °C (decomp.)] | Demethylmaytansinol 3-(N-acetyl-N-benzyl) alanine ester | 0.46 |
| Maytansinol 3-(N-acetyl-N-methyl)-L-leucine ester | Demethylmaytansinol 3-(N-acetyl-N-methyl)-L-leucine ester | 0.43 |
| Maytansinol 3-(N-acetyl-N-methyl)-phenylalanine ester [m.p. 189–193 °C (decomp.)] | Demethylmaytansinol 3-(N-acetyl-N-methyl)-phenyl-alanine ester | 0.45 |

| Starting compound (II) | Product compound (III) | Rf value |
|---|---|---|
| L-maytansine | Demethyl-L-maytansine | 0.39 |
| L-maytansine | Demethyl-D-maytansine | 0.39 |
| L-maytanprine | Demethyl-L-maytanprine | 0.43 |
| L-maytanprine | Demethyl-D-maytanprine | 0.44 |
| L-maytanbutine | Demethyl-L-maytanbutine | 0.44 |
| D-maytanbutine | Demethyl-D-maytanbutine | 0.46 |
| D-maytanvaline | Demethyl-D-maytanvaline | 0.47 |
| Dechloro-D-maytansine | Demethyldechloro-D-maytansine | 0.39 |
| Maytansinol 3-(N-methyl)-carbamate | Demethylmaytansinol 3-(N-methyl) carbamate | 0.27 |
| Maytansinol 3-(N-butyl)-carbamate | Demethylmaytansinol 3-(N-butyl) carbamate | 0.39 |
| Maytansinol 3-(N-phenyl)-carbamate | Demethylmaytansinol 3-(N-phenyl) carbamate | 0.40 |
| Maytansinol 3-(N-cyclohexyl)-carbamate | Demethylmaytansinol 3-(N-cyclohexyl) carbamate | 0.39 |
| Maytansinol 3-(3-pyridyl)-carbamate | Demethylmaytansinol 3-(3-pyridyl) carbamate | 0.22 |
| Maytansinol 3-isopropyl-carbonate | Demethylmaytansinol 3-iso-propylcarbonate | 0.43 |
| Dechloromaytansinol 3-benzyl-carbonate | Demethyldechloromaytansinol 3-benzylcarbonate | 0.44 |

Example 52

Using *Streptomyces platensis* IFO 12901, procedures similar to those of Examples 12, 31, 32 and 35 to 39 were conducted to obtain the following compounds. The table below sets forth the starting compounds, the product compounds and the Rf values of the product compounds (developer solvent: $CHCl_3$/MeOH 9:1; plate: silica gel-glass plate (Merck, Germany 60 $F_{254}$, 0.25 mm thick)):

| Starting compound (II) | Product compound (III) | Rf value |
|---|---|---|
| Maytanacine | Demethylmaytanacine | 0.38 |
| Maytansinol propionate | Demethylmaytansinol propionate | 0.40 |
| Ansamitocin P–3 | PDM–3 | 0.42 |
| Ansamitocin P–3′ | PDM–3′ | 0.43 |
| Ansamitocin P–4 | PDM–4 | 0.44 |
| Maytansinol 3-valerate | Demethylmaytansinol 3-valerate | 0.45 |
| Maytansinol 3-hexanoate | Demethylmaytansinol 3-hexanoate | 0.48 |
| Maytansinol 3-heptanoate | Demethylmaytansinol 3-heptanoate | 0.49 |
| Maytansinol 3-octanoate | Demethylmaytansinol 3-octanoate | 0.50 |
| Maytansinol 3-phenyl-acetate | Demethylmaytansinol 3-phenyl-acetate | 0.43 |
| Maytansinol 3-phenyl-propionate | Demethylmaytansinol 3-phenyl-propionate | 0.45 |
| Maytansinol 3-benzoate | Demethylmaytansinol 3-benzoate | 0.42 |
| Maytansinol 3-cyclohexane-carboxylate | Demethylmaytansinol 3-cyclo-hexanecarboxylate | 0.45 |
| Maytansinol 3-*p*-chloro-benzoate | Demethylmaytansinol 3-*p*-chlorobenzoate | 0.43 |
| Maytansinol 3-nicotinate | Demethylmaytansinol 3-nicotinate | 0.31 |
| Maytansinol 3-isonicotinate | Demethylmaytansinol 3-iso-nicotinate | 0.30 |
| Maytansinol 3-picolinate | Demethylmaytansinol 3-picolinate | 0.40 |

| Starting compound (II) | Product compound (III) | Rf value |
|---|---|---|
| Maytansinol 3-cyclopropane-carboxylate | Demethylmaytansinol 3-cyclo-propanecarboxylate | 0.39 |
| Dechloromaytansinol 3-iso-butyrate | Demethyldechloromaytansinol 3-isobutyrate | 0.42 |
| Dechloromaytansinol 3-phenyl-acetate | Demethyldechloromaytansinol 3-phenylacetate | 0.43 |
| Dechloromaytansinol 3-nicotinate | Demethyldechloromaytansinol 3-nicotinate | 0.31 |
| Dechloromaytansinol 3-cyclo-hexanecarboxylate | Demethyldechloromaytansinol 3-cyclohexanecarboxylate | 0.45 |
| Maytansinol 3-(N-acetyl-N-benzyl) alanine ester [m.p. 174–177 °C (decomp.)] | Demethylmaytansinol 3-(N-acetyl-N-benzyl) alanine ester | 0.46 |
| Maytansinol 3-(N-acetyl-N-benzyl) alanine ester [m.p. 163–166 °C (decomp.)] | Demethylmaytansinol 3-(N-acetyl-N-benzyl) alanine ester | 0.45 |
| Maytansinol 3-(N-acetyl-N-methyl)-L-leucine ester | Demethylmaytansinol 3-(N-acetyl-N-methyl)-L-leucine ester | 0.43 |
| Maytansinol 3-(N-acetyl-N-methyl) phenylalanine ester [m.p. 189–193 °C (decomp.)] | Demethylmaytansinol 3-(N-acetyl-N-methyl) phenylalanine ester | 0.45 |
| Maytansinol 3-(N-acetyl-N-methyl) phenylalanine ester [m.p. 212–214 °C (decomp.)] | Demethylmaytansinol 3-(N-acetyl-N-methyl) phenylalanine ester | 0.42 |
| L-maytansine | Demethyl-L-maytansine | 0.39 |
| D-maytansine | Demethyl-D-maytansine | 0.39 |
| L-maytanprine | Demethyl-L-maytanprine | 0.43 |
| D-maytanprine | Demethyl-D-mantanprine | 0.44 |
| L-maytanbutine | Demethyl-L-maytanbutine | 0.44 |
| D-maytanbutine | Demethyl-D-maytanbutine | 0.46 |
| L-maytanvaline | Demethyl-L-maytanvaline | 0.45 |

| Starting compound (II) | Product compound (III) | Rf value |
|---|---|---|
| D-maytanvaline | Demethyl-D-maytanvaline | 0.47 |
| Dechloro-D-maytansine | Demethyldechloro-D-maytansine | 0.39 |
| Maytansinol 3-(N-butyl)-carbamate | Demethylmaytansinol 3-(N-butyl) carbamate | 0.39 |
| Maytansinol 3-(N-phenyl)-carbamate | Demethylmaytansinol 3-(N-phenyl) carbamate | 0.40 |
| Maytansinol 3-(N-cyclohexyl)-carbamate | Demethylmaytansinol 3-(N-cyclohexyl) carbamate | 0.39 |
| Maytansinol 3-(3-pyridyl)-carbamate | Demethylmaytansinol 3-(3-pyridyl) carbamate | 0.22 |
| Maytansinol 3-isopropyl-carbonate | Demethylmaytansinol 3-iso-propylcarbamate | 0.43 |

Example 53

In 20 ml of tetrahydrofuran were dissolved 50 mg of crystals of demethyldechloromaytansinol 3-isobutyrate crystals obtained in Example 34. After the solution was cooled to −5°C, 50 mg of lithium aluminium hydride were added. The reaction mixture was treated as in Example 13 and preparative TLC on silica gel was carried out with ethyl acetate/methanol (19:1) over a distance of 17 cm, the zone absorbing at Rf = 0.20 to 0.25 being scraped up and extracted with ethyl acetate containing a small amount of water. The extract was washed with water, dried over anhydrous sodium sulfate and concentrated under reduced pressure. By the above procedure there were obtained 43 mg of demethyldechloromaytansinol as a powdery residue. It was then dissolved in a small amount of ethyl acetate, the solution was allowed to stand and the resulting crystals were collected by filtration and dried. Yield: 33 mg.

m.p. 198—201°C (decomp.).

Elemental analysis (%):

| | |
|---|---|
| Found | C, 62.48; H, 7.25; N, 5.19; O, 24.89 |
| Calcd. for $C_{27}H_{36}N_2O_8$ | C, 62.77; H, 7.02; N, 5.42; O, 24.77 |

Mass spectrum (m/e): 455, 437

UV spectrum ($\lambda_{max}^{MeOH}$) nm: 230, 240, 248, 277, 285

Example 54

In 20 ml of tetrahydrofuran there were dissolved 50 mg of crystals of L-demethylmaytansine obtained in Example 37. After the solution had been cooled to −5°C, 50 mg of lithium aluminium hydride were added. The reaction mixture was put in a water bath and stirred for 30 minutes. After the addition of 10 ml ethyl acetate and 10 ml of 1/200N-HCl, the mixture was extracted with 50 ml of ethyl acetate. The ethyl acetate layer was washed with water, dried by the addition of anhydrous sodium sulfate and concentrated under reduced pressure. Preparative TLC of the product was carried out on the residue with silica gel and with ethyl acetate/methanol (19:1), over a distance of 17 cm. The zone absorbing at Rf = 0.25 was scraped up and extracted with ethyl acetate containing a small amount of water. The extract was washed with water, dried over anhydrous sodium sulfate and concentrated under reduced pressure. By the above procedure there were obtained 41 mg of demethylmaytansinol.

The physico-chemical properties of this product were identical with those of the demethylmaytansinol obtained in Example 5.

m.p. 195°C

Elemental analysis (%):

| | |
|---|---|
| Found | C, 58.67; H, 6.54; N, 4.83; Cl, 6.19 |
| Calcd. for $C_{27}H_{35}ClN_2O_8$ | C, 58.85; H, 6.40; N, 5.08; Cl, 6.43 |

Mass spectrum (m/e): 489, 471
UV spectrum (nm): 232, 242, 251, 280, 288.

Example 55

In 14 ml of tetrahydrofuran were dissolved 35 mg of the D-demethylmaytansine in powder form obtained in Example 39. The solution was cooled to —5°C, and 35 mg of lithium aluminium hydride were added. The mixture was treated and the product purified as in Example 54 to give 19 mg of demethylmaytansinol as a white powder. The physico-chemical properties of this product were identical with those of the demethylmaytansinol obtained in Example 54. m.p. 196°C

Example 56

In 14 ml of tetrahydrofuran were dissolved 30 mg of the demethylmaytansinol 3-(N-phenyl)carbamate powder obtained in Example 22. After the solution had been cooled to —5°C or below, 30 mg of lithium aluminium hydride were added. A similar treatment and purification procedure to those in Example 54 gave 17 mg of demethylmaytansinol as a white powder. The physico-chemical properties of this product were identical with those of the demethylmaytansinol obtained in Example 54. m.p. 194°C.

**Claims**

1. A demethylmaytansinoid compound of the formula (I):—

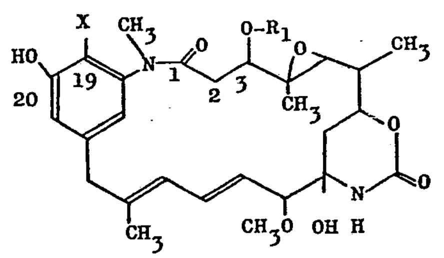

(I)

(wherein X is Cl or H; and $R_1$ is H or an acyl group).

2. A compound as claimed in Claim 1, wherein $R_1$ is an acyl group derived from a carboxylic acid, carbamic acid or half-ester of a carbonic acid derivative having a molecular weight of not more than 300.

3. A compound as claimed in Claim 1, wherein $R_1$ is an acyl group containing from 1 to 20 carbon atoms.

4. A compound as claimed in Claim 1, wherein $R_1$ is an acyl group selected from saturated or unsaturated aliphatic acyl groups; saturated or unsaturated alicyclic acyl groups; aromatic acyl groups; saturated or unsaturated heterocyclic acyl groups; N-acyl-$\alpha$-amino acid type acyl groups; carbamoyl type acyl groups; and acyl groups derived from half-esters of carbonic acid.

5. A compound as claimed in Claim 1, wherein X is Cl or H, and $R_1$ is an acyl group represented by the formula:—

$$—CO—R_2$$

(wherein $R_2$ is hydrogen or an optionally substituted or unsubstituted alkyl, alkenyl, cycloalkyl, cycloalkenyl, aryl or heterocyclic group, said cycloalkyl, cycloalkenyl, aryl or heterocyclic group being optionally attached to the carbonyl carbon through an alkylene chain).

6. A compound as claimed in Claim 1, wherein X is Cl or H, and $R_1$ is an acyl group represented by the formula:

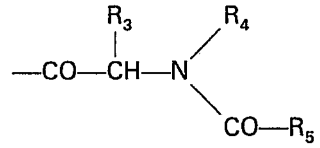

(wherein $R_3$ is hydrogen or an optionally substituted or unsubstituted alkyl, cycloalkyl, aryl, indolyl or imidazolyl group, said cycloalkyl, aryl, indolyl or imidazolyl groups being optionally attached to the *alpha*-carbon atom through an alkylene chain; $R_4$ is hydrogen or an optionally substituted or

unsubstituted alkyl, cycloalkyl, cycloalkylalkyl, aryl or benzyl group; and $R_5$ is hydrogen, alkoxy, bornyloxy, isobornyloxy, benzyloxy or an optionally substituted or unsubstituted alkyl, alkenyl, cycloalkyl, cycloalkenyl, aryl or heterocyclic group, said cycloalkyl, cycloalkenyl, aryl or heterocyclic groups being optionally attached to the carbonyl carbon atom adjacent the nitrogen atom through an alkylene chain).

7. A compound as claimed in Claim 1, wherein X is Cl or H, and $R_1$ is an acyl group represented by the formula:

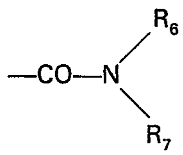

(wherein $R_6$ and $R_7$ may be the same or different and each is hydrogen or an optionally substituted or unsubstituted hydrocarbon residue or heterocyclic group, $R_6$ and $R_7$ optionally forming a heterocyclic group as taken together with the adjacent nitrogen atom).

8. A compound as claimed in Claim 1, wherein X is Cl or H and $R_1$ is an acyl group represented by the formula:

$$—CO—O—R_8$$

(wherein $R_8$ is an optionally substituted or unsubstituted hydrocarbon residue).

9. A compound as claimed in Claim 8, wherein the optionally substituted or unsubstituted hydrocarbon residue $R_8$ is an optionally substituted or unsubstituted alkyl, cycloalkyl, aryl or aralkyl.

10. A demethyl maytansinoid compound of the formula (I) as claimed in Claim 1, wherein said compound (I) is 20-demethoxy-20-hydroxymaytanacine.

11. A demethyl maytansinoid compound of the formula (I) as claimed in Claim 1, wherein said compound (I) is 20-demethoxy-20-hydroxymaytansinol propionate.

12. A demethyl maytansinoid compound of the formula (I) as claimed in Claim 1, wherein said compound (I) is 20-demethoxy-20-hydroxymaytansinol 3-isobutyrate.

13. A demethyl maytansinoid compound of the formula (I) as claimed in Claim 1, wherein said compound (I) is 20-demethoxy-20-hydroxymaytansinol 3-butyrate.

14. A demethyl maytansinoid compound of the formula (I) as claimed in Claim 1, wherein said compound (I) is 20-demethoxy-20-hydroxymaytansinol 3-isovalerate.

15. A demethyl maytansinoid compound of the formula (I) as claimed in Claim 1, wherein said compound (I) is 20-demethoxy-20-hydroxymaytansinol 3-valerate.

16. A demethyl maytansinoid compound of the formula (I) as claimed in Claim 1, wherein said compound (I) is 20-demethoxy-20-hydroxymaytansinol 3-hexanoate.

17. A demethyl maytansinoid compound of the formula (I) as claimed in Claim 1, wherein said compound (I) is 20-demethoxy-20-hydroxymaytansinol 3-cyclohexanecarboxylate.

18. A demethyl maytansinoid compound of the formula (I) as claimed in Claim 1, wherein said compound (I) is 20-demethoxy-20-hydroxymaytansinol 3-*p*-chlorobenzoate.

19. A demethyl maytansinoid compound of the formula (I) as claimed in Claim 1, wherein said compound (I) is 20-demethoxy-20-hydroxymaytansinol 3-phenylacetate.

20. A demethyl maytansinoid compound of the formula (I) as claimed in Claim 1, wherein said compound (I) is 20-demethoxy-20-hydroxymaytansinol 3-phenylpropionate.

21. A demethyl maytansinoid compound of the formula (I) as claimed in Claim 1, wherein said compound (I) is 20-demethoxy-20-hydroxymaytansinol 3-picolinate.

22. A demethyl maytansinoid compound of the formula (I) as claimed in Claim 1, wherein said compound (I) is 20-demethoxy-20-hydroxymaytansinol 3-(2-furan)-carboxylate.

23. A demethyl maytansinoid compound of the formula (I) as claimed in Claim 1, wherein said compound (I) is 20-demethoxy-20-hydroxydechloromaytansinol-3-phenylacetate.

24. A demethyl maytansinoid compound of the formula (I) as claimed in Claim 1, wherein said compound (I) is 20-demethoxy-20-hydroxydechloromaytansinol-3-isobutyrate.

25. A demethyl maytansinoid compound of the formula (I) as claimed in Claim 1, wherein said compound (I) is 20-demethoxy-20-hydroxyl-L-maytansine.

26. A demethyl maytansinoid compound of the formula (I) as claimed in Claim 1, wherein said compound (I) is 20-demethoxy-20-hydroxy-L-maytanprine.

27. A demethyl maytansinoid compound of the formula (I) as claimed in Claim 1, wherein said compound (I) is 20-demethoxy-20-hydroxy-L-maytanbutine.

28. A demethyl maytansinoid compound of the formula (I) as claimed in Claim 1, wherein said compound (I) is 20-demethoxy-20-hydroxy-L-maytanvaline.

29. A demethyl maytansinoid compound of the formula (I) as claimed in Claim 1, wherein said compound (I) is 20-demethoxy-20-hydroxymaytansinol 3-(N-acetyl-N-benzyl)alanine ester.

30. A demethyl maytansinoid compound of the formula (I) as claimed in Claim 1, wherein said compound (I) is 20-demethoxy-20-hydroxymaytansinol 3-(N-acetyl-N-methyl)-L-leucine ester.

31. A demethyl maytansinoid compound of the formula (I) as claimed in Claim 1, wherein said compound (I) is 20-demethoxy-20-hydroxymaytansinol 3-(N-acetyl-N-methyl)phenylalanine ester.

32. A demethyl maytansinoid compound of the formula (I) as claimed in Claim 1, wherein said compound (I) is 20-demethoxy-20-hydroxymaytansinol 3-(N-phenyl)-carbamate.

33. A demethyl maytansinoid compound of the formula (I) as claimed in Claim 1, wherein said compound (I) is 20-demethoxy-20-hydroxymaytansinol 3-isopropylcarbonate.

34. A demethyl maytansinoid compound of the formula (I) as claimed in Claim 1, wherein said compound (I) is 20-demethoxy-20-hydroxydechloromaytansinol-3-benzylcarbonate.

35. A demethyl maytansinoid compound of the formula (I) as claimed in Claim 1, wherein said compound (I) is 20-demethoxy-20-hydroxymaytansinol.

36. A demethyl maytansinoid compound of the formula (I) as claimed in Claim 1, wherein said compound (I) is 20-demethoxy-20-hydroxydechloromaytansinol.

37. A method of producing a demethylmaytansinoid compound of the formula (III):—

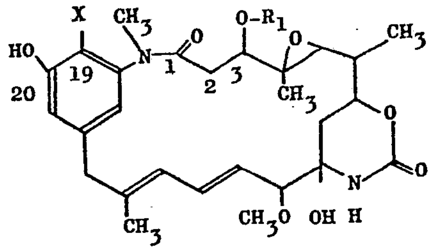

(I).

(wherein X is Cl or H; $R_1$ is H or acyl) characterised in that a maytansinoid compound of the formula (II):

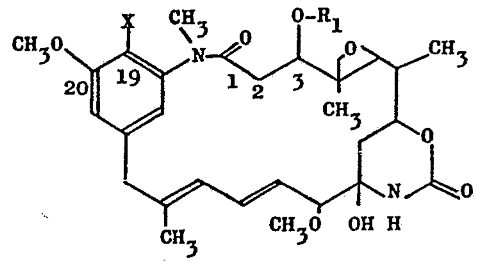

(II)

(wherein X and $R_1$ are as respectively defined above) is contacted with a culture broth, inclusive of a processed matter of the culture broth, of a microorganism belonging to the genus *Bacillus*, the genus *Streptomyces* or the genus *Actinomyces* which is able to transform the 20-methoxy group of said maytansinoid compound into a hydroxy group, the cells or disrupted cells containing a demethylation enzyme system.

38. A method as claimed in Claim 37, wherein the microorganism belongs to the species *Bacillus megaterium*, *Streptomyces flavotricini*, *Streptomyces platensis*, *Streptomyces libani* or *Actinomyces nigrescens*.

39. A method as claimed in Claim 38, wherein the microorganism is *Bacillus megaterium* IFO 12108, *Streptomyces flavotricini* IFO 12770, *Streptomyces platensis* IFO 12901, *Streptomyces libani* IFO 13452 or *Actinomyces nigrescens* IFO 12894.

40. A method of producing a demethylmaytansinoid compound of the formula (VI):

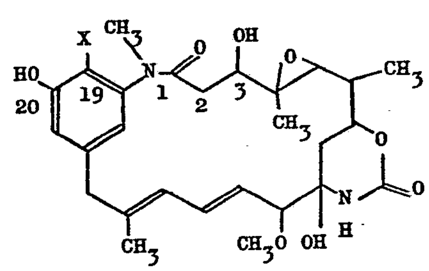

(VI)

(wherein X is Cl or H)
characterized in that a maytansinoid compound of the formula (IV):

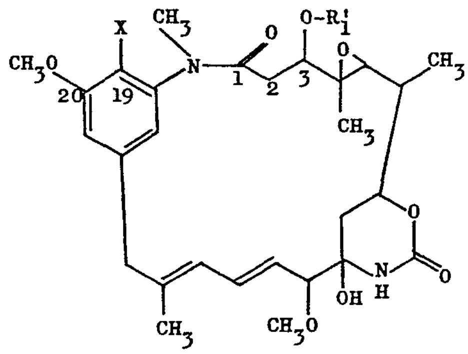

(IV)

(wherein X is as defined above; and $R'_1$ is acyl group) is contacted with a culture broth, inclusive of a processed matter of the culture broth, of a microorganism of the genus *Bacillus*, the genus *Streptomyces* or the genus *Actinomyces* which is able to transform the 20-methoxy group of said maytansinoid compound (IV) into a hydroxy group to yield a compound of the formula (V):

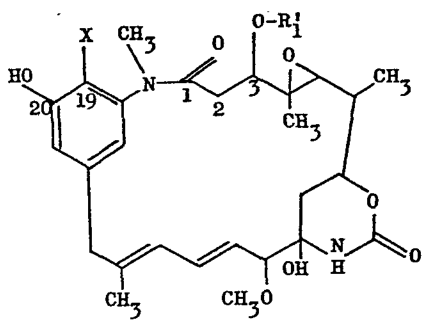

(V)

(wherein X and $R'_1$ are as respectively defined above), and this latter compound (V) is then subjected to deacyllation.

41. A method as claimed in Claim 40, wherein the microorganism belongs to the species *Bacillus megaterium, Streptomyces flavotricini, Streptomyces platensis, Streptomyces libani* or *Actinomyces nigrescens.*

42. A method as claimed in Claim 40, wherein the microorganism is *Bacillus megaterium* IFO 12108, *Streptomyces flavotricini* IFO 12770, *Streptomyces platensis* IFO 12901, *Streptomyces libani* IFO 13452 or *Actinomyces nigrescens* IFO 12894.

43. A method as claimed in any of Claims 40 to 42, wherein the deacylation is conducted by employing a complex metal hydride.

44. A method as claimed in Claim 43, wherein the complex metal hydride is lithium aluminium hydride.

45. Compound (I) as defined in Claim 1 or as claimed in any of Claims 2 to 36, inclusive for use as antifungal, antiprotozoal or antitumour agents.

**Revendications**

1. Composé déméthylmaytansinoïde de formule (I):

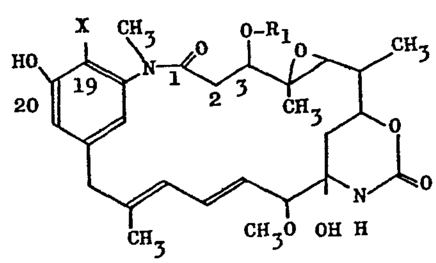

(I)

(dans laquelle X est Cl ou H; et $R_1$ est H ou un groupe acyle).

2. Composé selon la revendication 1, dans lequel $R_1$ est un groupe acyle provenant d'un acide carboxylique, d'un acide carbamique, ou d'un hémi-ester d'un dérivé d'acide carbonique ayant un poids moléculaire ne dépassant pas 300.

3. Composé selon la revendication 1, dans lequel $R_1$ est un groupe acyle contenant de 1 à 20 atomes de carbone.

4. Composé selon la revendication 1, dans lequel $R_1$ est un groupe acyle choisi parmi les groupes acyle aliphatiques saturés ou insaturés; les groupes acyle alicycliques saturés ou insaturés; les groupes acyl aromatiques; les groupes acyle hétérocycliques saturés ou insaturés; les groupes acyle du type acide N-acyl-$\alpha$-amino; les groupes acyle du type carbamoyle; et les groupes acyle dérivés d'hémi-esters d'acide carbonique.

5. Composé selon la revendication 1, dans lequel X est Cl ou H, et $R_1$ est un groupe acyle représenté par la formule:

$$—CO—R_2$$

(dans laquelle $R_2$ est de l'hydrogène ou un groupe alkyle, alcényle, cycloalkyle, cycloalcényle, aryle ou hétérocyclique, éventuellement substitué ou non substitué, ledit groupe cycloalkyle, cycloalcényle, aryle ou hétérocyclique étant éventuellement lié au carbone du carbonyle par l'intermédiaire d'une chaîne alkylène).

6. Composé selon la revendication 1, dans lequel X est Cl ou H, et $R_1$ est un groupe acyle représenté par la formule:

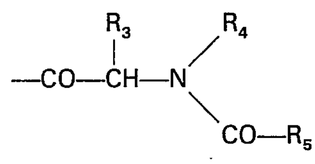

(dans laquelle $R_3$ est de l'hydrogène ou un groupe alkyle, cycloalkyle, aryle, indolyle ou imidazolyle, éventuellement substitué ou non substitué, lesdits groupes cycloalkyle, aryle, indolyle ou imidazolyle étant éventuellement liés à l'atome de carbone *alpha* par l'intermédiaire d'une chaîne alkylène; $R_4$ est de l'hydrogène ou un groupe alkyle, cycloalkyle, cycloalkylalkyle, aryle ou benzyle, éventuellement substitué ou non substitué; et $R_5$ est de l'hydrogène, un groupe alcoxy, bornyloxy, isobornyloxy, benzyloxy, ou un groupe alkyle, alcényle, cycloalkyle, cycloalcényle, aryle ou hétérocyclique, éventuellement substitué ou non substitué, lesdits groupes cycloalkyle, cycloalcényle, aryle ou hétérocyclique étant éventuellement lié à l'atome de carbone du carbonyle adjacent à l'atome d'azote par l'intermédiaire d'une chaîne alkylène).

7. Composé selon la revendication 1, dans lequel X est Cl ou H, et $R_1$ est un groupe acyle représenté par la formule:

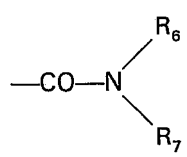

(dans laquelle $R_6$ et $R_7$ peuvent être identiques ou différents et chacun est de l'hydrogène ou un résidu hydrocarboné ou un groupe hétérocyclique, éventuellement substitué ou non substitué, $R_6$ et $R_7$ formant éventuellement un groupe hétérocyclique quand ils sont pris ensemble avec l'atome d'azote adjacent).

8. Composé selon la revendication 1, dans lequel X est Cl ou H, et $R_1$ est un groupe acyle représenté par la formule:

$$—CO—O—R_8$$

(dans laquelle $R_8$ est un résidu hydrocarboné éventuellement substitué ou non substitué).

9. Composé selon la revendication 8, dans lequel le résidu hydrocarboné $R_8$ éventuellement substitué ou non substitué est un groupe alkyle, cycloalkyle, aryle ou aralkyle, éventuellement substitué ou non substitué.

10. Composé déméthylmaytansinoïde de la formule (I) selon la revendication 1, dans lequel ledit composé (I) est la 20-déméthoxy-20-hydroxymaytanacine.

11. Composé déméthylmaytansinoïde de la formule (I) selon la revendication 1, dans lequel ledit composé (I) est le 20-déméthoxy-20-hydroxymaytansinol-propionate.

12. Composé déméthylmaytansinoïde de la formule (I) selon la revendication 1, dans lequel ledit composé (I) est le 20-déméthoxy-20-hydroxymaytansinol-3-isobutyrate.

13. Composé déméthylmaytansinoïde de la formule (I) selon la revendication 1, dans lequel ledit composé (I) est le 20-déméthoxy-20-hydroxymaytansinol-3-butyrate.

14. Composé déméthylmaytansinoïde de la formule (I) selon la revendication 1, dans lequel ledit

composé (I) est le 20-déméthoxy-20-hydroxymaytansinol-3-isovalérate.

15. Composé déméthylmaytansinoïde de la formule (I) selon la revendication 1, dans lequel ledit composé (I) est le 20-déméthoxy-20-hydroxymaytansinol-3-valérate.

16. Composé déméthylmaytansinoïde de la formule (I) selon la revendication 1, dans lequel ledit composé (I) est le 20-déméthoxy-20-hydroxymaytansinol-3-hexanoate.

17. Composé déméthylmaytansinoïde de la formule (I) selon la revendication 1, dans lequel ledit composé (I) est le 20-déméthoxy-20-hydroxymaytansinol-3-cyclohexanecarboxylate.

18. Composé déméthylmaytasinoïde de la formule (I) selon la revendication 1, dans lequel ledit composé (I) est le 20-déméthoxy-20-hydroxymaytansinol-3-p-chlorobenzoate.

19. Composé déméthylmaytansnoïde de la formule (I) selon la revendication 1, dans lequel ledit composé (I) est le 20-déméthoxy-20-hydroxymaytansinol-3-phénylacétate.

20. Composé déméthylmaytansinoïde de la formule (I) selon la revendication 1, dans lequel ledit composé (I) est le 20-déméthoxy-20-hydroxymaytansinol-3-phénylpropionate.

21. Composé déméthylmaytansinoïde de la formule (I) selon la revendication 1, dans lequel ledit composé (I) est le 20-déméthoxy-20-hydroxymaytansinol-3-picolinate.

22. Composé déméthylmaytansinoïde de la formule (I) selon la revendication 1, dans lequel ledit composé (I) est le 20-déméthoxy-20-hydroxymaytansinol-3-(2-furanne)-carboxylate.

23. Composé déméthylmaytansinoïde de la formule (I) selon la revendication 1, dans lequel ledit composé (I) est le 20-déméthoxy-20-hydroxydéchloromaytansinol-3-phénylacétate.

24. Composé déméthylmaytansinoïde de la formule (I) selon la revendication 1, dans lequel ledit composé (I) est le 20-déméthoxy-20-hydroxydéchloromaytansinol-3-isobutyrate.

25. Composé déméthylmaytansinoïde de la formule (I) selon la revendication 1, dans lequel ledit composé (I) est la 20-déméthoxy-20-hydroxyl-L-maytansine.

26. Composé déméthylmaytansinoïde de la formule (I) selon la revendication 1, dans lequel ledit composé (I) est la 20-déméthoxy-20-hydroxy-L-maytanprine.

27. Composé déméthylmaytansinoïde de la formule (I) selon la revendiction 1, dans lequel ledit composé (I) est la 20-déméthoxy-20-hydroxy-L-maytanbutine.

28. Composé déméthylmaytansinoïde de la formule (I) selon la revendication 1, dans lequel ledit composé (I) est la 20-déméthoxy-20-hydroxy-L-maytanvaline.

29. Composé déméthylmaytansinoïde de la formule (I) selon la revendication 1, dans lequel ledit composé (I) est le 20-déméthoxy-20-hydroxymaytansinol-3-(N-acétyl-N-benzyl)-alanine-ester.

30. Composé déméthylmaytansinoïde de la formule (I) selon la revendication 1, dans lequel ledit composé (I) est le 20-déméthoxy-20-hydroxymaytansinol-3-(N-acétyl-N-méthyl)-L-leucine-ester.

31. Composé déméthylmaytansinoïde de la formule (I) selon la revendication 1, dans lequel ledit composé (I) est le 20-déméthoxy-20-hydroxymaytansinol-3-(N-acétyl-N-méthyl)-phénylalanine-ester.

32. Composé déméthylmaytansinoïde de la formule (I) selon la revendication 1, dans lequel ledit composé (I) est le 20-déméthoxy-20-hydroxymaytansinol-3-(N-phényl)-carbamate.

33. Composé déméthylmaytansinoïde de la formule (I) selon la revendication 1, dans lequel ledit composé (I) est le 20-déméthoxy-20-hydroxymaytansinol-3-isopropylcarbonate.

34. Composé déméthylmaytansinoïde de la formule (I) selon la revendication 1, dans lequel ledit composé (I) est le 20-déméthoxy-20-hydroxydéchloromaytansinol-3-benzylcarbonate.

35. Composé déméthylmaytansinoïde de la formule (I) selon la revendication 1, dans lequel ledit composé (I) est le 20-déméthoxy-20-hydroxymaytansinol.

36. Composé déméthylmaytansinoïde de la formule (I) selon la revendication 1, dans lequel ledit composé (I) est le 20-déméthoxy-20-hydroxydéchloromaytansinol.

37. Procédé de préparation d'un composé déméthylmaytansinoïde de la formula (I):

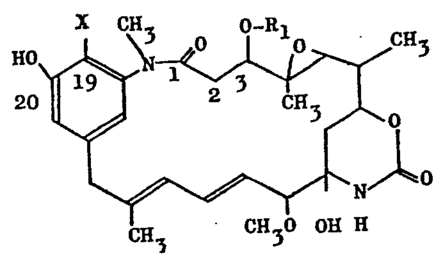

(I)

(dans laquelle X est Cl ou H; $R_1$ est H ou un groupe acyle)
caractérisé par le fait qu'un composé maytansinoïde de la formule (II):

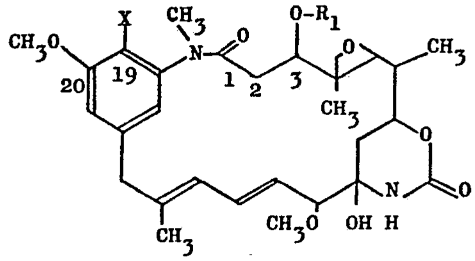

(II)

(dans laquelle X et $R_1$ sont tels que définis respectivement ci-dessus) est mis en contact avec un bouillon de culture, qui comprend une matière traitée du bouillon de culture, d'un micro-organisme appartenant au genre *Bacillus,* au genre *Streptomyces* ou au genre *Actinomyces*, qui est capable de transformer le groupe 20 méthoxy dudit composé maytansinoïde en un groupe hydroxyle, les cellules ou les cellules rompues contenant un système enzymique de déméthylation.

38. Procédé selon la revendication 37, dans lequel le micro-organisme appartient à l'espèce *Bacillus megaterium*, *Streptomyces flavotricini*, *Streptomyces platensis*, *Streptomyces libani* ou *Actinomyces nigrescens*.

39. Procédé selon la revendication 38, dans lequel le micro-organisme est le *Bacillus megaterium* IFO 12108, *Streptomyces flavotricini* IFO 12770, *Streptomyces platensis* IFO 12901, *Streptomyces libani* IFO 13452 ou *Actinomyces nigrescens* IFO 12894.

40. Procédé de préparation d'un composé déméthylmaytansinoïde de la formule (VI):

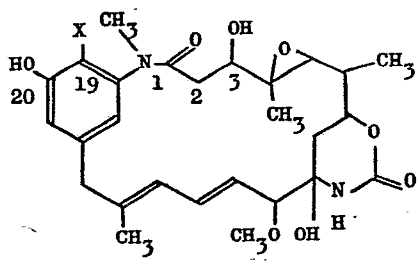

(VI)

(dans laquelle X est Cl ou H)
caractérisé par le fait qu'un composé maytansinoïde de la formule (IV):

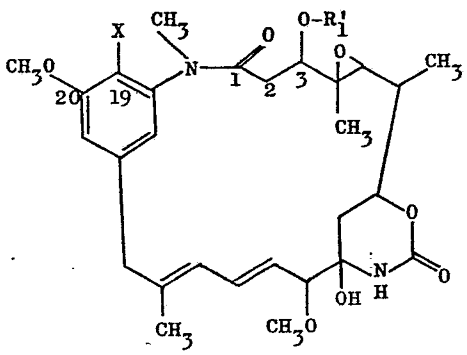

(IV)

(dans laquelle X est tel que défini ci-dessus; et $R_1'$ est un groupe acyle) est mis en contact avec un bouillon de culture, qui comprend une matière traitée de bouillon de culture, d'un micro-organisme du genre *Bacillus*, du genre *Streptomyces* ou du genre *Actinomyces*, qui est capable de transformer le groupe méthoxy-20 dudit composé maytansinoïde (IV) en un groupe hydroxyle pour donner un composé de formule (V):

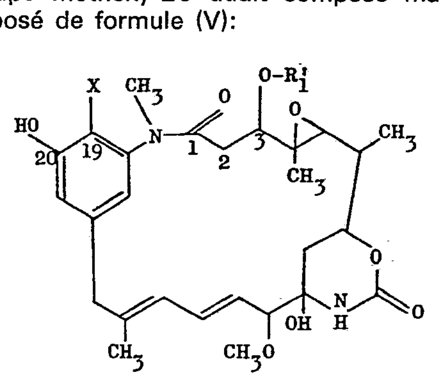

(V)

(dans laquelle X et $R_1'$ sont tels que définis respectivement di-dessus), et que ce dernier composé (V) est ensuite soumis à une désacylation.

41. Procédé selon la revendication 40, dans lequel le micro-organisme appartient à l'espèce *Bacillus megaterium, Streptomyces flavotricini, Streptomyces platensis, Streptomyces libani* ou *Actinomyces nigrescens*.

42. Procédé selon la revendication 40, dans lequel le micro-organisme est *Bacillus megaterium* IFO 12108, *Streptomyces flavotricini* IFO 12770, *Streptomyces platensis* IFO 12901, *Streptomyces libani* IFO 13452 ou *Actinomyces nigrescens* IFO 12894.

43. Procédé selon l'une quelconque des revendications 40 à 42, dans lequel la désacylation est effectuée en utilisant un hydrure métallique complexe.

44. Procédé selon la revendication 43, dans lequel l'hydrure métallique complexe est l'hydrure d'aluminium-lithium.

45. Composé (I) selon la revendication 1 ou selon l'une quelconque des revendications 2 à 36, qui est utilisé sous forme d'agents antifongiques, antiprotozoaires ou anti-mitotiques.

**Patentansprüche**

1. Demethylmaytansinoide Verbindung der Formel (I)

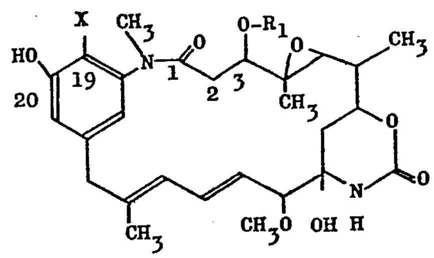

(I)

in der X für Cl oder H und $R_1$ für H oder eine Acyl-Gruppe steht.

2. Verbindung nach Anspruch 1, dadurch gekennzeichnet, daß $R_1$ für eine von einer Carbonsäure, Carbaminsäure oder von einem Halbester eines Kohlensäure-Derivats mit einem Molekulargewicht, von nicht mehr als 300 abgeleitete Acyl-Gruppe steht.

3. Verbindung nach Anspruch 1, dadurch gekennzeichnet, daß $R_1$ für eine Acyl-Gruppe mit 1 bis 20 Kohlenstoff-Atomen steht.

4. Verbindung nach Anspruch 1, dadurch gekennzeichnet, daß $R_1$ für eine Acyl-Gruppe, ausgewählt aus den folgenden Gruppen, steht: Gesättigte und ungesättigte aliphatische Acyl-Gruppen; gesättigte oder ungesättigte alicyclische Acyl-Gruppen; aromatische Acyl-Gruppen; gesättigte oder ungesättigte heterocyclische Acyl-Gruppen; Acyl-Gruppen vom N-Acyl-$\alpha$-aminosäure-Typ; Acyl-Gruppen vom Carbamoyl-Typ und von Halbestern der Kohlensäure abgeleitete Acyl-Gruppen.

5. Verbindung nach Anspruch 1, dadurch gekennzeichnet, daß X für Cl oder H und $R_1$ für eine durch die Formel

$$—CO—R_2$$

bezeichnete Acyl-Gruppe steht, worin $R_2$ Wasserstoff oder eine wahlweise substituierte oder unsubstituierte Alkyl-, Alkenyl-, Cycloalkyl-, Cycloalkenyl-, Aryl- oder heterocyclische Gruppe bezeichnet, wobei die Cycloalkyl-, Cycloalkenyl-, Aryl- oder heterocyclische Gruppe gegebenenfalls durch eine Alkylen-Kette an das Carbonyl-Kohlenstoff-Atom gebunden sein kann.

6. Verbindung nach Anspruch 1, dadurch gekennzeichnet, daß X für Cl oder H und $R_1$ für eine durch die Formel

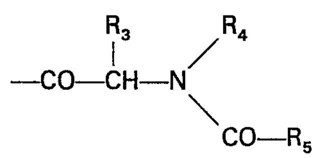

bezeichnete Acyl-Gruppe steht, in der $R_3$ Wasserstoff oder eine wahlweise substituierte oder unsubstituierte Alkyl-, Cycloalkyl-, Aryl-, Indolyl- oder Imidazolyl- Gruppe bezeichnet, wobei die Cycloalkyl-, Aryl-, Indolyl- oder Imidazolyl-Gruppe gegebenenfalls durch eine Alkylen-Kette an das $\alpha$-Kohlenstoff-Atom gebunden sein kann, in der $R_4$ Wasserstoff oder eine wahlweise substituierte oder unsubstituierte Alkyl, Cycloalkyl-, Cycloalkylalkyl-, Aryl- oder Benzyl-Gruppe bezeichnet und in der $R_5$ Wasserstoff oder eine Alkoxy-, Bornyloxy-, Isobornyloxy-, Benzyloxy-Gruppe oder eine wahlweise substituierte

oder unsubstituierte Alkyl-, Alkenyl-, Cycloalkyl-, Cycloalkenyl- Aryl- oder heterocyclische Gruppe bezeichnet, wobei die Cycloalkyl-, Cycloalkenyl-, Aryl- oder heterocyclische Gruppe gegebenenfalls durch eine Alkylen-Kette an das dem Stickstoff-Atom benachbarte Carbonyl-Kohlenstoff-Atom gebunden sein kann.

7. Verbindung nach Anspruch 1, dadurch gekennzeichnet, daß X für Cl oder H und $R_1$ für eine durch die Formel

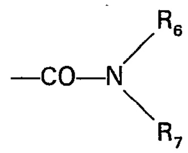

bezeichnete Acyl-Gruppe steht, in der $R_6$ und $R_7$ gleich oder verschieden voneinander sein können und jeweils ein Wasserstoff-Atom oder eine wahlweise substituierte oder unsubstituierte Kohlenwasserstoffrest- oder heterocyclische Gruppe bezeichnen oder gegebenenfalls $R_6$ und $R_7$ gemeinsam mit dem benachbarten Stickstoff-Atom eine heterocyclische Gruppe bilden.

8. Verbindung nach Anspruch 1, dadurch gekennzeichnet, daß X für Cl oder H und $R_1$ für eine durch die Formel

$$—CO—O—R_8$$

bezeichnete Acyl-Gruppe steht, worin $R_8$ einen gegebenenfalls substituierten oder unsubstituierten Kohlenwasserstoff-Rest bezeichnet.

9. Verbindung nach Anspruch 8, dadurch gekennzeichnet, daß der gegebenenfalls substituierte oder unsubstituierte Kohlenwasserstoff-Rest ein gegebenenfalls substituiertes oder unsubstituiertes Alkyl, Cycloalkyl, Aryl oder Aralkyl ist.

10. Demethylmaytansinoide Verbindung der Formel (I) nach Anspruch 1, dadurch gekennzeichnet daß die Verbindung (I) 20-Demethoxy-20-hydroxy-maytanacin ist.

11. Demethylmaytansinoide Verbindung der Formel (I) nach Anspruch 1, dadurch gekennzeichnet, daß die Verbindung (I) 20-Demethoxy-20-hydroxy-maytansinol-propionat ist.

12. Demethylmaytansinoide Verbindung der Formel (I) nach Anspruch 1, dadurch gekennzeichnet, daß die Verbindung (I) 20-Demethoxy-20-hydroxy-maytansinol-3-isobutyrat ist.

13. Demethylmaytansinoide Verbindung der Formel (I) nach Anspruch 1, dadurch gekennzeichnet, daß die Verbindung (I) 20-Demethoxy-20-hydroxy-maytansinol-3-butyrat ist.

14. Demethylmaytansinoide Verbindung der Formel (I) nach Anspruch 1, dadurch gekennzeichnet, daß die Verbindung (I) 20-Demethoxy-20-hydroxy-maytansinol-3-isovalerat ist.

15. Demethylmaytansinoide Verbindung der Formel (I) nach Anspruch 1, dadurch gekennzeichnet, daß die Verbindung (I) 20-Demethoxy-20-hydroxy-maytansinol-3-valerat ist.

16. Demethylmaytansinoide Verbindung der Formel (I) nach Anspruch 1, dadurch gekennzeichnet, daß die Verbindung (I) 20-Demethoxy-20-hydroxy-maytansinol-3-hexanoat ist.

17. Demethylmaytansinoide Verbindung der Formel (I) nach Anspruch 1, dadurch gekennzeichnet, daß die Verbindung (I) 20-Demethoxy-20-hydroxy-maytansinol-3-cyclohexancarboxylat ist.

18. Demethylmaytansinoide Verbindung der Formel (I) nach Anspruch 1, dadurch gekennzeichnet, daß die Verbindung (I) 20-Demethoxy-20-hydroxy-maytansinol-3-p-chlorobenzoat ist.

19. Demethylmaytansinoide Verbindung der Formel (I) nach Anspruch 1, dadurch gekennzeichnet, daß die Verbindung (I) 20-Demethoxy-20-hydroxy-maytansinol-3-phenylacetat ist.

20. Demethylmaytansinoide Verbindung der Formel (I) nach Anspruch 1, dadurch gekennzeichnet, daß die Verbindung (I) 20-Demethoxy-20-hydroxy-maytansinol-3-phenylpropionat ist.

21. Demethylmaytansinoide Verbindung der Formel (I) nach Anspruch 1, dadurch gekennzeichnet, daß die Verbindung (I) 20-Demethoxy-20-hydroxy-maytansinol-3-picolinat ist.

22. Demethylmaytansinoide Verbindung der Formel (I) nach Anspruch 1, dadurch gekennzeichnet, daß die Verbindung (I) 20-Demethoxy-20-hydroxy. maytansinol-3-(2-furan)-carboxylat ist.

23. Demethylmaytansinoide Verbindung der Formel (I) nach Anspruch 1, dadurch gekennzeichnet, daß die Verbindung (I) 20-Demethoxy-20-hydroxy-dechloromaytansinol-3-phenylacetat ist.

24. Demethylmaytansinoide Verbindung der Formel (I) nach Anspruch 1, dadurch gekennzeichnet, daß die Verbindung (I) 20-Demethoxy-20-hydroxy-dechloromaytansinol-3-isobutyrat ist.

25. Demethylmaytansinoide Verbindung der Formel (I) nach Anspruch 1, dadurch gekennzeichnet, daß die Verbindung (I) 20-Demethoxy-20-hydroxy-L-maytansin ist.

26. Demethylmaytansinoide Verbindung der Formel (I) nach Anspruch 1, dadurch gekennzeichnet, daß die Verbindung (I) 20-Demethoxy-20-hydroxy-L-maytanprin ist.

27. Demethylmaytansinoide Verbindung der Formel (I) nach Anspruch 1, dadurch gekennzeichnet, daß die Verbindung (I) 20-Demethoxy-20-hydroxy-L-maytanbutin ist.

28. Demethylmaytansinoide Verbindung der Formel (I) nach Anspruch 1, dadurch gekennzeichnet, daß die Verbindung (I) 20-Demethoxy-20-hydroxy-L-maytanvalin ist.

29. Demethylmaytansinoide Verbindung der Formel (I) nach Anspruch 1, dadurch gekennzeichnet, daß die Verbindung (I) 20-Demethoxy-2-hydroxy-maytansinol-3-(N-acetyl-N-benzyl)-alaninester ist.

30. Demethylmaytansinoide Verbindung der Formel (I) nach Anspruch 1, dadurch gekennzeichnet, daß die Verbindung (I) 20-Demethoxy-20-hydroxy-maytansinol-3-(N-acetyl-N-methyl)-L-leucinester ist.

31. Demethylmaytansinoide Verbindung der Formel (I) nach Anspruch 1, dadurch gekennzeichnet, daß die Verbindung (I) 20-Demethoxy-20-hydroxy-maytansinol-3-(N-acetyl-N-methyl)-phenylalaninester ist.

32. Demethylmaytansinoide Verbindung der Formel (I) nach Anspruch 1, dadurch gekennzeichnet, daß die Verbindung (I) 20-Demethoxy-20-hydroxy-maytansinol-3-(N-phenyl)-carbamat ist.

33. Demethylmaytansinoide Verbindung der Formel (I) nach Anspruch 1, dadurch gekennzeichnet, daß die Verbindung (I) 20-Demethoxy-20-hydroxy-maytansinol-3-isopropylcarbonat ist.

34. Demethylmaytansinoide Verbindung der Formel (I) nach Anspruch 1, dadurch gekennzeichnet, daß die Verbindung (I) 20-Demethoxy-20-hydroxy-dechloromaytansinol-3-benzylcarbonat ist.

35. Demethylmaytansinoide Verbindung der Formel (I) nach Anspruch 1, dadurch gekennzeichnet, daß die Verbindung (I) 20-Demethoxy-20-hydroxy-maytansinol ist.

36. Demethylmaytansinoide Verbindung der Formel (I) nach Anspruch 1, dadurch gekennzeichnet, daß die Verbindung (I) 20-Demethoxy-20-hydroxy-dechloromaytansinol ist.

37. Verfahren zur Herstellung einer demethylmaytansinoiden Verbindung der Formel (I)

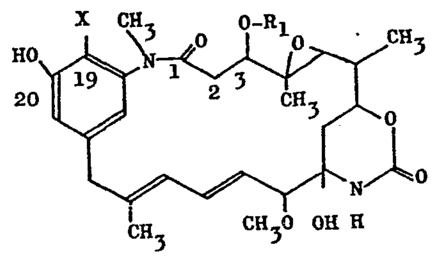

(I)

in der X für Cl oder H und $R_1$ für H oder Acyl steht, dadurch gekennzeichnet, daß eine maytansinoide Verbindung der Formel (II)

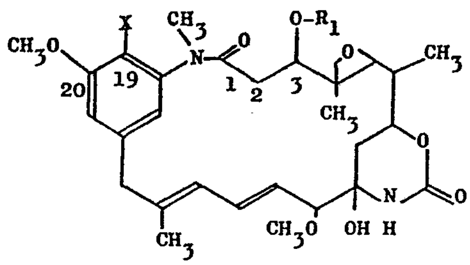

(II)

in der X und $R_1$ die vorstehend genannte Bedeutung haben, mit einer Kulturbrühe, einschließlich weiterverarbeiteten Materials der Kulturbrühe, eines Mikroorganismus gehörend zu dem Genus Bacillus, dem Genus Streptomyces oder dem Genus Actinomyces, der fähig ist, die 20-Methoxy-Gruppe der maytansinoiden Verbindung in eine Hydroxy-Gruppe umzuwandeln, in Berührung gebracht wird, worin die Zellen oder die zerschlagenen Zellen ein Demethylierungs-Enzymsystem enthalten.

38. Verfahren nach Anspruch 37, dadurch gekennzeichnet, daß der Mikroorganismus zu einer der Species Bacillus megaterium, Streptomyces flavotricini, Streptomyces platensis, Streptomyces libani oder Actinomyces nigrescens gehört.

39. Verfahren nach Anspruch 38, dadurch gekennzeichnet, daß der Mikroorganismus Bacillus megaterium IFO 12108, Streptomyces flavotricini IFO 12770, Streptomyces platensis IFO 12901, Streptomyces libani IFO 13452 oder Actinomyces nigrescens IFO 12894 ist.

40. Verfahren zur Herstellung einer demethylmaytansinoiden Verbindung der Formel (VI)

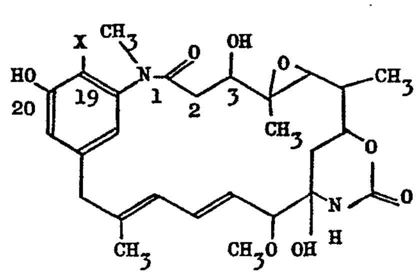

(VI)

worin X für Cl oder H steht,
dadurch gekennzeichnet, daß eine maytansinoide Verbindung der Formel (IV)

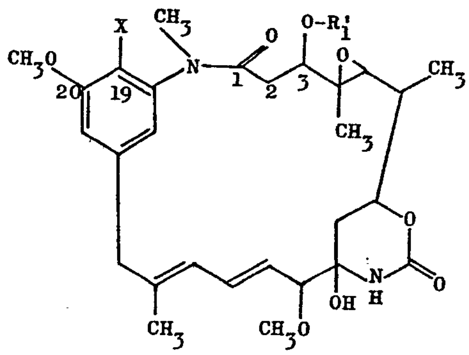

(IV)

worin X die vorstehend genannte Bedeutung hat und $R_1'$ für eine Acyl-Gruppe steht, mit einer Kulturbrühe, einschließlich weiterverarbeiteten Materials einer Kulturbrühe eines Mikroorganismus des Genus Bacillus, des Genus Streptomyces oder des Genus Actinomyces, der fähig ist, die 20-Methoxy-Gruppe der maytansinoiden Verbindung (IV) in eine Hydroxy-Gruppe umzuwandeln, in Berührung gebracht wird, wodurch eine Verbindung der Formel (V) entsteht,

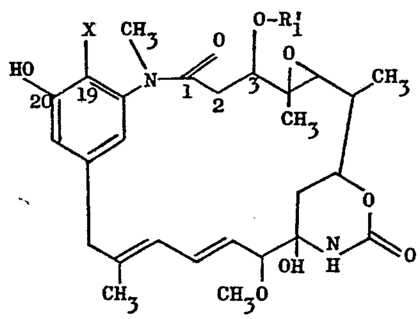

(V)

in der X und $R_1'$ die vorstehend genannte Bedeutung haben, und die letztere Verbindung (V) anschließend der Deacylierung unterworfen wird.

41. Verfahren nach Anspruch 40, dadurch gekennzeichnet, daß der Mikroorganismus zu einer der Species Bacillus megaterium, Streptomyces flavotricini, Streptomyces platensis, Streptomyces libani oder Actinomyces nigrescens gehört.

42. Verfahren nach Anspruch 40, dadurch gekennzeichnet, daß der Mikroorganismus Bacillus megaterium IFO 12108, Streptomyces flavotricini IFO 12770, Streptomyces platensis IFO 12901, Streptomyces libani IFO 13452 oder Actinomyces nigrescens IFO 12894 ist.

43. Verfahren nach Anspruch 40 bis 42, dadurch gekennzeichnet, daß die Deacylierung unter Verwendung eines komplexen Metallhydrids durchgeführt wird.

44. Verfahren nach Anspruch 43, dadurch gekennzeichnet, daß das komplexe Metallhydrid Lithiumaluminiumhydrid ist.

45. Verbindung (I), wie in Anspruch 1 definiert oder wie in den Ansprüchen 2 bis 36 beansprucht, insgesamt zur Verwendung als Antifungi-, Antiprotozoen- oder Antitumor-Mittel.